(19) Europäisches Patentamt
European Patent Office
Office européen des brevets

(11) Veröffentlichungsnummer: **0 200 915 B1**

# (12) EUROPÄISCHE PATENTSCHRIFT

(45) Veröffentlichungstag der Patentschrift: **27.09.89**

(51) Int. Cl.⁴: **C07C 103/44**, C07C 103/737, C07C 127/15, C07D 227/02, C07C 121/75, A61K 31/16, A61K 31/275, A61K 31/395

(21) Anmeldenummer: **86104392.5**

(22) Anmeldetag: **01.04.86**

(54) **Amino-propanol-Derivate, Verfahren zu deren Herstellung, Verwendung derselben und diese enthaltende Arzneimittel.**

(30) Priorität: **06.04.85 DE 3512627**

(43) Veröffentlichungstag der Anmeldung: **17.12.86 Patentblatt 86/46**

(45) Bekanntmachung des Hinweises auf die Patenterteilung: **27.09.89 Patentblatt 89/39**

(84) Benannte Vertragsstaaten: **AT BE CH DE FR GB IT LI LU NL SE**

(56) Entgegenhaltungen:
**EP-A- 0 004 532**
**EP-A- 0 034 461**
**LU-A- 68 997**

**Die Akte enthält technische Angaben, die nach dem Eingang der Anmeldung eingereicht wurden und die nicht in dieser Patentschrift enthalten sind.**

(73) Patentinhaber: **BOEHRINGER MANNHEIM GMBH, Sandhofer Strasse 116, D-6800 Mannheim 31(DE)**

(72) Erfinder: **Simon, Herbert, Dr. rer. nat., Händelstrasse 5, D-6840 Lampertheim(DE)**
Erfinder: **Michel, Helmut, Ziegelgasse 2a, D-6800 Mannheim 31(DE)**
Erfinder: **Bartsch, Wolfgang, Dr. med. vet., Franconviller-Strasse 5, D-6806 Viernheim(DE)**
Erfinder: **Strein, Klaus, Prof., Eichenstrasse 45, D-6944 Hemsbach(DE)**

EP 0 200 915 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Gegenstand der vorliegenden Erfindung sind neue Amino-propanol-Derivate der allgemeinen Formel I

$$Ar-O-CH_2-\overset{OH}{\overset{|}{CH}}-CH_2-NH-A-NH-CO-X-B-(ONO_2)_n \qquad (I)$$

worin

Ar eine Phenylgruppe, die unsubstituiert oder substituiert sein kann durch Halogen, Cyano, Hydroxy, Amino, Oxo, Nitro, Carboxyl, Carbamoyl, Trifluormethyl, $C_1$–$C_{10}$-Alkyl, $C_2$–$C_{10}$-Alkenyl, $C_2$–$C_{10}$-Alkinyl, $C_3$–$C_8$-Cycloalkyl, $C_4$–$C_{11}$-Bicycloalkyl, $C_1$–$C_7$-Alkanoyl, $C_1$–$C_{10}$-Alkoxy, $C_2$–$C_{10}$-Alkoxylakyl, Alkoxyalkoxyalkyl oder Alkoxyalkoxy mit bis zu 10 C-Atomen, $C_2$–$C_{10}$-Alkenyloxy, $C_2$–$C_{10}$-Alkenyloxyalkyl, $C_2$–$C_{10}$-Alkinyloxy, $C_3$–$C_{10}$-Alkinyloxyalkyl, $C_3$–$C_8$-Cycloalkoxy, $C_1$–$C_{10}$-Alkylthio, $C_2$–$C_{10}$-Alkylthioalkyl, $C_1$–$C_7$-Acylamino, Acylaminoalkyl mit bis zu 8 C-Atomen, $C_1$–$C_6$-Acyloxy, $C_1$–$C_5$-Alkoxycarbonyl, $C_4$–$C_8$-Cycloalkoxycarbonyl, $C_2$–$C_4$-Alkylaminocarbonylamino, $C_3$–$C_7$-Dialkylaminocarbonylamino, $C_4$–$C_{11}$-Dialkylaminocarbonylaminoalkyl, $C_4$–$C_{11}$-Dialkylaminocarbonylalkyl, $C_4$–$C_{10}$-Dialkylaminocarbonylalkoxy, wobei bei den letzteren vier Gruppen die beiden endständigen Alkylgruppen zusammen mit dem Stickstoffatom auch eine cyclische Gruppe mit 4 oder 5 C-Atomen bilden können, $C_4$–$C_8$-Cycloalkylaminocarbonylamino, $C_3$–$C_9$-Alkylaminocarbonylaminoalkyl, $C_5$–$C_{12}$-Cycloalkylaminocarbonylaminoalkyl, $C_3$–$C_{12}$-Alkoxycarbonylaminoalkyl, $C_5$–$C_{12}$-Cycloalkoxycarbonylaminoalkyl, $C_2$–$C_5$-Carbamoylalkyl, $C_3$–$C_9$-Alkylaminocarbonylalkyl, $C_5$–$C_{12}$-Cycloalkylaminocarbonylalkyl, oder $C_3$–$C_{10}$-Alkylaminocarbonylalkoxy, oder Ar eine Naphthyl-, Indenyl-, Indolyl-, Indazolyl-, Imidazolyl-, Benzimidazolyl-, Benztriazolyl-, Benzofuranyl-, Benzodioxolyl-, Benzothiophenyl-, Benzthiazolyl-, Benzisothiazolyl-, Chinolyl-, Isochinolyl-, Benzodiazinyl-, Benzopyranyl-, Benzothiinyl-, Benzothiazinyl-, Benzothia-diazinyl-, Benzoxathiinyl-, Benzoxazolyl-, Benzisoxazolyl- oder Carbazolylgruppe bedeutet, wobei eine oder mehrere Doppelbindungen hydriert sein können, und diese zuvor genannten Gruppen unsubstituiert oder substituiert sein können durch $C_1$–$C_{10}$-Alkyl, Cyano, Hydroxyalkyl, Hydroxy, Oxo, Formyl, Alkanoyl oder Alkylcarbonylamino.

A eine geradkettige oder verzweigte Alkylenkette von 1–8 C-Atomen, wobei eine $-CH_2$-Gruppe durch eine Cycloalkylen-Gruppe von 3–7 C-Atomen ersetzt sein kann, bedeutet,

B eine geradkettige, mono- oder bicyclische, gegebenenfalls verzweigte, gesättigte oder ungesättigte Alkylenkette von 1–12 C-Atomen, worin eine $-CH_2$-Gruppe durch eine Cycloalkylen-Gruppe von 3–7 C-Atomen ersetzt sein kann und/oder bis zu 2 $-CH_2$-Gruppen jeweils durch ein Sauerstoffatom oder Schwefelatom oder eine $-S(=O)-$ oder $-S(=O)_2$-Gruppe ersetzt sein können, bedeutet,

X eine Bindung, ein Sauerstoffatom oder eine Gruppe $-NR^1-$, worin $R^1$ Wasserstoff oder eine geradkettige oder verzweigte, gesättigte oder ungesättigte Alkyl- oder Nitroxyalkyl-Gruppe von 1–6 C-Atomen darstellt, oder $R^1$ auch gemeinsam mit dem Stickstoffatom der Gruppe $-NR^1-$ und einer $-CH_2$-Gruppe der Kette B einen heteroaliphatischen Ring mit 4–6 C-Atomen aufspannen kann, bedeutet, n die Zahlen 1 - 3 bedeutet,

sowie deren physiologisch verträgliche Salze.

Die Phenylgruppe $A_2$ kann vorzugsweise einfach (insbesondere in o-Stellung und in p-Stellung, aber auch in m-Stellung) oder zweifach (insbesondere in 2,5-Stellung, aber auch z.B. in 2,3-, 2,4-, 3,4- oder 3,5-Stellung) substituiert sein; sie kann aber auch drei- (insbesondere in 3,4,5-Stellung, aber auch z.B. in 2,3,4,-, 2,3,5- oder 2,4,5-Stellung), vier- (z.B. in 2,3,4,5-Stellung) oder fuenffach substituiert sein. Als Substituenten an der Phenylgruppe kommen insbesondere in Frage: F, Cl, Br, J; CN; OH; $NH_2$; $NO_2$; COOH; $CONH_2$, $CF_3$; Alkyl mit 1-10, vorzugsweise 1-4 C-Atomen, vorzugsweise Methyl oder Ethyl, ferner z.B. n-Propyl, Isopropyl, n-Butyl, Isobutyl, sek.-Butyl, tert.-Butyl, Pentyl wie n-Pentyl, Hexyl wie n-Hexyl, Heptyl wie n-Heptyl, Octyl wie n-Octyl, Nonyl wie n-Nonyl oder Decyl wie n-Decyl; Alkenyl mit bis zu 10, vorzugsweise 2-4 C-Atomen, z.B. Vinyl, Allyl, Propenyl, Isopropenyl, Butenyl wie 1-Buten-1-, -2-, -3- oder -4-yl, 2-Buten-1-yl, 2-Buten-2-yl, Pentenyl, Hexenyl oder Decenyl; Alkinyl mit bis zu 10, vorzugsweise 2-4 C-Atomen, z.B. Ethinyl, 1-Propin-1-yl, Propargyl, Butinyl wie 2-Butin-1-yl, Pentinyl, Decinyl; Cycloalkyl mit 3-8, vorzugsweise 5 oder 6 C-Atomen, vorzugsweise Cyclopentyl oder Cyclohexyl, ferner z.B. Cyclopropyl, Cyclobutyl, 1-, 2- oder 3-Methylcyclopentyl, 1-, 2-, 3- oder 4-Methylcyclohexyl, Cycloheptyl oder Cyclooctyl;

Bicycloalkyl mit 4-11, vorzugsweise 7 C-Atomen, vorzugsweise exo- oder endo 2-Norbonyl, ferner z.B. 2-Isobornyl- oder 5-camphyl;

Alkanoyl mit 1-7, vorzugsweise 1-4 C-Atomen, vorzugsweise Formyl, Acetyl oder Propionyl, ferner z.B. Butyryl, Isobutyryl, Valeroyl, Caproyl, Heptanoyl; Alkoxy mit 1-10, vorzugsweise 1-4 C-Atomen, vorzugsweise Methoxy oder Ethoxy, ferner z.B. n-Propoxy, Isopropoxy, n-Butoxy, Isobutoxy, sek.-Butoxy, tert.-Butoxy, Pentyloxy, Hexyloxy, Heptyloxy, Octyloxy, Nonyloxy oder Decyloxy; Alkoxyalkyl mit bis zu 10, vorzugsweise 2-6 C-Atomen, z.B. Alkoxymethyl wie Methoxymethyl, Alkoxyethyl wie 1- oder 2-Methoxyethyl, 1- oder 2-n-Butoxyethyl, 1- oder 2-n-Octyloxyethyl; Alkoxyalkoxyalkyl mit bis zu 10, vorzugsweise 4-7 C-Atomen, z.B. Alkoxyalkoxymethyl wie 2-Methoxyethoxy-methyl, 2-Ethoxyethoxy-methyl oder 2-Isopropoxyethoxy-methyl, Alkoxyalkoxyethyl wie 2-(2-Methoxyethoxy)-ethyl oder 2-(2-Ethoxyethoxy)-ethyl; Alkoxyalkoxy mit bis zu 10, vorzugsweise 3-6 C-Atomen, z.B. 2-Methoxyethoxy, 2-Ethoxyethoxy oder 2-n-Butoxyethoxy; Alkenyloxy mit bis zu 10, vorzugsweise 2-4 C-Atomen, vorzugsweise Allyloxy, ferner z.B. Vinyloxy, Propenyloxy, Isopropenyloxy, Butenyloxy wie 1-Buten-1-, -2-, -3- oder 4-yloxy, 2-Buten-1-yloxy, 2-Buten-2-yloxy, Pentenyloxy, Hexenyloxy oder Decenyloxy; Alkenyloxyalkyl mit bis zu 10, vorzugsweise 3-6 C-Atomen, z.B. Allyloxymethyl; Alkinyloxy mit bis zu 10, vorzugsweise 2-4 C-Atomen, vorzugsweise Propargyloxy, ferner z.B. Ethinyloxy, 1-Propin-1-yloxy, Butinyloxy wie 2-Butin-1-yloxy, Pentinyloxy oder Decinyloxy; Alkinyloxyalkyl mit bis zu 10, vorzugsweise 3-6 C-Atomen, z.B. Ethinyloxymethyl, Propargyloxymethyl oder -2-(2-Butin-1-yloxy)-ethyl; Cycloalkoxy mit 3-8, vorzugsweise 5 oder 6 C-Atomen, vorzugsweise Cylcopentyloxy oder Cyclohexyloxy, ferner z.B. Cyclopropyloxy, Cyclobutyloxy, 1-, 2- oder 3-Methylcyclopentyloxy, 1-, 2-, 3- oder 4-Methylcyclohexyloxy, Cycloheptyloxy oder Cyclooctyloxy; Alkylthio mit 1-10, vorzugsweise 1-4 C-Atomen, vorzugsweise Methylthio oder Ethylthio, ferner z.B. n-Propylthio, Isopropylthio, n-Butylthio, Isobutylthio, sek.-Butylthio, tert.-Butylthio, Pentylthio, Hexylthio, Heptylthio, Octylthio, Nonylthio oder Decylthio; Alkylthioalkyl mit bis zu 10, vorzugsweise 2-6 C-Atomen, z.B. Methylthiomethyl, 2-Methylthioethyl oder 2-n-Butylthioethyl; Acylamino, vorzugsweise Alkanoylamino mit 1-7, vorzugsweise 1-4 C-Atomen wie Formylamino, Acetylamino, ferner Propionylamino, Butyrylamino, Isobutyrylamino, Valeroylamino, Caproylamino, Heptanoylamino, ferner auch Aroylamino wie Benzoylamino; Acylaminoalkyl, vorzugsweise Alkanoylaminoalkyl mit 1-8, vorzugsweise 3-6 C-Atomen wie Formylaminoethyl, Acetylaminoethyl, Propionylaminoethyl, n-Butyrylaminoethyl, Formylaminopropyl, Acetylaminopropyl, Propionylaminopropyl, Formylaminobutyl, Acetylaminobutyl, ferner Propionylaminobutyl, Butyrylaminobutyl; Acyloxy mit 1-6, vorzugsweise 2-4 C-Atomen, vorzugsweise Acetyloxy, Propionyloxy oder Butyryloxy, ferner z.B. Formyloxy, Valeroyloxy, Caproyloxy; Alkoxycarbonyl mit 1-5, vorzugsweise 2 und 3 C-Atomen, vorzugsweise Methoxycarbonyl oder Ethoxycarbonyl, ferner z.B. n-Propoxycarbonyl, Isopropoxycarbonyl, n-Butoxycarbonyl, Isobutoxycarbonyl, sek.-Butoxycarbonyl oder tert.-Butoxycarbonyl; Cycloalkoxycarbonyl mit 4-8, vorzugsweise 6 oder 7 C-Atomen, vorzugsweise Cyclopentyloxycarbonyl, Cyclohexyloxycarbonyl, ferner Cyclopropyloxycarbonyl, Cyclobutyloxycarbonyl oder Cycloheptyloxycarbonyl; Alkylaminocarbonylamino mit 2-4 C-Atomen wie Methylaminocarbonylamino, Ethylaminocarbonylamino, Propylaminocarbonylamino; Dialkylaminocarbonylamino mit 3-7, vorzugsweise 3-5 C-Atomen, vorzugsweise Dimethylaminocarbonylamino, Diethylaminocarbonylamino, ferner Di-n-propylaminocarbonylamino, Diisopropylaminocarbonylamino; (1-Pyrrolidino)-carbonylamino; (1-Piperidino) carbonylamino; Cycloalkylaminocarbonylamino mit 4-8, vorzugsweise 6 oder 7 C-Atomen, vorzugsweise Cyclopentylaminocarbonylamino, Cyclohexylaminocarbonylamino, ferner Cyclopropylaminocarbonylamino, Cyclobutylaminocarbonylamino, Cycloheptylaminocarbonylamino; Alkylaminocarbonylaminoalkyl mit 3-9, vorzugsweise 4-7 C-Atomen, vorzugsweise Methylaminocarbonylaminoethyl, Ethylaminocarbonylaminoethyl, Ethylaminocarbonylaminopropyl, Ethylaminocarbonylaminobutyl, ferner z.B. Methylaminocarbonylaminomethyl, n-Propylaminocarbonylaminobutyl, n-Butylaminocarbonylaminobutyl; Dialkylaminocarbonylaminoalkyl mit 4-11 C-Atomen, z.B. Dimethylaminocarbonylaminomethyl, Diethylaminocarbonylaminoethyl, Diethylaminocarbonylaminopropyl, Diethylaminocarbonylaminobutyl, (1-Pyrrolidino)carbonylaminoethyl, (1-Piperidino)carbonylaminoethyl; Cycloalkylaminocarbonylaminoalkyl mit 5-12, vorzugsweise 8-11 C-Atomen, vorzugsweise Cyclopentylaminocarbonylaminoethyl, Cyclopentylaminocarbonylaminopropyl, Cyclopentylaminocarbonylaminobutyl, Cyclohexylaminocarbonylaminoethyl, Cyclohexylaminocarbonylaminopropyl, Cyclohexylaminocarbonylaminobutyl, ferner z.B. Cyclopropylaminocarbonylaminomethyl, Cycloheptylaminocarbonylaminoethyl; Alkoxycarbonylaminoalkyl mit 3-12, vorzugsweise 4-9, vorzugsweise Methoxycarbonylaminoethyl, Ethoxycarbonylaminoethyl, n-Propoxycarbonylaminoethyl, Isopropoxycarbonylaminoethyl, n-Butoxycarbonylaminoethyl, Isobutoxycarbonylaminoethyl, sek.-Butoxycarbonylaminoethyl, tert.-Butoxycarbonyl- aminoethyl, Ethoxycarbonylaminopropyl, n-Butoxycarbonylaminopropyl, Ethoxycarbonylaminobutyl, n-Butoxycarbonylaminobutyl, ferner z.B. n-Propoxycarbonylaminopropyl, n-Propoxycarbonylaminobutyl, Isopropoxycarbonylaminobutyl; Cycloalkoxycarbonylaminoalkyl mit 5-12, vorzugsweise 8-11 C-Atomen, vorzugsweise Cyclopentyloxycarbonylaminoethyl, Cyclopentyloxycarbonylaminopropyl, Cyclopentyloxycarbonylaminobutyl, Cyclohexyloxycarbonylaminoethyl, Cyclohexyloxycarbonylaminopropyl, Cyclohexyloxycarbonylaminobutyl, ferner z.B. Cyclopropyloxycarbonylaminomethyl, Cycloheptyloxycarbonylaminoethyl; Carbamoylalkyl mit 2-5, vorzugsweise 2 C-Atomen, vorzugsweise Carbamoylmethyl, ferner Carbamoylethyl, Carbamoylpropyl, Carbamoylbutyl; Alkylaminocarbonylalkyl mit 3-9, vorzugsweise 3-6 C-Atomen, vorzugsweise Methylaminocarbonylmethyl, Ethylaminocarbonylmethyl, n-Propylaminocarbonylmethyl, Isopropylaminocarbonylmethyl, n-Butylaminocarbonylmethyl, Isobutylaminocarbonylmethyl, sek.-Butylaminocarbonylmethyl, tert.-Butylaminocarbonylmethyl, ferner z.B. Ethylaminocarbonylethyl, Ethylaminocarbonylpropyl, Ethylaminocarbonylbutyl, n-Propylaminocarbonylbutyl, n-Butylaminocarbonylbutyl; Dialkylaminocarbonylalkyl mit 4-11, vorzugsweise 4-8 C-Atomen, vorzugsweise Dimethylaminocarbonylmethyl, Diethylaminocarbonylmethyl, Di-n-propylaminocarbonylmethyl, (1-Pyrrolidino)carbonylmethyl, (1-Piperidino)carbonylmethyl, ferner z.B. Diethylaminocarbonylethyl, (1-Piperidino)carbonylethyl, Diethylaminocarbonylpropyl, Diethylaminocarbonylbutyl; Cycloalkylaminocarbonylalkyl mit 5-12, vorzugsweise 7 oder 8 C-Atomen, vorzugsweise Cyclopentylaminocarbonylmethyl, Cyclohexylaminocarbonylmethyl, ferner z.B. Cyclopropylaminocarbonylmethyl, Cyclobutylaminocarbonylmethyl, Cycloheptylaminocarbonylmethyl, Cyclohexylaminocarbonylethyl, Cyclohexylaminocarbonylpropyl, Cyclohexylaminocarbonylbutyl; Alkylaminocarbonylalkoxy mit 3-10, vorzugsweise 3-5 C-Atomen, vorzugsweise Methylaminocarbonylmethoxy, ferner z.B. Methylaminocarbonylethoxy, Methylaminocarbonylpropoxy; Dialkylaminocarbonylalkoxy mit 4-10, vorzugsweise 4-7 C-Atomen, vorzugsweise Dimethylaminocarbonylmethoxy, Diethylaminocarbonylethoxy, (1-Piperidino)carbonylmethoxy, Cycloalkylaminocarbonylalkoxy mit 5-11, vorzugsweise 7 und 8 C-Atomen, vorzugsweise Cyclopentylaminocarbonylmethoxy, Cyclohexylaminocarbonylmethoxy.

Der Rest Ar kann ferner beispielsweise bedeuten: 1- oder 2-Naphthyl; 1-, 2-, 3-, (bevorzugt) 4-, 5-, 6- oder 7-Indanyl; 1-Oxo-4-, -5-, -6- oder (bevorzugt) -7-Indanyl; Alkyl-1-oxo-indanyl, bevorzugt 1-Oxo-5-methyl-7-indanyl; 1-, 2-, 3-, (bevorzugt) 4-, 5-, 6- oder 7-Indenyl; 1-, 2-, 3-, 4-, (bevorzugt) 5-, 6-, 7- oder 8-Tetralyl; Oxo-tetralyl, bevorzugt 1-Oxo-5-tetralyl, ferner 2-, 3- oder 4-oxo-5-tetralyl oder 1-, 2-, 3- oder 4-oxo-6-tetralyl; (bevorzugt)4-, 5-, 6- oder 7-Indolyl; Alkylindolyl, vorzugsweise Methylindolyl, z.B. 2-Methyl-4-indolyl, 3-Methyl-4-indolyl oder 6-Methyl-4-indolyl, ferner z.B. 2-Ethyl-4-indolyl oder 6-Ethyl-4-indolyl; Dialkylindolyl, vorzugsweise Dimethylindolyl, z.B. 2,3-Dimethyl-4-indolyl, 2,6-Dimethyl-4-indolyl, ferner z.B. 2-Methyl-3-ethyl-4-indolyl, 2-Ethyl-3-methyl-4-indolyl, 2,3-Diethyl-4-indolyl; Cyanoindolyl, z.B. 2-Cyano-4-indolyl, 3-Cyano-4-indolyl; Alkyl-cyano-indolyl, bevorzugt 2-Cyano-6-methyl-4-indolyl, ferner z.B. 3-Cyano-6-methyl-4-indolyl; Carbamoyl-indolyl, vorzugsweise 2-Carbamoyl-4-indolyl, 3-Carbamoyl-4-indolyl, ferner z.B. 6-Carbamoyl-4-indolyl; Alkyl-carbamoyl-indolyl, vorzugsweise Methyl-carbamoyl-indolyl, z.B. 2-Carbamoyl-6-methyl-4-indolyl; Hydroxyalkyl-indolyl, bevorzugt 2-Hydroxymethyl-4-indolyl, ferner z.B. 2-Hydroxymethyl-5-indolyl, 3-Hydroxymethyl-4-indolyl, 2-(2-Hydroxyethyl)-4-indolyl; 2-Oxo-indolinyl, bevorzugt 2-Oxo-indolin-4-yl, ferner 2-Oxo-indolin-5-yl; Alkyl-2-oxo-indolinyl, bevorzugt Methyl-2-oxo-indolin-4-yl, z.B. 3-Methyl-2-oxo-indolin-4-yl, ferner z.B. 3-Ethyl-2-oxo-indolin-4-yl, 3-Isopropyl-2-oxo-indolin-4-yl; Dialkyl-2-oxo-indolinyl, z.B. 3,3-Dimethyl-2-oxo-indolin-4-yl, 3,3-Diethyl-2-oxo-indolin-4- yl; Indazol-(bevorzugt)-4-, -5-, -6- oder -7-yl; Benzimidazol-4-yl; Alkyl-benzimidazol-4-yl, bevorzugt Methyl-benzimidazol-4-yl, z.B. 3-Methyl-benzimidazol-4-yl, 1-Methyl-benzimidazol-4-yl, 2-Methyl-benzimidazol-4-yl, 6-Methyl-benzimidazol-4-yl, 7-Methyl-benzimidazol-4-yl; Benzimidazolin-2-on-4-yl (bevorzugt), Benzimidazolin-2-on-5-yl; Alkyl-benzimidazolin-2-on-4-yl, bevorzugt Methyl-benzimidazolin-2-on-4-yl, z.B. 6-Methyl-benzimidazolin-2-on-4-yl, 7-Methyl-benzimidazolin-2-on-4-yl; Benztriazol (bevorzugt) 4- oder -5-yl; Benzofuran-(bevorzugt) 4-, -5-, -6- oder -7-yl; Alkyl-benzofuran-4-yl, z.B. 2-Methyl-benzofuran-4-yl, 3-Methyl-benzofuran-4-yl, 6-Methyl-bezofuran-4-yl; Alkanoyl-benzofuran-4-yl; z.B. 2-Acetyl-benzofuran-4-yl, 6-Acetyl-benzofuran-4-yl; Bis-alkanoyl-benzofuran-yl, z.B. 2,4-Diacetyl-benzofuran-5-yl, 2,6-Diacetyl-benzofuran-4-yl; 1,3-Benzodioxolyl, bevorzugt 1,3-Benzodioxol-4-yl; Alkyl-1,3-benzodioxolyl, bevorzugt 2-Methyl-1,3-benzodioxol-4-yl, ferner z.B. 6-Methyl-1,3-benzodioxol-4-yl; Dialkyl-1,3-benzodioxolyl, bevorzugt 2,2-Dimethyl-1,3-benzodioxol-4-yl, ferner z.B. 2,2-Diethyl-1,3-benzodioxol-4-yl, 2,6-Dimethyl-1,3-benzodioxol-4-yl; 1,2-Benzisoxazol-(bevorzugt) 4-,-5-,-6- oder -7-yl; Alkyl-1,2-benzisoxazolyl, bevorzugt 3-Methyl-1,2-benzisoxazol-4-yl, ferner z.B. 3-Ethyl-1,2-benzisoxazol-4-yl, 3-Propyl-1,2-benzisoxazol-4-yl, 3-Isoproyl-1,2-benzisoxazol-4-yl, 6-Methyl-1,2-benzisoxasol-4-yl; 1,3-Benzoxazol-(bevorzugt) 4-, -5-, -6-oder-7-yl; Alkyl-1,3-benzoxazolyl, bevorzugt 2-Methyl-1,3-benzoxazol-4-yl, ferner z.B. 2-Ethyl-1,3-benzoxazol-4-yl, 6-Methyl-1,3-benzoxazol-4-yl, 6-Methyl-1,3-benzoxazol-4-yl; Aryl-1,3-benzoxazolyl, bevorzugt 2-Phenyl-1,3-benzoxazol-4-yl, 2-(4-Pyridyl)-1,3-benzoxazol-4-yl; Benzthiophen- (bevorzugt) 4-, -5-, -6- oder -7-yl; 1,2-Benzisothiazol- (bevorzugt) 4-, -5-, -6- oder -7-yl; Alkyl-1,2-benzisothiazol-yl, z.B. 6-Methyl-1,2-benzisothiazol-4-yl; 1,3-Benzthiazol-4-, -5-, -6- oder -(bevorzugt) 7-yl; Alkyl-1,3-benzthiazol-7-yl, z.B. 2-Methyl-1,3-benzthiazol-7-yl; 4-Methyl-1,3-benzthiazol-7-yl, 2-Ethyl-1,3-benzthiazol-7-yl; 2-Aryl-1,3-benzthiazol-7-yl, z.B. 2-Phenyl-1,3-benzthiazol-7-yl; 2-(4-Chlorphenyl)-1,3-benzthiazol-7-yl; 2-(4-Pyridyl)-1,3-benzthiazol-7-yl; 1,2-Dihydro-2-oxo-3-, -4-, (bevorzugt) -5-, -6-, -7- oder -8-chinolyl; 1,2,3,4-Tetrahydro-(bevorzugt) -5-, -6-, -7- oder -8-chinolyl; 1,2,3,4-Tetrahydro-2-oxo (bevorzugt) -5-, -6-, -7- oder -8-chinolyl; 1,2-Dihydro-8-hydroxy-2-oxo-(bevorzugt) 5-, -6- oder -7-chinolyl; 1,2-Dihydro-8-alkoxy-2-oxo-(bevorzugt) 5-, -6- oder -7-chinolyl, z.B. 1,2-Dihydro-8-methoxy-2-oxo-5-chinolyl; 1,2,3,4-Tetrahydro-8-hydroxy-2-oxo (bevorzugt) 5-, -6-, oder -7-chinolyl; 1,2,3,4-Tetrahydro- 8-alkoxy-2-oxo-(bevorzugt) 5-, -6- oder -7-chinolyl, z.B. 1,2,3-4-Tetrahydro-8-methoxy-2-oxo-5-chinolyl; 1,2,3,4-Tetrahydro-8-alkanoylamino-2-oxo-(bevorzugt) 5-, -6- oder -7-chinolyl, z.B. 1,2,3,4-Tetrahydro-8-acetylamino-2-oxo-5-chinolyl; 1,2-Dihydro-3-cyano-2-oxo-(bevorzugt) 5-, -6-, -7- oder -8-chinolyl; 1,2-Dihydro-3-cyano-2-oxo-7-methyl-5-chinolyl; 1,2-Dihydro-1-oxo-(bevorzugt) 4-, -5-, -6-, -7- oder -8-isochinolyl; 1,2-Dihydro-2-alkyl -1-oxo-(bevorzugt) 4-, -5-, -6-, -7- oder -8-isochinolyl, z.B. 1,2-Dihydro-2-methyl-1-oxo-4-isochinolyl; 1,2,3,4-Tetrahydro-2-alkanoyl-(bevorzugt) 5-, -6-, -7- oder -8-isochinolyl, bevorzugt 1,2,3,4-Tetrahydro-2-formyl-5-isochinolyl, ferner z.B. 1,2,3,4-Tetrahydro-2-acetyl-5-isochinolyl; 1,2-Dihydro-2-oxo-1,3-benzodiazin-(bevorzugt) 5-, -6-, -7- oder -8-yl; 2H-3,4-Dihydro-5-, -6-,-7- oder - (bevorzugt) -8- benzopyranyl ; 2H-2-oxo-5-alkyl-7- oder - (bevorzugt) 8-benzopyranyl, z.B. 2H-2-oxo-5-methyl-8-benzopyranyl; 2H-3-Cyano-5-, -6-, -7- oder - (bevorzugt) 8-benzopyranyl; 2H-3,4-Dihydro-5-, -6-, -7- oder -(bevorzugt) 8-benzothiinyl, 3,4-Dihydro-1H-2,2-dioxo-2,1-benzothiazin- (bevorzugt) 5-, -6-, -7- oder -8-yl; 3,4-Dihydro-1H-1-alkyl-2,2-dioxo-2,1-benzothiazin-(bevorzugt) 5-, -6-, -7- oder -8-yl, z.B. 3,4-Dihydro-1H- 1-methyl-2,2-dioxo-2,1-benzothiazin-5-yl; 3,4-Dihydro-2H-3-oxo-1,4-benzothiazin-5-, -6-, -7- oder - (bevorzugt) 8-yl; 5- oder 6-Alkyl-3,4-dihydro-3-oxo-1,4-benzothiazin-8-yl, z.B. 6-Methyl-3,4-dihydro-3-oxo-1,4-benzothiazin-8-yl; 1,1-Dioxo-1,2,4-benzothiadiazin-5-, -6-, -7- oder -8-yl; 1,1-Dioxo-3-alkyl-1,2,4-benzothiadiazin-5-, -6-, -7- oder -8-yl, z.B. 1,1-Dioxo-3-methyl-1,2,4-benzothiadiazin-5-, -6-, -7- oder -8-yl; 1,1-Dioxo-3-alkanoyl-1,2,4-benzothiadiazin-5-, -6-, -7- oder -8-yl, z.B. 1,1-Dioxo-3-formyl-1,2,4-benzothiadiazin-5-, -6-, -7- oder -8-yl, 1,1-Dioxo-3-acetyl-1,2,4-benzothiadiazin-5-, -6-, -7- oder -8-yl; 1,1-Dioxo-3-aroyl-1,2,4-benzothiadiazin-5-, -6-, -7- oder -8-yl; z.B. 1,1-Dioxo-3-benzoyl-1,2,4-benzothiadiazin-5-, -6-, -7- oder -8-yl, 1,1-Dioxo-3-(4-pyridyl-carbonyl)-1,2,4-benzothiadiazin-5-, -6-, -7- oder -8-yl; 3,4-Dihydro-2,2-dioxo-1,2-benzoxathiin-(bevorzugt) 5-, -6-, -7- oder -(bevorzugt) 8-yl; 1-, 2-, 3- oder (bevorzugt) 4-Carbazolyl.

A kann eine geradkettige Alkylenkette von 1-8, bevorzugt 2-4 C-Atomen, bevorzugt Ethylen, Trimethylen, Tetramethylen, ferner Methylen, Pentamethylen, Hexamethylen, Heptamethylen, Octamethylen bedeuten. Die Alkylenkette A kann auch verzweigt sein und aus 3-8, bevorzugt 3-6 C-Atomen, bestehen. Bevorzugt sind z.B. Propylen, 1,1-Dimethyl-ethylen, 1-Methyl-trimethylen, 1,1-Dimethyl-trimethylen, 2,2-Dimethyl-trimethylen, 1-Methyl-tetramethylen; ferner kommen z.B. 1-Ethyl-trimethylen, 1,1-Dimethyl-tetramethylen, 1,2-Dimethyl-tetramethylen, 2,2-Dimethyl-tetramethylen, 2,3-Dimethyl-tetramethylen, 3,3-Dimethyl-pentamethylen, 1-Methyl-hexamethylen, 1-Methyl-heptamethylen in Frage. Eine $-CH_2-$ Gruppe oder Alkylenkette kann durch eine Cycloalkylen-Gruppe mit 3-7, vorzugsweise 6 C-Atomen ersetzt sein. Vorzugsweise kommen der 1,2- 1,3- und 1,4-Cyclohexylen-Rest in Frage, wobei die Stellung der Substituenten jeweils cis oder trans sein kann. Ferner kann z.B. die Methylen-1,2-, -1,3- oder -1,4-cyclohexylen-Gruppe, die Methylen-1,2-, -1,3- oder-1,4-cyclohexylen-methylen-Gruppe, die Ethylen-1,2-, -1,3- oder -1,4-cyclohexylen-Gruppe, die Trimethylen-1,2-, -1,3- oder -1,4-cyclohexylen-Gruppe, die Tetramethylen-1,2-, -1,3- oder -1,4-cyclohexylen-Gruppe oder auch die Methylen-1,2-, -1,3- oder -1,4-cyclohexylen-ethylen-Gruppe oder die Ethylen-1,2-, -1,3- oder -1,4-cyclohexylen-tetramethylen-Gruppe eingesetzt werden, die Konfiguration der Cycloalkylen-Gruppe kann jeweils cis oder trans sein. Weitere Alkylenketten A sind z.B. die 1,2-Cyclopropylen-Gruppe, die Methylen-1,2-cyclopropylen-Gruppe, die Tetramethylen-1,2-cyclopropylen-Gruppe, die Methylen-1,2-cyclopropylen-methylen-Gruppe, die Methylen-1,2-cyclopropylen-ethylen-Gruppe, die 1,2- oder 1,3-Cyclobutylen-Gruppe, die Methylen-1,2- oder -1,3-cyclobutylen-Gruppe, die Tetramethylen-1,2- oder -1,3-cyclobutylen-Gruppe, die Methylen-1,2- oder -1,3-cyclobutylen-methylen-Gruppe, die Methylen-1,2- oder -1,3-cyclobutylen-ethylen-Gruppe, die 1,2- oder 1,3-Cyclopentylen-Gruppe, die Methylen-1,2- oder -1,3-cyclopentylen-Gruppe, die Tetramethylen-1,2- oder -1,3-cyclopentylen-Gruppe, die Methylen-1,2- oder -1,3-cyclopentylen-methylen-Gruppe, die Methylen-1,2- oder -1,3-cyclopentylen-ethylen-Gruppe, die 1,2- 1,3- oder 1,4-Cycloheptylen-Gruppe, die Methylen-1,2-, -1,3- oder -1,4-cycloheptylen-Gruppe, die Tetramethylen-1,2-, -1,3- oder -1,4-cycloheptylen-Gruppe, die Methylen-1,2-, -1,3- oder -1,4-cycloheptylen-methylen-Gruppe, die Methylen-1,2-, -1,3- oder -1,4-cycloheptylen-ethylen-Gruppe, die Konfiguration der Cycloalkylen-Gruppe kann jeweils cis oder trans sein.

Die Gruppe B kann eine geradkettige oder verzweigte Alkylenkette von 1-12, vorzugsweise 4-6 C-Atomen bedeuten, vorzugsweise Tetramethylen, 1-Methyl-trimethylen, Pentamethylen, 1-Methyl-tetramethylen, 1-Ethyl-trimethylen, Hexamethylen, 1-Methyl-pentamethylen, 1-Ethyl-tetramethylen, 1-n-Propyl-trimethylen, 1,1-Dimethylethylen, ferner z.B. Methylen, Methylmethylen, Dimethylmethylen, 2,2-Dimethyl-trimethylen, 1,1-Dimethyl-hexamethylen, wobei in allen Faellen jeweils noch 1 oder 2 H-Atome durch die $-O-NO_2$-Gruppe ersetzt sein koennen. Als cyclische oder bicyclische Alkylenkette B kommen vorzugsweise die 1,2-Cyclopentylen-Gruppe, die 1,2- 1,3- oder 1,4-Cyclohexylen-Gruppe, die 1,2-Cyclohexylen-methylen- oder die 2,5-Isosorbid-Gruppe in Frage, wobei die Konfiguration der Cycloalkylen-Gruppe jeweils cis oder trans sein kann, ferner kommen z.B. die 1,2-, 1,3- oder 1,4-Phenylen-methylen-Gruppe, die Methylen-1,2-, -1,3- oder -1,4-Phenylen-methylen-Gruppe, die Ethylen-1,4-phenylen-Gruppe, die Ethylen-1,4-phenylen-ethylen-Gruppe, die Ethylen-1,4-phenylen-tetramethylen-Gruppe oder die Trimethylen-1,4-phenylen-trimethylen-Gruppe in Frage, wobei in allen Faellen noch 1 oder 2 H-Atome durch die $-O-NO_2$-Gruppe ersetzt sein koennen.

Im Fall, daß X die gruppe $-NR^1-$ darstellt, kann $R^1$ Wasserstoff oder einen geradkettigen oder verzweigten, gesaettigten oder ungesaettigten Alkylrest von 1-6, vorzugsweise 1-3 C-Atomen bedeuten, vorzugsweise die Methyl-Gruppe, die Ethyl-Gruppe, die n-Propyl-Gruppe, die Isopropyl-Gruppe oder die Allyl-Gruppe, ferner z.B. die n-Butyl-Gruppe, die n-Pentyl-Gruppe und die n-Hexyl-Gruppe. Im Fall, daß $R^1$ gemeinsam mit einer $-CH_2$-Gruppe der Kette B und dem Stickstoffatom einen heteroaliphatischen Ring von 4–6 C-Atomen aufspannen kann, kommen vorzugsweise der Pyrrolidinylen-, der Piperidinylen- und der 1,2-, 1,3- oder 1,4-Piperidinylen-methylen-Rest in Frage, ferner z.B. der Perhydroazepinylen-Rest.

Verbindungen ähnlicher Art sind bereits z.B. beschrieben in DE-OS 2 362 568, EP-OS 0 082 665, EP-OS 0 086 564, EP-OS 0 096 006, EP-OS 0 105 838, GB 2 111 500 A, WO 83/01 770, WO 83/01 772. Die dortigen Verbindungen unterscheiden sich jedoch dadurch von den erfindungsgemäßen Verbindungen der allgemeinen Formel I, daß sie nicht die Gruppierung $-O-NO_2$ besitzen.

Verbindungen mit der Gruppierung $O-NO_2$ sind in der EP-A 0 004 532 beschrieben. Diese Verbindungen haben eine kurze Wirkdauer und unterscheiden sich strukturell von den vorliegenden Verbindungen unter anderem dadurch, daß sie keine Alkylendiamin-Gruppierung aufweisen.

Die erfindungsgemäßen Verbindungen der allgemeinen Formel I besitzen wertvolle Eigenschaften. Sie besitzen nicht nur β-Rezeptoren blockierende Aktivität, sondern sie bewirken auch eine Verminderung des Sauerstoffbedarfs des Herzens, eine Erhöhung des Blutflusses und eine Erniedrigung des Blutdruckes. Sie eignen sich daher zur Prophylaxe und/oder Behandlung von Herz- und Kreislauferkrankungen, wie z.B. Hochdruck und Angina pectoris.

Die erfindungsgemäßen Verbindungen werden üblicherweise in Mengen von 20–500 mg pro Tag bezogen auf 75 kg Körpergewicht appliziert. Bevorzugt ist es, 2–3mal pro Tag 1–2 Tabletten mit einem Wirkstoffgehalt von 10–200 mg zu verabreichen. Die Tabletten können auch retardiert sein, wodurch nur noch 1mal pro Tag 1–2 Tabletten mit 20–500 mg Wirkstoff gegeben werden muß. Der Wirkstoff kann auch durch Injektion 1–8mal pro Tag bzw. durch Dauerinfusion gegeben werden, wobei Mengen von 5–200 mg/Tag normalerweise ausreichen.

Die erfindungsgemaeßen Verbindungen der allgemeinen Formel I koennen in an sich bekannter Weise dadurch hergestellt werden, daß man

a) eine Verbindung der allgemeinen Formel II

$$Ar-O-CH_2-\overset{OH}{\overset{|}{C}H}-CH_2-NH-A-NH-CO-X-B-(OH)_n \qquad (II)$$

in der A, Ar, B, X und n die oben angegebenen Bedeutungen besitzen, einer Nitratester-Bildungsreaktion unterwirft, oder

b) eine Verbindung der allgemeinen Formel III

Ar'-O-Z (III)

in der Ar' die gleiche Bedeutung wie Ar hat oder gegebenenfalls eine entsprechende Synthesevorstufe sein kann und Z eine der Gruppierungen

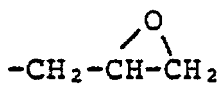 oder $-CH_2-\overset{OH}{\overset{|}{C}H}-CH_2-Y$

darstellt, worin Y eine reaktive Gruppe bedeutet,

b1) mit einem Amin der allgemeinen Formel IV

$H_2N-A-NH-CO-X-B-(ONO_2)_n$ (IV)

in der A, B, X und n die oben angegebenen Bedeutungen haben, oder

b2) mit einem Amin der allgemeinen Formel V

$H_2N-A-(NH_2)_{mask.}$ (V)

worin A die oben angegebene Bedeutung hat und $-(NH_2)_{mask.}$ eine freie $-NH_2$-Gruppe oder gegebenenfalls eine maskierte $-NH_2$-Gruppe darstellt,
zu einer Verbindung der allgemeinen Formel VI

$$Ar'-O-CH_2-\overset{OH}{\overset{|}{C}H}-CH_2-NH-A-(NH_2)_{mask.} \qquad (VI)$$

umsetzt und diese, gegebenenfalls nach Demaskierung der $-(NH_2)_{mask.}$-Gruppierung zur $-NH_2$-Gruppe und/oder nach Ueberfuehrung der Gruppe Ar' in die Gruppe Ar mit einer Verbindung der allgemeinen Formel VII

$Y-CO-X-B-(ONO_2)_n$ (VII)

in der B, X, Y und n die oben angegebenen Bedeutungen besitzen, umsetzt, oder

c) eine Verbindung der allgemeinen Formel VIII

Ar'-OH (VIII)

in der Ar' die oben angegebene Bedeutung hat

c1) mit einer Verbindung der allgemeinen Formel IX

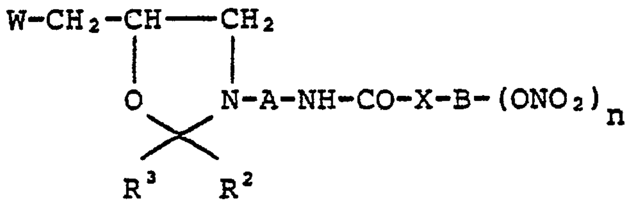

(IX)

worin A, B, X und n die oben angegebenen Bedeutungen besitzen, W Mesyloxy, Tosyloxy oder Halogen, $R^3$ Wasserstoff oder Alkyl und $R^2$ unabhaengig Wasserstoff, Alkyl oder Phenyl bedeuten oder $R^3$ und $R^2$ zusammen mit dem benachbarten Kohlenstoffatom ein Carbonyl-Radikal bilden, umsetzt, und gegebenenfalls die Gruppe Ar' in die Gruppe Ar ueberfuehrt und den Oxazolidin-Ring spaltet, oder

c2) mit einer Verbindung der allgemeinen Formel X

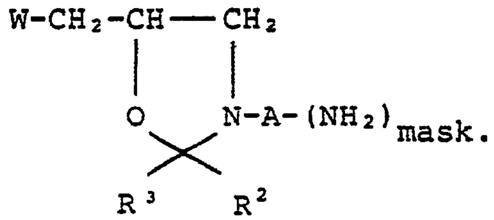

(X)

worin A, $R^3$, $R^2$, W und $-(NH_2)_{mask.}$ die oben genannten Bedeutungen haben, umsetzt, den Oxazolidin-Ring spaltete und die erhaltene Verbindung der allgemeinen Formel VI, gegebenenfalls nach Ueberfuehrung der Gruppe Ar' in die Gruppe Ar und/oder nach Demaskierung der Gruppe $-(NH_2)_{mask.}$ in die $-NH_2$-Gruppe, mit einer Verbindung der allgemeinen Formel VII umsetzt, oder

c3) mit einer Verbindung der allgemeinen Formel XI

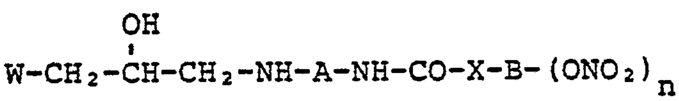

(XI)

in der A, B, W, X und n die oben angegebenen Bedeutungen besitzen, umsetzt und gegebenenfalls die Gruppe Ar' in die Gruppe Ar ueberfuehrt, oder

c4) mit einer Verbindung der allgemeinen Formel XII

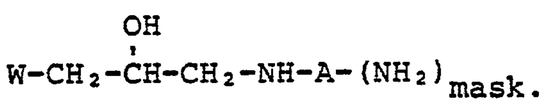

(XII)

in der A, W und $-(NH_2)_{mask.}$ die oben angegebenen Bedeutungen besitzen,
zu einer Verbindung der allgemeinen Formel VI umsetzt und diese, gegebenenfalls nach Ueberfuehrung der Gruppe Ar' in die Gruppe Ar und/oder nach Demaskierung der Gruppe $-(NH_2)_{mask.}$ in die $-NH_2$-Gruppe, mit einer Verbindung der allgemeinen Formel VII umsetzt, oder

d) eine Verbindung der allgemeinen Formel XIII

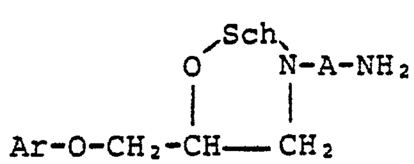

(XIII)

in der A und Ar die oben angegebenen Bedeutungen besitzen und Sch ein Dialkylsilylen-Radikal bedeutet, mit einer Verbindung der allgemeinen Formel VII umsetzt und die Sch-Gruppierung abspaltet.

Die Verbindungen der allgemeinen Formel II (Zwischenprodukte fuer die Herstellung von Verbindungen der allgemeinen Formel I) koennen hergestellt werden, indem man in an sich bekannter Weise

a) eine Verbindung der allgemeinen Formel III
a1) mit einem Amin der allgemeinen Formel XIV

$H_2N$-A-NH-CO-X-B-$(OH)_n$ (XIV)

in der A, B, X und n die oben angegebenen Bedeutungen haben, umsetzt, oder
a2) mit einem Amin der allgemeinen Formel V zu einer Verbindung der allgemeinen Formel VI umsetzt und diese, gegebenenfalls nach Demaskierung der -$(NH_2)_{mask.}$-Gruppierung zur -$NH_2$-Gruppe und/oder der Ueberfuehrung der Gruppe Ar' in die Gruppe Ar, mit einer Verbindung der allgemeinen Formel XV

Y-CO-X-B-$(OH)_n$ (XV)

in der B, X, Y und n die oben angegebenen Bedeutungen besitzen, umsetzt, oder
b) eine Verbindung der allgemeinen Formel VIII
b1) mit einer Verbindung der allgemeinen Formel XVI

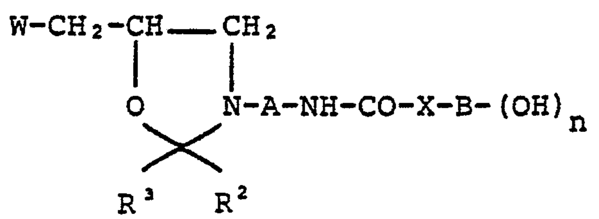
(XVI)

in der A, B, $R^3$, $R^2$, W, X und n die oben angegebenen Bedeutungen haben umsetzt und den Oxazolidin-Ring spaltet, oder
b2) mit einer Verbindung der allgemeinen Formel X umsetzt, den Oxazolidin-Ring spaltet und die so erhaltene Verbindung der allgemeinen Formel VI gegebenenfalls nach Ueberfuehrung der Gruppe Ar' in die Gruppe Ar und/oder nach Demaskierung der Gruppe -$(NH_2)_{mask.}$ in die -$NH_2$-Gruppe, mit einer Verbindung der allgemeinen Formel XV umsetzt, oder
b3) mit einer Verbindung der allgemeinen Formel XVII

$$W-CH_2-\overset{OH}{\overset{|}{C}H}-CH_2-NH-A-NH-CO-X-B-(OH)_n \quad (XVII)$$

in der A, B, W, X und n die oben angegebenen Bedeutungen besitzen, umsetzt und gegebenenfalls die Gruppe Ar' in die Gruppe Ar ueberfuehrt, oder
b4) mit einer Verbindung der allgemeinen Formel XII zu einer Verbindung der allgemeinen Formel VI umsetzt, und diese, gegebenenfalls nach Ueberfuehrung der Gruppe Ar' in die Gruppe Ar und/oder nach Demaskierung der Gruppe -$(NH_2)_{mask.}$ in die -$NH_2$-Gruppe, mit einer Verbindung der allgemeinen Formel XV umsetzt, oder
c) eine Verbindung der allgemeinen Formel XIII mit einer Verbindung der allgemeinen Formel XV umsetzt und die Sch-Gruppierung abspaltet.

Die Amine IV koennen in an sich bekannter Weise dadurch hergestellt werden, daß man

a) Verbindungen der allgemeinen Formel XVIII
$H_2N$-A-$NH_2$ (XVIII)
in der A die oben angegebene Bedeutung hat, mit aktivierten Verbindungen der allgemeinen Formel VII umsetzt, oder
b) Verbindungen der allgemeinen Formel XVIII mit aktivierten Verbindungen der allgemeinen Formel XV zu Verbindungen der allgemeinen Formel XIV umsetzt und diese einer Nitratester-Bildungsreaktion unterwirft, oder
c) maskierte Verbindungen der allgemeinen Formel V mit aktivierten Verbindungen der allgemeinen Formel VII umsetzt und die -$(NH_2)_{mask.}$-Gruppierung zur -$NH_2$-Gruppe demaskiert, oder
d) Verbindungen der allgemeinen Formel V mit aktivierten Verbindungen der allgemeinen Formel XV umsetzt und, nach Demaskierung der -$(NH_2)_{mask.}$-Gruppe zur -$NH_2$-Gruppe, die erhaltenen Verbindungen der allgemeinen Formel XIV einer Nitratester-Bildungsreaktion unterwirft.

Die Nitratester-Bildungsreaktion der Verbindungen der allgemeinen Formeln II und XIV kann durchgefuehrt werden, indem die Verbindungen der allgemeinen Formeln II und XIV mit einem nitratesterbildenden Reagenz, wie rauchender Salpetersaeure, einer Mischung aus rauchender Salpetersaeure und Acetanhydrid oder einer Mischung aus rauchender Salpetersaeure und konz. Schwefelsaeure bei niedrigen Temperaturen in Gegenwart oder Abwesenheit eines inerten Loesungsmittels umsetzt. Die Reaktionstemperaturen liegen zwischen Raumtemperatur und -60°C, bevorzugt zwischen -10°C und -30°C. Das Molverhaeltnis der Reaktionspartner liegt zwischen 1 und 10.

Alternativ kann die Nitratester-Bildungsreaktion durchgefuehrt werden, indem man in einer Verbindung der allgemeinen Formel II oder XIV selektiv eine aliphatische Hydroxylgruppe durch eine Halogengruppe ersetzt und anschließend das Reaktionsprodukt mit Silbernitrat in Gegenwart oder Abwesenheit eines Lösungsmittels bei Temperaturen zwischen Raumtemperatur und 100°C umsetzt. Das Molverhaeltnis der Umsetzungsreaktion zwischen der Halogenverbindung und Silbernitrat kann zwischen 1 und 10 liegen.

Die Halogenierungsreaktion kann nach literaturbekannten Verfahren durchgefuehrt werden, indem man eine Verbindung der allgemeinen Formel II oder XIV mit Mesylchlorid oder Tosylchlorid in Gegenwart eins sauerebindenden Mittels umsetzt und das erhaltene Reaktionsprodukt anschließend mit einem Alkalihalogenid in einem organischen Loesungsmittel, z.B. Dimethylformamid, zur Reaktion bringt.

Die Umsetzungen der Verbindungen der allgemeinen Formel VIII mit Epihalogenhydrinen oder Verbindungen der allgemeinen Formeln IX, X, XI, XII, XVI und XVII werden in an sich bekannter Weise durchgefuehrt, indem man die Reaktionspartner in einem organischen Loesungsmittel und/oder Wasser in Gegenwart eines saeurebindenden Mittels wie Alkalihydroxiden oder -hydriden oder organischen Stickstoffbasen bei Temperaturen zwischen Raumtemperatur und 100°C umsetzt. Die Molverhaeltnisse der Verbindungen der allgemeinen Formel VIII zu den Epihalogenhydrinen oder den Verbindungen der allgemeinen Formeln, IX, X, XI, XII, XVI und XVII können zwischen 1 und 100 liegen. Als organische Lösungsmittel kommen z.B. Methanol, Ethanol, Propanol, Benzol, Toluol, Tetrahydrofuran, Dimethylformamid oder Dimethylsulfoxid in Frage. Die Spaltung der Oxazolidin-Ringe in den Formeln IX und X erfolgt unter sauren Bedingungen im Fall $R^2$, $R^3$ = H, Alkyl oder Aryl oder unter basischen Bedingungen (z.B. 4n NaOH / Ethanol) im Fall

$$R^2,\ R^3 = {>}CO.$$

Die Umsetzungen der Verbindungen der allgemeinen Formel III mit einem Amin der allgemeinen Formeln IV, V und XIV erfolgen in Gegenwart oder Abwesenheit eines Loesungsmittels zwischen 0°C und 150°C, bevorzugt zwischen 20°C und 50°C. Als Loesungsmittel koennen z.B. Methanol, Ethanol, Propanol, iso-Propanol, Benzol, Toluol oder Dimethylformamid verwendet werden. Das molare Verhaeltnis der Reaktionspartner ist nicht kritisch. Es koennen Verhaeltnisse zwischen 1 und 100 gewaehlt werden.

Eine maskierte $-(NH_2)_{mask.}$-Gruppe in Verbindungen der allgemeinen Formeln V, VI, X und XII kann beispielsweise eine acylierte, vorzugsweise acetylierte, Gruppe -NH-Acyl, eine durch 1 oder 2 Benzylgruppen geschuetze Gruppe $-NH-C_7H_7$ oder $-N(C_7H_7)_2$, eine Nitro-Gruppe $-NO_2$ sein oder auch mit dem benachbarten C-Atom zusammen eine -C≡N-Gruppe bilden. Die Demaskierung der -NH-Acyl-Gruppe erfolgt vorzugsweise mit anorganischen Saeuren oder Basen, z.B. Salzsaeure oder Natronlauge, in Gegenwart oder Abwesenheit eines organischen Loesungsmittels, z.B. Methanol oder Ethanol, bei Temperaturen zwischen Raumtemperatur und 150°C, die Molverhaeltnisse koennen zwischen 1 und 200 liegen. Die Abspaltung von Benzyl-Schutzgruppen erfolgt hydrogenolytisch in Gegenwart eines organischen Loesungsmittels und/oder Wasser sowie Palladium auf Kohle als Katalysator. Die Temperaturen koennen zwischen Raumtemperatur und 250°C, vorzugsweise Raumtemperatur und 60°C, liegen, der Wasserstoffdruck kann zwischen 1 und 300 bar vorzugsweise 1 bis 5 bar betragen. Zur Reduktion der $-NO_2$-Gruppe kommen zahlreiche Verfahren, z.B. mit Zink in Salzsaeure, mit Eisen in Salzsaeure, mit Lithiumaluminiumhydrid in Ethern, mit anorganischen Sulfiden wie NaHS, $(NH_4)_2S$ oder $Na_2S_2O_4$ in waessriger und/oder alkoholischer Loesung, oder hydrogenolytisch mit Katalysatoren wie $PtO_2$, Pd, Raney-Nickel, vorzugsweise in einem alkoholischen Loesungsmittel wie Methanol und Ethanol, bei Drucken zwischen 1 und 200 bar, in Frage. Die Temperaturen koennen zwischen -20°C und +200°C, vorzugsweise zwischen Raumtemperatur und 100°C, liegen. Die Reduktion der C≡N-Gruppe zur $-CH_2-NH_2$-Gruppe kann z.B. mit Lithiumaluminiumhydrid in Ethern, Diboran in Ethern oder hydrogenolytisch mit Katalysatoren wie Raney-Nickel oder $PtO_2$ in organischen Loesungsmitteln und/oder Wasser bei Drucken zwischen 1 und 300 bar erfolgen. Die Temperaturen koennen zwischen -30°C und +200°C liegen.

Die Umsetzungen der Amine, V,XIII und XVIII oder demaskierten Amine ($-(NH_2)_{mask.} = -NH_2$) VI mit aktivierten Verbindungen der allgemeinen Formeln VII oder XV werden in einem organischen Loesungsmittel wie Hexan, Diethylether, Tetrahydrofuran, Methylenchlorid, Benzol, Toluol oder Dimethylformamid oder in waessriger Loesung bei Temperaturen zwischen -50°C und +100°C, bevorzugt zwischen -30°C und Raumtemperatur, durchgefuehrt.

Im Fall

a) X = Bindung

koennen die entsprechenden aktivierten Carbonsaeuren z.B. in Form von Estern, Lactonen, Carbonsaeurehalogeniden oder -anhydriden vorliegen, oder die Aktivierung der Carbonsaeuren erfolgt durch aktivierende Reagenzien wie N,N'-Carbonyldiimidazol,N,N'-Dicyclohexylcarbodiimid, 1-Ethyl-3-(3-dimethylaminopropyl)carbodiimid, 1-Alkyl-2-halogen-pyridiniumsalzen o.ä.. Die Aktivierung und die Umsetzung mit den Aminen V, XIII und XVIII oder den demaskierten Aminen VI kann dabei in einem Syntheseschritt durchgefuehrt werden. Es koennen molare Verhaeltnisse zwischen 1 und 10 gewaehlt werden. Gegebenenfalls koennen die Umsetzungen in Gegenwart einer Hilfsbase, vorzugsweise einem organischen Amin, durchgefuehrt werden,

b) X = Sauerstoffatom

koennen entsprechende Alkohole mit carbonylbildenden Reagenzien wie Phosgen, Chlorameisensaeureestern, Kohlensaeurediestern, N,N'-Carbonyldiimidazol o.ä. umgesetzt werden und die erhaltenen Zwischenprodukte mit den Aminen V, XIII und XVIII oder den demaskierten Aminen ($-(NH_2)_{mask.} = -NH_2$) VI zur Reaktion gebracht werden. Die Verknuepfung der Alkohole und der entsprechenden Amine zu den Urethanen kann dabei auch im Eintopfverfahren durchgefuehrt werden. Gegebenenfalls kann eine Hilfsbase, vorzugsweise ein organisches Amin, zugesetzt werden. Die Molverhaeltnisse zwischen den Alkoholen und carbonylbildenden Reagenzien einerseits und den Aminen V, XIII und XVIII oder den demaskierten Aminen VI andererseits koennen zwischen 1 und 10 liegen, vorzugsweise bei 1,

c) X = $-NR^1-$

koennen entsprechende Amine mit carbonylbildenden Reagenzien wie Phosgen, Harnstoff, Chlorameisensaeureestern, Kohlensaeurediestern, N,N'-Carbonyldiimidazol o.ä. umgesetzt werden und die erhaltenen Zwischenprodukte mit den Aminen V, XIII und XVIII oder den demaskierten Aminen ($-(NH_2)_{mask.} = -NH_2$) VI zur Reaktion gebracht werden. Die Harnstoffbildung kann auch im Eintopfverfahren durchgefuehrt werden. Gegebenenfalls kann eine Hilfsbase, vorzugsweise ein organisches Amin, zugesetzt werden. Anstatt eines Amins und des carbonylbildenden Reagenz' koennen auch entsprechende Isocyanate $O{=}C{=}N{-}B{-}(ONO_2)_n$ und $O{=}C{=}N{-}B(OH)_n$, wobei B und n die oben angegebenen Bedeutungen haben, ver wendet werden. Die Molverhaeltnisse zwischen den Isocyanaten bzw. den entsprechenden Aminen und den carbbonylbildenden Reagenzien einerseits und den Aminen V, XIII und XVIII oder den demaskierten Aminen ($-(NH_2)_{mask.} = -NH_2$) VI andererseits koennen zwischen 1 und 10 liegen, vorzugsweise bei 1.

Zur Herstellung von Verbindungen der allgemeinen Formel XIII setzt man Verbindungen der allgemeinen Formel VI($-(NH_2)_{mask.} = -NH_2$) mit bifunktionellen Siliciumverbindungen wie z.B. Dimethyldichlorsilan, Bis(acetyloxy)dimethylsilan, Bis(N-acetyl-N-methyl-amino)dimethylsilan, Bis(dimethylamino)-dimethylsilan, Bis(1,3-imidazol-1-yl)dimethylsilan oder 1,3-Dichloro-1,1,3,3-tetraisopropyldisiloxan in einem organischen Loesungsmittel, wie z.B. Diethylether, Tetrahydrofuran, 1,4-Dioxan, Benzol, Toluol oder Dimethylformamid in Gegenwart einer organischen Base, wie z.B. Triethylamin oder Imidazol, um. Die Temperaturen liegen zwischen -30°C und +100°C, vorzugsweise zwischen Raumtemperatur und 100°C. Die Molverhaeltnisse koennen zwischen 1 und 10 liegen. Nach der, gegebenenfalls erforderlichen, Ueberfuehrung der Gruppe Ar' in die Gruppe Ar wird die erhaltene Verbindung der allgemeinen Formel XIII mit aktivierten Verbindungen der allgemeinen Formel VII oder XV umgesetzt und die Schutzgruppe Sch durch Zugabe von Alkoholen, wie z.B. Methanol oder Isopropanol, oder Fluoridsalzen, wie z.B. Natriumfluorid, Tetra-n-butylammoniumfluorid o.ä. in einem organischen Loesungsmittel, wie z.B. Tetrahydrofuran oder Diethylether abgespalten. Die Abspaltung der Schutzgruppe Sch kann auch mit waessriger Ammoniakloesung oder mit Sauere/Wasser-Gemischen (z.B. Essigsaeure/Wasser 2:1) oder hydrogenolytisch ueber Pd/C erfolgen. Die Temperaturen liegen zwischen -20°C und 150°C, vorzugsweise zwischen 0°C und Raumtemperatur. Die Molverhaeltnisse koennen zwischen 1 und 100 liegen.

Die erfindungsgemaeßen Verbindungen der allgemeinen Formel I besitzen asymmetrische Kohlenstoffatome.

Gegenstand der Erfindung sind daher auch saemtliche moeglichen Diastereomerengemische, Racemate und saemtliche optisch aktiven Formen der erfindungsgemaeßen Verbindungen der allgemeinen Formel I.

Zur Uberfuehrung der Verbindungen der algemeinen Formel I in ihre pharmakologisch unbedenklichen Salze setzt man diese, vorzugsweise in einem organischen Loesungsmittel, mit der aequivalenten Menge einer anorganischen oder organischen Saeure, z.B. Salzsaeure, Bromwasserstoffsaeure, Salpetersaeure, Phosphorsaeure, Schwefelsaeure, Ameisensaeure, Essigsaeure, Propionsaeure, Oxalsaeure, Fumarsaeure, Maleinsaeure, Bernsteinsaeure, Adipinsaeure, Benzoesaeure, Salicylsaeure, o-Acetoxybenzoesaeure, Zimtsaeure, Naphthoesaeure, Mandelsaeure, Citronensaeure, Äpfelsaeure, Weinsaeure, Asparaginsaeure, Glutaminsaeure, Methansulfonsaeure oder p-Toluolsulfonsaeure um.

Die erfindungsgemaeße neue Substanz I und ihre Salze koennen in fluessiger oder fester Form enteral oder parenteral appliziert werden. Als Injektionsmedium kommt vorzugsweise Wasser zur Anwendung, welches die bei Injektionsloesungen ueblichen Zusaetzte wie Stabilisierungsmittel, Loesungsvermittler oder Puffer enthaelt. Derartige Zusaetze sind z.B. Tartrat- und Citratpuffer, Ethanol, Komplexbildner (wie Ethylendiamintetraessigsaeure und deren nicht toxische Salze), hochmolekulare Polymere (wie fluessiges Polyethylenoxid) zur Viskositaetsregulierung. Feste Traegerstoffe sind z.B. Staerke, Lactose, Mannit, Methylcellulose, Talkum, hochdisperse Kieselgelsaeuren, hoehermolekulare Fettsaeuren (wie Stearinsaeure), Gelatine, Agar-Agar, Calciumphosphat, Magnesiumstearat, tierische und pflanzliche Fette und feste hochmolekulare Polymere (wie z.B. Polyethylenglykole), fuer orale Applikation geeignete Zubereitungen koennen gewuenschtenfalls Geschmacks- und Sueßstoffe enthalten.

Neben den nachfolgend aufgefuehrten Beispielen sind insbesondere die folgenden Verbindungen im Sinne der Anmeldung bevorzugt.

1-(1-Naphthyloxy)-3-[2-(3-nitroxy-butanoylamino)-ethylamino]-2-propanol
1-(2-Methyl-indol-4-yloxy)-3-[2-(3-nitroxy-butanoylamino)-ethylamino]-2-propanol
1-(2-Allylphenoxy)-3-[2-(2,2-dimethyl-3-nitroxy-propanoylamino)ethylamino]-2-propanol
3-[2-(2,2-Dimethyl-3-nitroxy-propanoylamino)-ethylamino]-1-(indol-4-yloxy)-2-propanol
3-[2-(2,2-Dimethyl-3-nitroxy-propanoylamino)-ethylamino]-1-(2-methyl-indol-4-yloxy)-2-propanol
1-(4-Carbamoylmethyl-phenoxy)-3-[2-(4-nitroxy-butanoylamino)-ethylamino]-2-propanol
1-(4-Hydroxy-2,3,5-trimethyl-phenoxy)-3-[2-(4-nitroxy-butanoylamino)ethylamino]-2-propanol
3-[1,1-Dimethyl-2-(4-nitroxy-butanoylamino)ethylamino]-1-(2-methyl-indol-4-yloxy)-2-propanol
3-[1,1-Dimethyl-2-(4-nitroxy-butanoylamino)ethylamino]-1-(6-methyl-indol-4-yloxy)-2-propanol
1-(2-Cyano-indol-4-yloxy)-3-[1,1-dimethyl-2-(4-nitroxy-butanoylamino)ethylamino]-2-propanol
1-(1-Naphthyloxy)-3-[2-(4-nitroxy-pentanoylamino)ethylamino]-2-propanol
1-(2-Methyl-indol-4-yloxy)-3-[2-(4-nitroxy-pentanoylamino)ethylamino]-2-propanol
1-(1,2,3,4-Tetrahydro-7-methyl-2-oxo-chinolin-5-yloxy)-3-[2-(4-nitroxy-pentanoylamino)ethylamino]-2-propanol
1-(2-Allylphenoxy)-3-[2-(5-nitroxy-pentanoylamino)ethylamino]-2-propanol
1-[4-(2-Methoxy-ethyl)phenoxy]-3-[2-(5-nitroxy-pentanoylamino)ethylamino]-2-propanol
1-(2-Cyclopentyl-phenoxy)-3-[2-(5-nitroxy-pentanoylamino)ethylamino]-2-propanol
1-(2-Allyloxy-phenoxy)-3-[2-(5-nitroxy-pentanoylamino)ethylamino]-2-propanol
1-(4-Methylcarbonyloxy-2,3,5-trimethyl-phenoxy)-3-[2-(5-nitroxy-pentanoylamino)ethylamino]-2-propanol
1-(4-Hydroxy-2,3,5,-trimethyl-phenoxy)-3-[2-(5-nitroxy-pentanoylamino)ethylamino]-2-propanol
1-(3-Cyano-6-methyl-indol-4-yloxy)-3-[2-(5-nitroxy-pentanoylamino)ethylamino]-2-propanol
1-(1,2-Dihydro-2-oxo-chinolin-5-yloxy)-3-[2-(5-nitroxy-pentanoylamino)ethylamino]-2-propanol
1-(1,2-Dihydro-7-methyl-2-oxo-chinolin-5-yloxy)-3-[2-(5-nitroxy-pentanoylamino)ethylamino]-2-propanol
1-(2,6-Dimethyl-indol-4-yloxy)-3-[2-(5-nitroxy-pentanoylamino)ethylamino]-2-propanol
1-(2-Allylphenoxy)-3-[2-[(t-3, c-4-dinitroxy-cyclohexyl)-r-1-carbonylamino]ethylamino]-2-propanol
1-(2-Cyano-phenoxy)-3-[2-[(t-3,c-4-dinitroxy-cyclohexyl)-r-1-carbonylamino]ethylamino]-2-propanol
3-[2-[(t-3,c-4-Dinitroxy-cyclohexyl)-r-1-carbonylamino]ethylamino]-1-(indol-4-yloxy)-2-propanol
3-[2-[(t-3,c-4-Dinitroxy-cyclohexyl)-r-1-carbonylamino]ethylamino]-1-(2-methyl-indol-4-yloxy)-2-propanol
trans-1-[4-(n-Butanoylamino)-2-methylcarbonyl-phenoxy]-3-[2-[(2-nitroxycyclohexyl)acetylamino]ethylamino]-2-propanol
trans-1-(2-Methyl-indol-4-yloxy)-3-[2-[(2-nitroxycyclohexyl)acetylamino]-ethylamino]-2-propanol
trans-1-(6-Methyl-indol-4-yloxy)-3-[2-[(2-nitroxycyclohexyl)acetylamino]ethylamino]-2-propanol
trans-1-(3-Cyano-6-methyl-indol-4-yloxy)-3-[2-[(2-nitroxycyclohexyl)acetylamino]ethylamino]-2-propanol
trans-1-(1,2,3,4-Tetrahydro-7-methyl-2-oxo-chinolin-5-yloxy)-3-[2-[(2-nitroxycyclohexyl)acetylamino]ethylamino]-2-propanol
trans-1-(Carbazol-4-yloxy)-3-[2-[(2-nitroxycyclohexyl)acetylamino]ethylamino]-2-propanol
1-(2-Chloro-5-methyl-phenoxy]-3-[2-(2-methyl-4-nitroxy-butanoylamino)ethylamino]-2-propanol
1-(4-Methylcarbonyloxy-2,3,5-trimethyl-phenoxy)-3-[2-(2-methyl-4-nitroxy-butanoylamino)ethylamino]-2-propanol
1-(4-Hydroxy-2,3,5-trimethyl-phenoxy)-3-[2-(2-methyl-4-nitroxy-butanoylamino)ethylamino]-2-propanol
1-(6-Methyl-indol-4-yloxy)-3-[2-(2-methyl-4-nitroxy-butanoylamino)ethylamino]-2-propanol
1-(1,2,3,4-Tetrahydro-2-oxo-chinolin-5-yloxy)-3-[2-(2-methyl-4-nitroxy-butanoylamino)ethylamino]-2-propanol
1-(2-Methyl-indol-4-yloxy)-3-[2-(5-nitroxy-hexanoylamino)ethylamino]-2-propanol

1-[4-(2-Methoxy-ethyl)phenoxy]-3-[2-[(2-nitroxy-ethoxy)acetylamino]ethylamino]-2-propanol
1-(2-Cyclopentyl-phenoxy)-3-[2-[(2-nitroxy-ethoxy)acetylamino]ethylamino]-2-propanol
1-(2-Cyano-phenoxy)-3-[2-[(2-nitroxy-ethoxy)acetylamino]ethylamino]-2-propanol
1-(2-Chloro-5-methyl-phenoxy)-3-[2-[(2-nitroxy-ethoxy)acetylamino]ethylamino-2-propanol
1-(4-Methylcarbonyloxy-2,3,5-trimethyl-phenoxy)-3-[2-[(2-nitroxy-ethoxy)acetylamino]ethylamino]-2-propanol
1-(4-Hydroxy-2,3,5,-trimethyl-phenoxy)-3-[2-[(2-nitroxy-ethoxy)acetylamino]ethylamino]-2-propanol
1-(1-Naphthyloxy)-3-[2-[(2-nitroxy-ethoxy)acetylamino]ethylamino]-2-propanol
1-(Indol-4-yloxy)-3-[2-[(2-nitroxy-ethoxy)acetylamino]ethylamino]-2-propanol
1-(2-Methyl-indol-4-yloxy)-3-[2-[(2-nitroxy-ethoxy)acetylamino]ethylamino]-2-propanol
1-(6-Methyl-indol-4-yloxy)-3-[2-[(2-nitroxy-ethoxy)acetylamino]ethylamino]-2-propanol
1-(2-Cyano-indol-4-yloxy)-3-[2-[(2-nitroxy-ethoxy)acetylamino]ethylamino]-2-propanol
1-(3-Cyano-6-methyl-indol-4-yloxy)-3-[2-[(2-nitroxy-ethoxy)acetylamino]ethylamino]-2-propanol
3-[2-[(2-Nitroxy-ethoxy)acetylamino]ethylamino]-1-(2-oxo-indolin-4-yloxy)-2-propanol
1-(1,2,3,4-Tetrahydro-2-oxo-chinolin-5-yloxy)-3-[2-[(2-nitroxy-ethoxy)acetylamino]ethylamino]-2-propanol
1-(1,2-Dihydro-2-oxo-chinolin-5-yloxy)-3-[2-[(2-nitroxy-ethoxy)acetylamino]ethylamino]-2-propanol
1-(3-Methyl-benzimidazol-4-yloxy)-3-[(2-nitroxy-ethoxy)acetylamino]ethylamino]-2-propanol
1-(2,6-Dimethyl-indol-4-yloxy)-3-[2-[(2-nitroxy-ethoxy)acetylamino]ethylamino]-2-propanol
1-(2-Allylphenoxy)-3-[2-[(2-nitroxy-ethylmercapto)acetylamino]ethylamino]-2-propanol
1-[4-(2-Methoxy-ethyl)phenoxy]-3-[2-[(2-nitroxy-ethylmercapto)acetylamino]ethylamino]-2-propanol
1-(2-Cyclopentyl-phenoxy)-3-[2-[(2-nitroxy-ethylmercapto)acetylamino]ethylamino]-2-propanol
1-(2-Cyano-phenoxy)-3-[2-[(2-nitroxy-ethylmercapto)acetylamino]ethylamino]-2-propanol
1-(4-Methylcarbonyloxy-2,3,5-trimethyl-phenoxy)-3-[2-[(2-nitroxy-ethylmercapto)acetylamino]ethylamino]-2-propanol
1-(4-Hydroxy-2,3,5-trimethyl-phenoxy)-3-[2-[(2-nitroxy-ethylmercapto)acetylamino]ethylamino]-2-propanol
1-(1-Naphthyloxy)-3-[2-[(2-nitroxy-ethylmercapto)acetylamino]ethylamino]-2-propanol
1-(Indol-4-yloxy)-3-[2-[(2-nitroxy-ethylmercapto)acetylamino]ethylamino]-2-propanol
1-(6-Methyl-indol-4-yloxy)-3-[2-[(2-nitroxy-ethylmercapto)acetylamino]ethylamino]-2-propanol
1-(2-Cyano-indol-4-yloxy)-3-[2-[(2-nitroxy-ethylmercapto)acetylamino]ethylamino]-2-propanol
1-(3-Cyano-indol-4-yloxy)-3-[2-[(2-nitroxy-ethylmercapto)acetylamino]ethylamino]-2-propanol
1-(3-Cyano-6-methyl-indol-4-yloxy)-3-[2-[(2-nitroxy-ethylmercapto)acetylamino]ethylamino]-2-propanol
3-[2-[l2-Nitroxy-ethylmercapto)acetylamino]ethylamino]-1-(2-oxo-indolin-4-yloxy)-2-propanol
1-(1,2,3,4-Tetrahydro-2-oxo-chinolin-5-yloxy)-3-[2-[(2-nitroxy-ethylmercapto)acetylamino]ethylamino]-2-propanol
1-(1,2-Dihydro-2-oxo-chinolin-5-yloxy)-3-[2-[(2-nitroxy-ethylmercapto)acetylamino]ethylamino]-2-propanol
1-(1,2-Dihydro-7-methyl-2-oxo-chinolin-5-yloxy)-3-[2-[(2-nitroxy-ethylmercapto)acetylamino]ethylamino-2-propanol
1-(3-Methyl-benzimidazol-4-yloxy)-3-[2-[(2-nitroxy-ethylmercapto)acetylamino]ethylamino]-2-propanol
1-(2,6-Dimethyl-indol-4-yloxy)-3-[2-[(2-nitroxy-ethylmercapto)acetylamino]ethylamino]-2-propanol
1-(2-Cyano-phenoxy)-3-[2-[2-(2-nitroxy-ethylmercapto)propanoylamino]ethylamino]-2-propanol
1-(4-Hydroxy-2,3,5-trimethyl-phenoxy)-3-[2-[2-(2-nitroxy-ethylmercapto)propanoylamino]ethylamino]-2-propanol
1-(2-Methyl-indol-4-yloxy)-3-[2-[2-(2-nitroxy-ethylmercapto)propanoylamino]ethylamino]-2-propanol
1-(1,2,3,4-Tetrahydro-7-methyl-2-oxo-chinolin-5-yloxy)-3-[2-[2-(2-nitroxy-ethylmercapto)propanoylamino]ethylamino-2-propanol
trans-1-(2-Chloro-5-methyl-phenoxy)-3-[2-[(2-nitroxy-cyclopentylmercapto)acetylamino]ethylamino]-2-propanol
trans-1-(6-Methyl-indol-4-yloxy)-3-[2-[(2-nitroxy-cyclopentylmercapto)acetylamino]ethylamino]-2-propanol
trans-1-(1,2-Dihydro-2-oxo-chinolin-5-yloxy)-3-[2-[(2-nitroxy-cyclopentylmercapto)acetylamino]ethylamino]-2-propanol
trans-1-(2,6-Dimethyl-indol-4-yloxy)-3-[2-[(2-nitroxy-cyclohexylmercapto)acetylamino]ethylamino]-2-propanol
trans-1-(2-Allylphenoxy)-3-[2-[(2-nitroxy-cyclohexylmercapto)acetylamino]ethylamino]-2-propanol
trans-1-(2-Allyloxy-phenoxy)-3-[2-[(2-nitroxy-cyclohexylmercapto)acetylamino]ethylamino]-2-propanol
trans-1-(1-Naphthyloxy)-3-[2-[(2-nitroxy-cyclohexylmercapto)acetylamino]ethylamino]-2-propanol
trans-1-(Indol-4-yloxy)-3-[2-[(2-nitroxy-cyclohexylmercapto)acetylamino]ethylamino]-2-propanol
trans-1-(2-Methyl-indol-4-yloxy)-3-[2-[(2-nitroxy-cyclohexylmercapto)acetylamino]ethylamino]-2-propanol

trans-3-[2-[(2-Nitroxy-cyclohexylmercapto)acetylamino]ethylamino]-1-(2-oxo-indolin-4-yloxy)-2-propanol
trans-1-(1,2,3,4-Tetrahydro-7-methyl-2-oxo-chinolin-5-yloxy)-3-[2-[(2-nitroxy-cyclohexylmercapto)acetylamino]ethylamino]-2-propanol
1-(Carbazol-4-yloxy)-3-[2-(4-nitroxy-butanoylamino)ethylamino]-2-propanol
1-(2-Hydroxymethyl-indol-4-yloxy)-3-[2-(4-nitroxy-butanoylamino)ethylamino]-2-propanol
1-(2-Allyloxy-phenoxy)-3-[1,1-dimethyl-2-(2,2-dimethyl-3-nitroxy-propanoylamino)ethylamino]-2-propanol
1-[4-(n-Butanoylamino)-2-methylcarbonyl-phenoxy]-3-[1,1-dimethyl-2-(2,2-dimethyl-3-nitroxy-propanoylamino)ethylamino]-2-propanol
1-[4-(n-Butanoylamino)-2-methylcarbonyl-phenoxy]-3-[1,1-dimethyl-2-(4-nitroxy-butanoylamino)ethylamino]-2-propanol
1-[4-(n-Butanoylamino)-2-methylcarbonyl-phenoxy]-3-[2-(2,2-dimethyl-3-nitroxy-propanoylamino)ethylamino]-2-propanol
3-[1,1-Dimethyl-2-(2,2-dimethyl-3-nitroxy-propanoylamino)ethylamino]-1-(2-oxo-indolin-4-yloxy)-2-propanol
1-(2-Cyclopentylphenoxy)-3-[1,1-dimethyl-2-[(5-0-nitro-isosorbid-2-yloxy)carbonylamino]ethylamino]-2-propanol
1-(2-Allylphenoxy)-3-[1,1-dimethyl-2-(3-nitroxy-butanoylamino)ethylamino]-2-propanol
1-(2-Allylphenoxy)-3-[1,1-dimethyl-2-(4-nitroxy-pentanoylamino)ethylamino]-2-propanol
1-(2-Allylphenoxy)-3-[1,1-dimethyl-2-(5-nitroxy-pentanoylamino)ethylamino]-2-propanol
trans-1-[4-(2-Methoxyethyl)phenoxy]-3-[1,1-dimethyl-2-[(2-nitroxycyclohexyl)acetylamino]ethylamino]-2-propanol
1-[4-(2-Methoxyethyl)phenoxy]-3-[1,1-dimethyl-2-(5-nitroxy-pentanoylamino)ethylamino]-2-propanol
trans-1-(2-Cyclopentylphenoxy)-3-[1,1-dimethyl-2-[(2-nitroxy-cyclohexyl)acetylamino]ethylamino]-2-propanol
1-(2-Cyclopentylphenoxy)-3-[1,1-dimethyl-2-(5-nitroxy-pentanoylamino)ethylamino]-2-propanol
trans-3-[1,1-Dimethyl-2-[(4-nitroxy-cyclohexyl)carbonylamino]ethylamino]-1-[2-(2-norbornylexo)phenoxy]-2-propanol
3-[1,1-Dimethyl-2-(5-nitroxy-pentanoylamino)ethylamino-1-[2-norbornylexo-phenoxy]-2-propanol
3-[1,1-Dimethyl-2-(4-nitroxy-butanoylamino)ethylamino]-1-[2-(N-methyl-amino-carbonyl-methoxy)phenoxy]-2-propanol
trans-3-[1,1-Dimethyl-2-[(2-nitroxy-cyclohexyl)acetylamino]ethylamino]-1-[2-(N-methyl-amino-carbonyl-methoxy)phenoxy]-2-propanol
trans-3-[1,1-Dimethyl-2-[(4-nitroxy-cyclohexyl)carbonylamino]ethylamino]-1-[2-(N-methyl-amino-carbonyl-methoxy)phenoxy]-2-propanol
3-[1,1-Dimethyl-2-(5-nitroxy-pentanoylamino)ethylamino]-1-[2-(N-methyl-amino-carbonyl-methoxy)phenoxy]-2-propanol
trans-1-(2-Allyloxy-phenoxy)-3-[1,1-dimethyl-2-[(2-nitroxy-cyclohexyl)acetylamino]ethylamino]-2-propanol
trans-1-(2-Allyloxy-phenoxy)-3-[1,1-dimethyl-2-[(4-nitroxy-cyclohexyl)carbonylamino]ethylamino]-2-propanol
1-(2-Allyloxy-phenoxy)-3-[1,1-dimethyl-2-(5-nitroxy-pentanoylamino)ethylamino]-2-propanol
3-[1,1-Dimethyl-2-(4-nitroxy-butanoylamino)ethylamino]-1-(2-methylmercapto-phenoxy)-2-propanol
trans-3-[1,1-Dimethyl-2-[(4-nitroxy-cyclohexyl)carbonylamino]ethylamino]-1-(2-methylmercapto-phenoxy)-2-propanol
3-[1,1-Dimethyl-2-(5-nitroxy-pentanoylamino)ethylamino]-1-(2-methylmercapto-phenoxy)-2-propanol
trans-1-(2-Cyanophenoxy)-3-[1,1-dimethyl-2-[(2-nitroxy-cyclohexyl)acetylamino]ethylamino]-2-propanol
trans-1-(2-Cyanophenoxy)-3-[1,1-dimethyl-2-[(4-nitroxy-cyclohexyl)carbonylamino]ethylamino]-2-propanol
1-(2-Cyano-phenoxy)-3-[1,1-dimethyl-2-(3-nitroxy-butanoylamino)ethylamino]-2-propanol
1-(2-Cyano-phenoxy)-3-[1,1-dimethyl-2-(5-nitroxy-pentanoylamino)ethylamino]-2-propanol
1-(2,5-Dichloro-phenoxy)-3-[1,1-dimethyl-2-(4-nitroxy-butanoylamino)ethylamino]-2-propanol
1-(2-Cyano-phenoxy)-3-[1,1-dimethyl-2-[(2-nitroxyethoxy)acetylamino]ethylamino]-2-propanol
trans-1-(2,5-Dichloro-phenoxy)-3-[1,1-dimethyl-2-[(2-nitroxy-cyclohexyl)acetylamino]ethylamino]-2-propanol
trans-1-(2,5-Dichloro-phenoxy)-3-[1,1-dimethyl-2-[(4-nitroxycyclohexyl)carbonylamino]ethylamino]-2-propanol
1-(2,5-Dichloro-phenoxy)-3-[1,1-dimethyl-2-(5-nitroxy-pentanoylamino)ethylamino]-2-propanol
1-(2,5-Dichloro-phenoxy)-3-[1,1-dimethyl-2-[(2-nitroxy-ethoxy)acetylamino]ethylamino]-2-propanol
trans-1-(2-Chloro-5-methyl-phenoxy)-3-[1,1-dimethyl-2-[(4-nitroxy-cyclohexyl)carbonylamino]ethylamino]-2-propanol
1-(2-Chloro-5-methyl-phenoxy)-3-[1,1-dimethyl-2-(3-nitroxy-butanoylamino)ethylamino]-2-propanol
1-(2-Chloro-5-methyl-phenoxy)-3-[1,1-dimethyl-2-(4-nitroxy-pentanoylamino)ethylamino]-2-propanol

1-(2-Chloro-5-methyl-phenoxy)-3-[1,1-dimethyl-2-(5-nitroxy-pentanoylamino)ethylamino]-2-propanol
1-(2-Chloro-5-methyl-phenoxy)-3-[1,1-dimethyl-3-(4-nitroxy-butanoylamino)propylamino]-2-propanol
1-[4-(n-Butanoylamino)-2-cyano-phenoxy]-3-[2-(4-nitroxy-butanoylamino)ethylamino]-2-propanol
1-[4-(n-Butanoylamino)-2-cyano-phenoxy]-3-[1,1-dimethyl-2-(4-nitroxy-butanoylamino)ethylamino]-2-propanol
trans-1-[4-(n-Butanoylamino)-2-cyano-phenoxy]-3-[1,1-dimethyl-2-[(2-nitroxy-cyclohexyl)acetylamino]ethylamino]-2-propanol
trans-1-[4-(n-Butanoylamino)-2-cyano-phenoxy]-3-[1,1-dimethyl-2-[(4-nitroxy-cyclohexyl)carbonylamino]ethylamino]-2-propanol
1-[4-(n-Butanoylamino)-2-cyano-phenoxy]-3-[1,1-dimethyl-2-(5-nitroxy-pentanoylamino)ethylamino]-2-propanol
trans-3-[1,1-Dimethyl-2-[(2-nitroxy-cyclohexyl)acetylamino]ethylamino-1-(4-methylcarbonyloxy-2,3,5-trimethyl-phenoxy)-2-propanol
trans-1-(4-Methylcarbonyloxy-2,3,5-trimethyl-phenoxy)-3-[2-[(4-nitroxy-cyclohexyl)carbonylamino]ethylamino]-2-propanol
trans-3-[1,1-Dimethyl-2-[(4-nitroxy-cyclohexyl)carbonylamino]ethylamino]-1-(4-methylcabonyloxy-2,3,5-trimethyl-phenoxy)-2-propanol
3-[1,1-Dimethyl-2-(3-nitroxy-butanoylamino)ethylamino]-1-(4-methylcarbonyloxy-2,3,5-trimethyl-phenoxy)-2-propanol
3-[1,1-Dimethyl-2-(5-nitroxy-pentanoylamino)ethylamino]-1-(4-methylcarbonyloxy-2,3,5-trimethyl-phenoxy)-2-propanol
trans-3-[1,1-Dimethyl-2-[(2-nitroxy-cyclohexyl)acetylamino]ethylamino]-1-(4-hydroxy-2,3,5-trimethyl-phenoxy)-2-propanol
trans-3-[1,1-Dimethyl-2-[(4-nitroxy-cyclohexyl)carbonylamino]ethylamino]-1-(4-hydroxy-2,3,5-trimethyl-phenoxy)-2-propanol
3-[1,1-Dimethyl-2-(3-nitroxy-butanoylamino)ethylamino]-1-(4-hydroxy-2,3,5-trimethyl-phenoxy)-2-propanol
3-[1,1-Dimethyl-2-(4-nitroxy-pentanoylamino)ethylamino]-1-(4-hydroxy-2,3,5-trimethyl-phenoxy)-2-propanol
3-[1,1-Dimethyl-2-(5-nitroxy-pentanoylamino)ethylamino]-1-(4-hydroxy-2,3,5-trimethyl-phenoxy)-2-propanol
trans-3-[1,1-Dimethyl-2-[(2-nitroxy-cyclohexyl)acetylamino]ethylamino]-1-(1-naphthyloxy)-2-propanol
trans-3-[1,1-Dimethyl-2-[(4-nitroxy-cyclohexyl)carbonylamino]ethylamino]-1-naphthyloxy)-2-propanol
3-[1,1-Dimethyl-2-(4-nitroxy-pentanoylamino)ethylamino]-1-(1-naphthyloxy)-2-propanol
3-[1,1-Dimethyl-2-(5-nitroxy-pentanoylamino)ethylamino]-1-(1-naphthloxy)-2-propanol
3-[1,1-Dimethyl-3-(4-nitroxy-butanoylamino)propylamino]-1-(1-naphthyloxy)-2-propanol
trans-3-[1,1-Dimethyl-2-[(2-nitroxy-cyclohexyl)acetylamino]ethylamino]-1-(indol-4-yloxy)-2-propanol
1-(2-Cyano-indol-4-yloxy)-3-[2-(4-nitroxy-butanoylamino)ethylamino]-2-propanol
1-(2-Cyano-indol-4-yloxy)-3-[2-(5-nitroxy-pentanoylamino)ethylamino]-2-propanol
1-(2-Cyano-indol-4-yloxy)-3-[1,1-dimethyl-2-(5-nitroxy-pentanoylamino)ethylamino]-2-propanol
1-(2,2-Dimethyl-1,3-benzodioxol-4-yloxy)-3-[1,1-dimethyl-2-(4-nitroxy-butanoylamino)ethylamino]-2-propanol
3-[1,1-Dimethyl-2-(4-nitroxy-butanoylamino)ethylamino]-1-(2-formyl-1,2,3,4-tetrahydro-isochinolin-5-yloxy)-2-propanol
1-(1,2-Dihydro-2-oxo-chinolin-5-yloxy)-3-[1,1-dimethyl-2-(4-nitroxy-butanoylamino)ethylamino]-2-propanol
1-(1,2-Dihydro-2-oxo-chinolin-5-yloxy)-3-[1,1-dimethyl-2-(3-nitroxy-butanoylamino)ethylamino]-2-propanol
3-[2-(5-Nitroxy-pentanoylamino)ethylamino]-1-(1,2,3,4-tetrahydro-7-methyl-2-oxo-chinolin-5-yloxy)-2-propanol
1-(1,2-Diyhdro-7-methyl-2-oxo-chinolin-5-yloxy)-3-[1,1-dimethyl-2-(4-nitroxy-butanoylamino)ethylamino]-2-propanol
1-(1,2-Diyhdro-7-methyl-2-oxo-chinolin-5-yloxy)-3-[1,1-dimethyl-2-(5-nitroxy-pentanoylamino)-ethylamino]-2-propanol
1-(8-Acetylamino-2-oxo-1,2,3,4-tetrahydro-chinolin-5-yloxy)-3-[2-(4-nitroxy-butanoylamino)-ethylamino]-2-propanol
1-(8-Acetylamino-2-oxo-1,2,3,4-tetrahydro-chinolin-5-yloxy)-3-[2-(5-nitroxy-pentanoylamino)-ethylamino]-2-propanol
1-(8-Acetylamino-2-oxo-1,2,3,4-tetrahydro-chinolin-5-yloxy)-3-[1,1-dimethyl-2-(4-nitroxy-butanoylamino)ethylamino]-2-propanol
1-(3-Cyano-1,2-dihydro-2-oxo-chinolin-5-yloxy)-3-[2-(4-nitroxy-butanoylamino)ethylamino]-2-propanol
1-(3-Cyano-1,2-dihydro-2-oxo-chinolin-5-yloxy)-3-[1,1-dimethyl-2-(4-nitroxy-butanoylamino)ethylamino]-2-propanol

1-(3-Cyano-1,2-dihydro-2-oxo-chinolin-5-yloxy)-3-[1,1-dimethyl-2-(5-nitroxy-pentanoylamino)ethylamino]-2-propanol
1-(3-Cyano-1,2-dihydro-7-methyl-2-oxo-chinolin-5-yloxy)-3-[2-(4-nitroxy-butanoylamino)ethylamino]-2-propanol
1-(3-Cyano-1,2-dihydro-7-methyl-2-oxo-chinolin-5-yloxy)-3-[2-(5-nitroxy-pentanoylamino)ethylamino]-2-propanol
1-(3-Cyano-1,2-dihydro-7-methyl-2-oxo-chinolin-5-yloxy)-3-[1,1-dimethyl-2-(4-nitroxy-butanoylamino)ethylamino]-2-propanol
1-(3-Cyano-1,2-dihydro-7-methyl-2-oxo-chinolin-5-yloxy)-3-[1,1-dimethyl-2-(5-nitroxy-pentanoylamino)ethylamino]-2-propanol
1-(3,4-Dihydro-2H-3-oxo-1,4-benzthiazin-8-yloxy]-3-[2-(4-nitroxy-butanoylamino)ethylamino]-2-propanol
1-(3,4-Dihydro-2H-3-oxo-1,4-benzthiazin-8-yloxy)-3-[2-(5-nitroxy-pentanoylamino)ethylamino]-2-propanol
1-(3,4-Dihydro-2H-3-oxo-1,4-benzthiazin-8-yloxy)-3-[1,1-dimethyl-2-(4-nitroxy-butanoylamino)ethylamino]-2-propanol
1-(3,4-Dihydro-2H-3-oxo-1,4-benzthiazin-8-yloxy)-3-[1,1-dimethyl-2-(5-nitroxy-pentanoylamino)ethylamino]-2-propanol
1-(3-Cyano-2H-benzpyran-8-yloxy)-3-[2-(4-nitroxy-butanoylamino)ethylamino]-2-propanol
trans-1-(3-Cyano-2H-benzpyran-8-yloxy)-3-[2-[(4-nitroxy-cyclohexyl)carbonylamino]ethylamino]-2-propanol
1-(3-Cyano-2H-benzpyran-8-yloxy)-3-[2-(3-nitroxy-butanoylamino)ethylamino]-2-propanol
1-(3-Cyano-2H-benzpyran-8-yloxy)-3-[1,1-dimethyl-2-(4-nitroxy-butanoylamino)ethylamino]-2-propanol
1-(3-Cyano-2H-benzpyran-8-yloxy)-3-[1,1-dimethyl-2-(3-nitroxy-butanoylamino)ethylamino]-2-propanol
1-(3-Cyano-2H-benzpyran-8-yloxy)-3-[1,1-dimethyl-2-(5-nitroxy-pentanoylamino)ethylamino]-2-propanol
trans-1-(3-Methyl-benzimidazol-4-yloxy)-3-[2-[(4-nitroxy-cyclohexyl)carbonylamino]ethylamino]-2-propanol
trans-1-(3-Methyl-benzimidazol-4-yloxy)-3-[1,1-dimethyl-2-[(2-nitroxy-cyclohexyl)acetylamino]ethylamino]-2-propanol
1-(1,2-Dihydro-2-oxo-1,3-benzdiazin-5-yloxy)-3-[2-(4-nitroxy-butanoylamino)ethylamino]-2-propanol
1-(1,2-Dihydro-2-oxo-1,3-benzdiazin-5-yloxy)-3-[1,1-dimethyl-2-(4-nitroxy-butanoylamino)ethylamino]-2-propanol
trans-1-(1,2-Dihydro-2-oxo-1,3-benzdiazin-5-yloxy)-3-[1,1-dimethyl-2-[(2-nitroxy-cyclohexyl)acetylamino]ethylamino]-2-propanol
1-(3,4-Dihydro-2,2-dioxo-1,2-benzoxathiin-5-yloxy)-3-[2-(4-nitroxy-butanoylamino)ethylamino]-2-propanol
1-(3,4-Dihydro-2,2-dioxo-1,2-benzoxathiin-5-yloxy)-3-[1,1-dimethyl-2-(4-nitroxy-butanoylamino)ethylamino]-2-propanol
1-(3,4-Dihydro-2,2-dioxo-1,2-benzoxathiin-8-yloxy)-3-[1,1-dimethyl-2-(4-nitroxy-butanoylamino)ethylamino]-2-propanol
1-(3,4-Dihydro-2,2-dioxo-1H-1-methyl-2,1-benzthiazin-5-yloxy)-3-[2-(4-nitroxy-butanoylamino)ethylamino]-2-propanol
1-(3,4-Dihydro-2,2-dioxo-1H-1-methyl-2,1-benzthiazin-5-yloxy)-3-[1,1-dimethyl-2-(4-nitroxy-butanoylamino)ethylamino]-2-propanol
trans-1-(Carbazol-4-yloxy)-3-[2-[(4-nitroxy-cyclohexyl)carbonylamino]ethylamino]-2-propanol
3-[1,1-Dimethyl-2-(4-nitroxy-butanoylamino)ethylamino]-1-(1-oxo-indan-7-yloxy)-2-propanol
3-[1,1-Dimethyl-2-(3-nitroxy-butanoylamino)ethylamino]-1-(1-oxo-indan-7-yloxy)-2-propanol
3-[1,1-Dimethyl-2-(5-nitroxy-pentanoylamino)ethylamino]-1-(1-oxo-indan-7-yloxy)-2-propanol
3-[1,1-Dimethyl-2-(4-nitroxy-butanoylamino)ethylamino]-1-(2-methoxy-phenoxy)-2-propanol
trans-3-[1,1-Dimethyl-2-[(2-nitroxy-cyclohexyl)acetylamino]ethylamino]-1-(2-methoxy-phenoxy)-2-propanol
3-[1,1-Dimethyl-2-(3-nitroxy-butanoylamino)ethylamino]-1-(2-methoxy-phenoxy)-2-propanol
3-[1,1-Dimethyl-2-(5-nitroxy-pentanoylamino)ethylamino]-1-(2-methoxy-phenoxy)-2-propanol
3-[1,1-Dimethyl-2-(4-nitroxy-butanoylamino)ethylamino]-1-(2-propargyloxy-phenoxy)-2-propanol
3-[1,1-Dimethyl-2-(4-nitroxy-butanoylamino)ethylamino]-1-(2-methyl-phenoxy)-2-propanol
3-[1,1-Dimethyl-2-(4-nitroxy-butanoylamino)ethylamino]-1-(3-methyl-phenoxy)-2-propanol
3-[1,1-Dimethyl-2-(4-nitroxy-butanoylamino)ethylamino]-1-(1,2, 3,4-tetrahydro-1-oxo-5-naphthyloxy)-2-propanol
3-[1,1-Dimethyl-2-(4-nitroxy-butanoylamino)ethylamino]-1-(3,4-dihydro-2H-benzthiin-8-yloxy)-2-propanol
1-(1,2-Benzthiazol-4-yloxy)-3-[1,1-dimethyl-2-(4-nitroxy-butanoylamino)ethylamino]-2-propanol
1-(1,3-Benzthiazol-7-yloxy)-3-[1,1-dimethyl-2-(4-nitroxy-butanoylamino)ethylamino]-2-propanol

trans-1-(2-Cyano-phenoxy)-3-[1,1-dimethyl-2-[(2-nitroxy-cyclohexyloxy)carbonylamino]ethylamino]-2-propanol
1-(2-Cyano-phenoxy)-3-[1,1-dimethyl-2-[(4-nitroxymethylpiperidin-1-yl)carbonylamino]ethylamino]-2-propanol
3-[1,1-Dimethyl-2-[[4-(1-nitroxyethyl)-piperidin-1-yl]carbonylamino]ethylamino]-1-(4-methyl-carbonyloxy-2,3,5-trimethylphenoxy)-2-propanol
3-[1,1-Dimethyl-2-[(4-nitroxymethyl-piperidin-1-yl)carbonylamino]ethylamino]-1-(2-methoxy-phenoxy)-2-propanol
trans-1-(2,5-Dichloro-phenoxy)-3-[1,1-dimethyl-2-(2-nitroxy-cyclohexylamino)ethylamino]-2-propanol
1-(2H-3,4-Dihydro-benzopyran-8-yloxyl)-3-[2-(4-nitroxy-butanoylamino)ethylamino] -2-propanol
1-(2H-3,4-Dihydro-benzopyran-8-yloxy)-3-[ 1,1-dimethyl-2-(4-nitroxy-butanoylamino)ethylamino] -2-propanol
trans-1-(2H-3,4-Dihydro-benzopyran-8-yloxy)-3-[1,1-dimethyl-2-[ (4-nitroxycyclohexyl) carbonylamino]ethylamino ]-2-propanol
1-(3-Methyl-1,2-benzisoxazol-4-yloxy)-3-[2-(4-nitroxy-butanoylamino)ethylamino] -2-propanol
3-[ 1,1-Dimethyl-2-(4-nitroxybutanoylamino)-ethylamino] -1- (3-methyl-1,2-benzisoxazol-4-yloxy)-2-propanol

Die Beispiele 1, 2, 7, 8 und 11–14 erläutern die Herstellung von erfindungsgemäßen Verbindungen; die Beispiele 3–6, 9 und 10 erläutern die Herstellung von Zwischenprodukten.

Beispiel 1

1-(3-Cyano-indol-4-yloxy)-3-[2-(3-nitroxy-butanoylamino)ethylamino]-2-propanol fumarat

4.2 g 1-(3-Cyano-indol-4-yloxy)-2,3-epoxy-propan (EP 0 045 910) werden in 100 ml n-Butanol suspendiert, mit 11.5 g 2-(3-Nitroxy-butanoylamino)ethylamin versetzt und 2 d bei Raumtemperatur geruehrt. Nach Entfernen des Loesungsmittels wird an Kieselgel mit Methylenchlorid/Methanol (95:5) und Methylenchlorid/methanolischem Ammoniak (95:5) chromatographiert. Aus den entsprechenden Fraktionen erhaelt man nach Entfernen des Loesungsmittels 4.6 g Base. Durch Loesen in Methanol und Zugabe der berechneten Menge Fumarsaeure werden nach Absaugen 3.9 g (40 % d.Th.) Fumarat vom Schmp. 155-156°C erhalten.

Beispiel 2

In analoger Weise, wie in Beispiel 1 beschrieben. erhaelt man aus 1-Aryloxy-2,3-epoxy-propanen bzw. 1-Hetaryloxy-2,3-epoxy-propanen (siehe z.B. EP 0 045 910; DE-OS 33 10 891; DE-OS 25 08 251; EP-OS 0 014 951; DE-OS 25 03222; DE-AS 1 620 342; DE-OS 26 26 890; DE-OS 23 62 278; J. Med. Chem. 17 (1974), 529; J. Med. Chem. 26 (1983), 1570; WHO Chronicle 26, (1972), 1125; DE-OS 27 35 570; Chem. Pharm. Bull. 20 (1972), 905; EP-OS 0 095 454) und Nitroxy-alkanoylamino-alkylaminen folgende Verbindungen:

| | Bezeichnung | Ausb. % | Schmelzpunkt °C (Lösungsmittel) |
|---|---|---|---|
| 1. | 3-[2-(3-Nitroxy-butanoylamino)-ethylamino]-1-(2-oxo-indolin-4-yloxy)-2-propanol hemifumarat<br>aus<br>2,3-Epoxy-1-(2-oxo-indolin-4-yloxy)-propan<br>und<br>2-(3-Nitroxy-butanoylamino)ethylamin | 27 | 165–167 (Ethanol) |
| 2. | 1-(2-Allylphenoxy)-3-[2-(4-nitroxy-butanoyamino)ethylamino]-2-propanol<br>aus<br>1-(2-Allylphenoxy)-2,3-epoxy-propan<br>und<br>2-(4-Nitroxy-butanoylamino)ethyl-amin | 18 | 117–119 (Ethylacetat) |
| 3. | 1-[4-(2-Methoxy-ethyl)phenoxy]-3-[2-(4-nitroxy-butanoylamino)ethyl-amino]-2-propanol tropat<br>aus<br>2,3-Epoxy-1-[4-(2-methoxy-ethyl)-phenoxy]propan<br>und<br>2-(4-Nitroxy-butanoylamino)ethylamin | 10 | 88–90 (Ethylacetat-/Diethylether) |
| 4. | 1-(2-Cyclopentyl-phenoxy)-3-[2-(4-nitroxy-butanoylamino)ethyl-amino]-2-propanol citrat<br>aus<br>1-(2-Cyclopentyl-phenoxy)-2,3-epoxypropan<br>und<br>2-(4-Nitroxy-butanoylamino)-ethylamin | 14 | 123–125 (Aceton/Diethyl-ether 1:1) |
| 5. | 1-(2-Allyloxy-phenoxy)-3-[2-(4-nitroxy-butanoylamino)ethylamino]-2-propanol<br>aus<br>1-(2-Allyloxy-phenoxy)-2,3-epoxy-propan<br>und<br>2-(4-Nitroxy-butanoylamino)ethylamin | 11 | Öl |
| 6. | 1-(2-Cyano-phenoxy)-3-[2-(4-nitroxy-butanoylamino)ethylamino]-2-propanol<br>aus<br>1-(2-Cyano-phenoxy)-2,3-epoxypropan<br>und<br>2-(4-Nitroxy-butanoylamino)-ethylamin | 20 | 108–110 (Ethylacetat) |
| 7. | 1-(2-Chloro-5-methyl-phenoxy)-3-[2-(4-nitroxy-butanoylamino)-ethylamino]-2-propanol<br>aus<br>1-(2-Chloro-5-methyl-phenoxy)-2,3-epoxy-propan<br>und<br>2-(4-Nitroxy-butanoylamino)-ethylamin | 30 | 98–100 (Ethylacetat) |
| 8. | 1-[4-(n-Butanoylamino)-2-methyl-carbonyl-phenoxy]-3-[2-(4-nitroxy-butanoylamino)ethylamino]-2-propanol<br>aus<br>1-[4-n-Butanoylamino)-2-methyl-carbonyl-phenoxy]-2,3-epoxy-propan<br>und<br>2-(4-Nitroxy-butanoylamino)-ethylamin | 10 | Öl |
| 9. | 1-(4-Methylcarbonyloxy-2,3,5-trimethyl-phenoxy)-3-[2-(4-nitroxy-butanoylamino)ethylamino]-2-propanol<br>aus<br>2,3-Epoxy-1-(4-methylcarbonyloxy-2,3,5-trimethyl-phenoxy)-propan<br>und<br>2-(4-Nitroxy-butanoylamino)-ethylamin | 47 | 105–107 (n-Butanol) |
| 10. | 1(1-Naphthyloxy)-3-[2-(4-nitroxy-butanoylamino)ethylamino]-2-propanol cyclaminat<br>aus<br>2,3-Epoxy-1-(1-naphthyloxy)propan<br>und<br>2-(4-Nitroxy-butanoylamino)ethylamin | 15 | 114–116 (Ethylacetat) |

Beispiel 2 (Fortsetzung)

| | Bezeichnung | Ausb. % | Schmelzpunkt °C (Lösungsmittel) |
|---|---|---|---|
| 11. | 1-(Indol-4-yloxy)-3-[2-(4-nitroxy-butanoylamino)ethylamino]-2-propanol hemifumarat<br>aus<br>2,3-Epoxy-1-(indol-4-yloxy)-propan<br>und<br>2-(4-Nitroxy-butanoylamino)ethylamin | 27 | 130–131 (Methanol) |
| 12. | 1-(2-Methyl-indol-4-yloxy)-3-[2-(4-nitroxy-butanoylamino)ethylamino]-2-propanol cyclaminat<br>aus<br>2,3-epoxy-1-(2-methyl-indol-4-yloxy)propan<br>und<br>2-(4-Nitroxy-butanoylamino)ethylamin | 30 | 120–121 (Ethylacetat) |
| 13. | 1-(6-Methyl-indol-4-yloxy)-3-[2-(4-nitroxy-butanoylamino)ethylamino]-2-propanol fumarat<br>aus<br>2,3-Epoxy-1-(6-methyl-indol-4-yloxy)propan<br>und<br>2-(4-Nitroxy-butanoylamino)ethylamin | 25 | 132–133 (Methanol) |
| 14. | 1-(2,6-Dimethyl-indol-4-yloxy)-3-[2-(4-nitroxy-butanoylamino)-ethylamino]-2-propanol cyclaminat<br>aus<br>1-(2,6-Dimethyl-indol-4-yloxy)-2,3-epoxy-propan<br>und<br>2-(4-Nitroxy-butanoylamino)-ethylamin | 53 | 135 (Ethylacetat) |
| 15. | 1-(3-Cyano-indol-4-yloxy)-3-[2-(4-nitroxy-butanoylamino)ethylamino]-2-propanol hemifumarat<br>aus<br>1-(3-Cyano-indol-4-yloxy)-2,3-epoxy-propan<br>und<br>2-(4-Nitroxy-butanoylamino)-ethylamin | 50 | 108–110 (Ethylacetat) |
| 16. | 1-(3-Cyano-6-methyl-indol-4-yloxy)-3-[2-(4-nitroxy-butanoyl-amino)ethylamino]-2-propanol cyclaminat<br>aus<br>1-(3-Cyano-6-methyl-indol-4-yloxy)-2,3-epoxy-propan<br>und<br>2-(4-Nitroxy-butanoylamino)-ethylamin | 33 | 138–140 (Ethanol) |
| 17. | 3-[2-(4-Nitroxy-butanoylamino)-ethylamino]-1-(2-oxo-indolin-4-yloxy)-2-propanol fumarat<br>aus<br>2,3-Epoxy-1-(2-oxo-indolin-4-yloxy)-propan<br>und<br>2-(4-Nitroxy-butanoylamino)-ethylamin | 35 | 138–139 (Methanol) |
| 18. | 1-(1,2,3,4-Tetrahydro-2-oxo-chinolin-5-yloxy)-3-[2-(4-nitroxy-butanoylamino)-ethylamino]-2-propanol fumarat<br>aus<br>1-(1,2,3,4-Tetrahydro-2-oxo-chinolin-5-yloxy)-2,3-epoxy-propan<br>und<br>2-(4-Nitroxy-butanoylamino)-ethylamin | 53 | 135 (Ethanol) |
| 19. | 1-(1,2-Dihydro-2-oxo-chinolin-5-yloxy)-3-[2-(4-nitroxy-butanoylamino)ethylamino]-2-propanol cyclaminat<br>aus<br>1-(1,2-Dihydro-2-oxo-chinolin-5-yloxy)-2,3-epoxy-propan<br>und<br>2-(4-Nitroxy-butanoylamino)-ethylamin | 10 | 128 (Methanol) |

Beispiel 2 (Fortsetzung)

| Bezeichnung | Ausb. % | Schmelzpunkt °C (Lösungsmittel) |
|---|---|---|
| 20. 1-(1,2,3,4-Tetrahydro-7-methyl-2-oxo-chinolin-5-yloxy)-3-[2-(4-nitroxy-butanoylamino)ethylamino]-2-propanol fumarat aus 1-(1,2,3,4-Tetrahydro-7-methyl-2-oxo-chinolin-5-yloxy)-2,3-epoxy-propan und 2-(4-Nitroxy-butanoylamino)-ethylamin | 44 | 138–140 (Ethanol) |
| 21. 1-(1,2-Dihydro-7-methyl-2-oxo-chinolin-5-yloxy)-3-[2-(4-nitroxy-butanoylamino)ethylamino]-2-propanol fumarat aus 1-(1,2-Dihydro-7-methyl-2-oxo-chinolin-5-yloxy)-2,3-epoxy-propan und 2-(4-Nitroxy-butanoylamino)-ethylamin | 20 | 145 (Ethanol) |
| 22. 1-(3-Methyl-benzimidazol-4-yloxy)-3-[2-(4-nitroxy-butanoylamino)-ethylamino]-2-propanol cyclaminat aus 2,3-Epoxy-1-(3-methyl-benzimidazol-4-yloxy)-propan und 2-(4-Nitroxy-butanoylamino)-ethylamin | 3 | 90–93 (Toluol/Diethylether 1:1) |
| 23. 1-(2-Formyl-1,2,3,4-tetrahydro-isochinolin-5-yloxy)-3-[2-(4-nitroxy-butanoylamino)ethylamino]-2-propanol aus 2,3-Epoxy-1-(2-formyl-1,2,3,4-tetrahydro-isochinolin-5-yloxy)-propan und 2-(4-Nitroxy-butanoylamino)ethylamin | 47 | 122–123 (Aceton) |
| 24. 1-(2-Methyl-indol-4-yloxy)-3-[3-(4-nitroxy-butanoylamino)propylamino]-2-propanol cyclaminat aus 2,3-Epoxy-1-(2-methyl-indol-4-yloxy)-propan und 3-(4-Nitroxy-butanoylamino)-propylamin | 5 | 119–121 (Ethanol) |
| 25. 3-[3-(4-Nitroxy-butanoylamino)-propylamino]-1-(2-oxo-indolin-4-yloxy)-2-propanol cyclaminat aus 2,3-Epoxy-1-(2-oxo-indolin-4-yloxy)propan und 3-(4-Nitroxy-butanoylamino)-propylamin | 5 | 122–125 (Ethanol) |
| 26. 1-(3-Cyano-indol-4-yloxy)-3-[2,2-dimethyl-3-(4-nitroxy-butanoylamino)propylamino]-2-propanol fumarat aus 1-(3-Cyano-indol-4-yloxy)-2,3-epoxy-propan und 2,2-Dimethyl-3-(4-nitroxy-butanoylamino)propylamin | 15 | 119–120 (Ethanol) |
| 27. 3-[2,2-Dimethyl-3-(4-nitroxy-butanoylamino)propylamino]-1-(2-oxo-indolin-4-yloxy)-2-propanol fumarat aus 2,3-Epoxy-1-(2-oxo-indolin-4-yloxy)-propan und 2,2-Dimethyl-3-(4-nitroxy-butanoylamino)propylamin | 30 | 117–118 (Ethanol) |
| 28. 1-[3-Cyano-indol-4-yloxy)-3-[2-(4-nitroxy-pentanoylamino)ethylaminol-2-propanol cyclaminat aus 1-(3-Cyano-indol-4-yloxy)-2,3-epoxy-propan und 2-(4-Nitroxy-pentanoylamino)ethylamin | 20 | 137–138 (Ethanol) |

Beispiel 2 (Fortsetzung)

| | Bezeichnung | Ausb. % | Schmelzpunkt °C (Lösungsmittel) |
|---|---|---|---|
| 29. | 3-[2-(4-Nitroxy-pentanoylamino)-ethylamino]-1-(2-oxo-indolin-4-yloxy)-2-propanol cyclaminat<br>aus<br>2,3-Epoxy-1-(2-oxo-indolin-4-yloxy)propan<br>und<br>2-(4-Nitroxy-pentanoylamino)ethylamin | 20 | 137–138 (Ethanol) |
| 30. | 1-(3-Cyano-indol-4-yloxy)-3-[2-(5-nitroxy-pentanoylamino)-ethylamino]-2-propanol hemifumarat<br>aus<br>1-(3-Cyano-indol-4-yloxy)-2,3-epoxy-propan<br>und<br>2-(5-Nitroxy-pentanoylamino)ethylamin | 52 | 156–160 (2-Propanol) |
| 31. | 3-[2-(5-Nitroxy-pentanoylamino)-ethylamino]-1-(2-oxo-indolin-4-yloxy)-2-propanol hemifumarat<br>aus<br>2,3-Epoxy-1-(2-oxo-indolin-4-yloxy)propan<br>und<br>2-(5-Nitroxy-pentanoylamino)-ethylamin | 30 | 162–164 (Ethanol) |
| 32. | 1-(3-Cyano-indol-4-yloxy)-3-[2-(3,3-dimethyl-2,4-dinitroxy-butanoylamino)-ethylamino]-2-propanolfumarat<br>aus<br>1-(3-Cyano-indol-4-yloxy)-2,3-opoxy-propan<br>und<br>2-(3,3-Dimethyl-2,4-dinitroxy-butanoylamino)ethylamin | 20 | 50–55 (2-Propanol) |
| 33. | 3-[2-(3,3-Dimethyl-2,4-dinitroxy-butanoylamino)-ethylamino]-1-(2-oxo-indolin-4-yloxy)-2-propanol hemifumarat<br>aus<br>2,3-Epoxy-1-(2-oxo-indolin-4-yloxy)-propan<br>und<br>2-(3,3-Dimethyl-2,4-dinitroxy-butanoylamino)-ethylamin | 20 | 127–129 (2-Propanol) |
| 34. | trans-3-[2-[(2-Nitroxycyclohexyl)-acetylamino]ethylamino]-1-(2-oxo-indolin-4-yloxy)-2-propanol hemifumarat<br>aus<br>2,3-Epoxy-1-(2-oxo-indolin-4-yloxy)-propan<br>und<br>trans-2-[(2-Nitroxycyclohexyl)acetylamino]ethylamin | 35 | 151–153 (Methanol) |
| 35. | 3-[2-(4-Nitroxy-butanoylamino)-ethylamino]-1-(1-oxo-indan-7-yloxy)-2-propanol cyclaminat<br>aus<br>2,3-Epoxy-1-(1-oxo-indan-7-yloxy)-propan<br>und<br>2-(4-Nitroxy-butanoylamino)-ethylamin | 6 | 104–105 (Ethanol) |
| 36. | 1-(5-Methyl-1-oxo-indan-7-yloxy)-3-[2-(4-nitroxy-butanoylamino)-ethylamino]-2-propanol cyclaminat<br>aus<br>2,3-Epoxy-1-(5-methyl-1-oxo-indan-7-yloxy)-propan<br>und<br>2-(4-Nitroxy-butanoylamino)-ethylamin | 10 | 116–118 (Ethylacetat) |
| 37. | 1-(Indol-4-yloxy)-3-[2-(2-methyl-4-nitroxy-butanoylamino)ethylamino]-2-propanol cyclaminat<br>aus<br>2,3-Epoxy-1-(indol-4-yloxy)-propan<br>und<br>2-(2-Methyl-4-nitroxy-butanoylamino)ethylamin | 10 | 107 (2-Propanol) |

Beispiel 2 (Fortsetzung)

| Bezeichnung | Ausb. % | Schmelzpunkt °C (Lösungsmittel) |
|---|---|---|
| 38. 1-(3,6-Dimethyl-indol-4-yloxy)-3-[2-(4-nitroxy-butanoylamino)-ethylamino]-2-propanol cyclaminat aus 1-(3,6-Dimethyl-indol-4-yloxy)-2,3-Epoxy-propan und 2-(4-Nitroxy-butanoylamino)-ethylamin | 50 | 135 (Ethylacetat) |
| 39. 1-(Indol-4-yloxy)-3-[2,2-dimethyl-3-(4-nitroxy-butanoylamino)propylamino]-2-propanol hemifumarat aus 2,3-Epoxy-1-(indol-4-yloxy)-propan und 2,2-Dimethyl-3-(4-nitroxy-butanoylamino)propylamin | 32 | 136–138 (Ethanol) |
| 40. 1-(6-Methyl-indol-4-yloxy)-3-[2-(3-nitroxy-butanoylamino)-ethylamino]-2-propanol cyclaminat aus 2,3-Epoxy-1-(6-methyl-indol-4-yloxy-propan und 2-(3-Nitroxy-butanoylamino)-ethylamin | 38 | 166 (Ethanol) |
| 41. 1-(2-Methyl-indol-4-yloxy-)-3-[2-(5-nitroxy-pentanoylamino)-ethylamino]-2-propanol cyclaminat aus 2,3-Epoxy-1-(2-methyl-indol-4-yloxy)-propan und 2-(5-Nitroxy-pentanoylamino)-ethylamin | 48 | 135–137 (Ethanol) |
| 42. 3-[1,1-Dimethyl-2-(4-nitroxy-butanoylamino)-ethylamino]-1-(indol-4-yloxy)-2-propanol cyclaminat aus 2,3-Epoxy-1-(indol-4-yloxy)-propan und 1,1-Dimethyl-2-(4-nitroxy-butanoylamino)-ethylamin | 50 | 108–110 (Ethanol) |
| 43. 1-(2-Chloro-5-methyl-phenoxy)-3-[2-(5-nitroxy-pentanoylamino)-ethylamino]-2-propanol cyclaminat aus 1-(2-Chloro-5-methyl-phenoxy)-2,3-epoxy-propan und 2-(5-Ntroxy-pentanoylamino)-ethylamin | 14 | 111 (Aceton) |
| 44. trans-1-[4-(2-Methoxy-ethyl)-pheonxy]-3-[2-[(2-nitroxy-cyclohexyl)acetylaminoethylamino]-2-propanol aus 2,3-Epoxy-1-[4-(2-methoxy-ethyl)phenoxy]-propan und trans-2-[(2-Nitroxy-cyclohexyl)acetylamino]ethylamin | 36 | 107–109 (Ethylacetat) |
| 45. trans-1-(2-Allylphenoxy)-3-[2-[(2-nitroxy-cyclohexyl)-acetylamino]ethylamino]-2-propanol aus 1-(2-Allylphenoxy)-2,3-epoxy-propan und trans-2-[(2-Nitroxy-cyclohexyl)-acetylamino]ethylamin | 36 | 103–105 (Ethylacetat) |
| 46. 1-(2-Chloro-5-methyl-phenoxy)-3-[1,1-dimethyl-2-(4-nitroxy-butanoylamino)-ethylamino]-2-propanol cyclaminat aus 1-(2-Chloro-5-methyl-phenoxy)-2,3-epoxy-propan und 1,1-Dimethyl-2-(4-nitroxy-butanoylamino)-ethylamin | 39 | 108–110 (Ethylacetat) |

Beispiel 2 (Fortsetzung)

| Bezeichnung | Ausb. % | Schmelzpunkt °C (Lösungsmittel) |
|---|---|---|
| 47. 1-(2,5-Dichloro-phenoxy)-3-[2-(4-nitroxy-butanoylamino]-ethylamino]-2-propanol aus 1-(2,5-Dichloro-phenoxy)-2,3-epoxy-propan und 2-(4-Nitroxy-butanoylamino)-ethylamin | 60 | 125–127 (Aceton) |
| 48. 1-(2-Cyanophenoxy)-3-[1,1-dimethyl-2-(4-nitroxy-butanoylamino)-ethylamino]-2-propanol fumarat aus 1-(2-Cyanophenoxy)-2,3-epoxy-propan und 1,1-Dimethyl-2-(4-nitroxy-butanoylamino)-ethylamin | 55 | 140–145 (Ethylacetat) |
| 49. 3-[2-(3,3-Dimethyl-5-nitroxy-pentanoylamino)ethylamino]-1-(4-methylcarbonyloxy-2,3,5-trimethyl-phenoxy)-2-propanol fumarat aus 2,3-Epoxy-1-(4-methylcarbonyloxy-2,3,5-trimethyl-phenoxy)-propan und 2-(3,3-Dimethyl-5-nitroxy-pentanoylamino)ethylamin | 20 | 120–122 (Ethylacetat) |
| 50. trans-1-(2-Allylphenoxy)-3-[1,1-dimethyl-2-[(2-nitroxy-cyclohexyl)acetylamino]ethylamino]-2-propanol fumarat aus 1-(2-Allylphenoxy)-2,3-epoxy-propan und trans-1,1-Dimethyl-2-[(2-nitroxy-cyclohexyl)acetylamino]ethylamin | 12 | 130–132 (Ethylacetat) |
| 51. 1-(2-Allylphenoxy)-3-[1,1-dimethyl-2-(3-nitroxy-butanoylamino)-ethylamino]-2-propanol fumarat aus 1-(2-Allylphenoxy)-2,3-epoxy-propan und 1,1-Dimethyl-2-(3-nitroxy-butanoylamino)-ethylamin | 41 | 121–123 (Ethylacetat) |
| 52. 1-(2-Chloro-5-methyl-phenoxy)-3-[1,1-dimethyl-2-(3-nitroxy-butanoylamino)-ethylamino]-2-propanol fumarat aus 1-(2-Chloro-5-methyl-pheonxy)-2,3-epoxy-propan und 1,1-Dimethyl-2-(3-nitroxy-butanoylamino)-ethylamin | 66 | 144–147 (Ethylacetat) |
| 53. 3-[1,1-Dimethyl-2-(3-nitroxy-butanoylamino)-ethylamino]-1-(1-naphthyloxy)-2-propanol fumarat aus 2,3-Epoxy-1-(1-naphthyloxy)-propan und 1,1-Dimethyl-2-(3-nitroxy-butanoylamino)-ethylamin | 32 | 110–112 (Ethylacetat) |
| 54. 3-[1,1-Dimethyl-2-(4-nitroxy-butanoylamino)-ethylamino]-1-(1,2,3,4-tetrahydro-2-oxo-chinolin-5-yloxy)-2-propanol fumarat aus 2,3-Epoxy-1-(1,2,3,4-tetrahydro-2-oxo-chinolin-5-yloxy)-propan und 1,1-Dimethyl-2-(4-nitroxy-butanoylamino)-ethylamin | 10 | 132–134 (Ethanol) |
| 55. 3-[1,1,-Dimethyl-2-)4-nitroxy-butanoylamino)-ethylamino]-1-(2-oxo-indolin-4-yloxy)-2-propanol fumarat aus 2,3-Epoxy-1-(2-oxo-indolin-4-yloxy)-propan und 1,1-Dimethyl-2-(4-nitroxy-butanoylamino)-ethylamin | 25 | 120–122 (Ethanol) |

Beispiel 2 (Fortsetzung)

| | Bezeichnung | Ausb. % | Schmelzpunkt °C (Lösungsmittel) |
|---|---|---|---|
| 56. | 3-[2-(4-Nitroxy-butanoylamino)-ethylamino]-1-[2-(2-norbornyl-exo)phenoxy]-2-propanol cyclaminat aus 2,3-Epoxy-1-[2-(2-norbornyl-exo)phenoxy]-propan und 2-(4-Nitroxy-butanoylamino)ethylamin | 46 | 117–118 (Ethylacetat/ Diethylether 1:2) |
| 57. | 3-[1,1-Dimethyl-2-(4-nitroxy-butanoylamino)-ethylamino]-1-[2-(2-norbornylexo)phenoxy]-2-propanol fumarat aus 2,3-Epoxy-1-[2-(2-norbornylexo)-phenoxy]-propan und 1,1-Dimethyl-2-(4-nitroxy-butanoylamino)-ethylamin | 24 | 138–140 (Ethylacetat) |
| 58. | trans-3-[1,1-Dimethyl-2-[(2-nitroxy-cyclohexyl)acetylamino]-ethylamino]-1-(2H-5-methyl-2-oxo-benzo[e]pyran-8-yloxy)-2-propanolfumarat aus 2,3-Epoxy-1-(2H-5-methyl-2-oxo-benzo[e]pyran-8-yloxy)-propan und 1,1-Dimethyl-2-[(2-nitroxycyclohexyl)acetylamino]ethylamin | 58 | 170 (Zers.) (Ethylacetat) |
| 59. | 3-[1,1-Dimethyl-2-(4-nitroxy-butanoylamino)-ethylamino]-1-(2H-5-methyl-2-oxo-benzo[e]pyran-8-yloxy)-2-propanolcyclaminat aus 2,3-Epoxy-1-(2H-5-methyl-2-oxo-benzo[e]pyran-8-yloxy)-propan und 1,1-Dimethyl-2-(4-nitroxy-butanoylamino)-ethylamin | 50 | 120–122 (Ethylacetat) |
| 60. | 1-(2H-5-methyl-2-oxo-benzo[e]pyran-8-yloxy)-3-[2-(4-nitroxy-butanoylamino)ethylamino]-2-propanol aus 2,3-Epoxy-1-(2H-5-methyl-2-oxo-benzo[e]pyran-8-yloxy)-propan und 2-(4-Nitroxy-butanoylamino)-ethylamin | 39 | 66–68 (Ethylacetat) |
| 61. | trans-1-(2-Chloro-5-methyl-phenoxy)-3-[1,1-dimethyl-2-[(2-nitroxy-cyclohexyl)acetylamino]-ethylamino]-2-propanolfumarat aus 1-(2-Chloro-5-methyl-phenoxy)-2,3-eopxy-propan und 1,1-Dimethyl-2-[(2-nitroxy-cyclohexyl)acetylamino]-ethylamin | 28 | 134–136 (Aceton/Diethylether 5:2) |
| 62. | 1-(2,2-Dimethyl-1,3-benzdioxol-4-yloxy)-3-[2-(4-nitroxy-butanoylamino)ethylamino]-2-propanol fumarat aus 1-(2,2-Dimethyl-1,3-benzdioxol-4-yloxy)-2,3-epoxy-propan und 2-(2-Nitroxy-butanoylamino)-ethylamin | 32 | 123–125 (Ethylacetat) |
| 63. | 1-(2-Allyloxy-phenoxy)-3-[1,1-dimethyl-2-(4-nitroxy-butanoylamino)-ethylamino]-2-propanol fumarat aus 1-(2-Allyloxy-phenoxy)-2,3-epoxy-propan und 1,1-Dimethyl-2-(4-nitroxy-butanoylamino)-ethylamin | 57 | 115–117 (Ethylacetat) |
| 64. | 3-[1,1-Dimethyl-2-(4-nitroxy-butanoylamino)-ethylamino]-1-[4-(2-methoxy-ethyl)-phenoxy]-2-propanol fumarat aus 2,3-Epoxy-1-[4-(2-methoxy-ethyl)-phenoxy]-propan und 1,1-Dimethyl-2-(4-nitroxy-butanoylamino)-ethylamin | 40 | 128–130 (Ethylacetat) |

Beispiel 2 (Fortsetzung)

| Bezeichnung | | Ausb. % | Schmelzpunkt °C (Lösungsmittel) |
|---|---|---|---|
| 65. | 1-(2,5-Dichloro-phenoxy)-3-[1,1-Dimethyl-2-(4-nitroxy-butanoylamino)-ethylamino]-2-propanol fumarat aus 1-(2,5-Dichloro-phenoxy)-2,3-epoxy-propan und 1,1-Dimethyl-2-(4-nitroxy-butanoylamino)-ethylamin | 66 | 138–140 (Ethylacetat) |
| 66. | 1-(2-Cyanophenoxy)-3-[2-(3-nitroxy-butanoylamino)ethylamino]-2-propanol fumarat aus 1-(2-Cyanophenoxy)-2,3-epoxy-propan und 2-(3-Nitroxy-butanoylamino)-ethylamin | 23 | 140–143 (Aceton) |
| 67. | 1-(Indol-4-yloxy)-3-[2-(3-nitroxy-butanoylamino)ethylamino]-2-propanol cyclaminat aus 2,3-Epoxy-1-(indol-4-yloxy)-propan und 2-(3-Nitroxy-butanoylamino)-ethylamin | 31 | 163 (Ethanol) |
| 68. | 1-(1,2-Dihydro-2-oxo-chinolin-5-yloxy)-3-[2-(3-nitroxy-butanoylamino)-ethylamino]-2-propannl hemifumarat aus 1-(1,2-Dihydro-2-oxo-chinolin-5-yoloxy)-2,3-epoxy-propan und 2-(3-Nitroxy-butanoylamino)-ethylamin | 30 | 162 (Ethanol) |
| 69. | 3-[2-(3-Nitroxy-butanoylamino)-ethylamino]-1-(1,2,3,4-tetrahydro-2-oxo-chinolin-5-yloxy)-2-propanol fumarat aus 2,3-Epoxy-1-(1,2,3,4-tetrahydro-2-oxo-chinolin-5-yloxy)-propan und 2-(3-Nitroxy-butanoylamino)-ethylamin | 51 | 145–146 (Ethanol) |
| 70. | 3-[2-(2,2-Dimethyl-3-nitroxy-propanoylamino)ethylamino]-1-(1,2,3,4-tetrahydro-2-oxo-chinolin-5-yloxy)-2-propanolfumarat aus 2,3-Epoxy-1-(1,2,3,4-tetrahydro-2-oxo-chinolin-5-yloxy)-propan und 2-(2,2-Dimethyl-3-nitroxy-propanoylamino)ethylamin | 28 | 148–149 (Ethanol) |
| 71. | 1-(2-Cyanophenoxy)-3-[2-(4-nitroxy-butanoylamino)ethylamino]-2-propanol aus 1-(2-Cyanophenoxy)-2,3-epoxy-propan und 2-(4-Nitroxy-butanoylamino)-ethylamin | 20 | 108–110 (Ethylacetat) |
| 72. | 1-(2-Allylphenoxy)-3-[1,1-dimethyl-2-(4-nitroxy-butanoyl-amino)-ethylamino]-2-propanol cyclaminat aus 1-(2-Allylphenoxy)-2,3-epoxy-propan und 1,1-Dimethyl-2-(4-nitroxy-butanoylamino)-ethylamin | 33 | 125–127 (Ethylacetat) |
| 73. | 3-[1,1-Dimethyl-2-(4-nitroxy-butanoylamino)-ethylamino]-1-(1-naphthyloxy)-2-propanol fumarat aus 2,3-Epoxy-1-(1-naphthyloxy)-propan und 1,1-Dimethyl-2-(4-nitroxy-butanoylamino)-ethylamin | 47 | 115–118 (Ethylacetat) |

Beispiel 2 (Fortsetzung)

| Bezeichnung | | Ausb. % | Schmelzpunkt °C (Lösungsmittel) |
|---|---|---|---|
| 74. | 1-(3-Cyano-indol-4-yloxy)-3-[3-(4-nitroxy-butanoylamino)-propylamino]-2-propanol cyclaminat<br>aus<br>1-(3-Cyano-indol-4-yloxy)-2,3-epoxy-propan<br>und<br>3-(4-Nitroxy-butanoylamino)-propylamin | 22 | 137–138 (Ethanol) |
| 75. | 1-(2-Cyano-phenoxy)-3-[2-(5-nitroxy-pentanoylamino)ethylamino]-2-propanol fumarat<br>aus<br>1-(2-Cyano-phenoxy)-2,3-epoxy-propan<br>und<br>2-(5-Nitroxy-pentanoylamino)-ethylamin | 31 | 125–128 (Aceton) |
| 76. | 1-(1-Naphthyloxy)-3-[2-(5-nitroxy-pentanoylamino)ethylamino]-2-propanol<br>aus<br>2,3-Epoxy-1-(1-naphthyloxy)-propan<br>und<br>2-(5-Nitroxy-pentanoylamino)-ethylamin | 49 | 92–95 (Ethylacetat) |
| 77. | 1-(Indol-4-yloxy)-3-[2-(5-nitroxy-pentanoylamino)ethylamino]-2-propanol hemifumarat<br>aus<br>2,3-Epoxy-1-(indol-4-yoloxy)-propan<br>und<br>2-(5-Nitroxy-pentanoylamino)-ethylamin | 46 | 127–129 (Ethanol) |
| 78. | 1-(6-Methyl-indol-4-yloxy)-3-[2-(5-nitroxy-pentanoylamino)ethylamino]-2-propanol cyclaminat<br>aus<br>2,3-Epoxy-1-(6-methyl-indol-4-yloxy)-propan<br>und<br>2-(5-Nitroxy-pentanoylamino)-ethylamin | 37 | 150–151 (Ethanol) |
| 79. | 3-[2-(5-Nitroxy-pentanoylamino)ethylamino]-1-(1,2,3,4-tetra-hydro-2-oxo-chinolin-5-yloxy)-2-propanolfumarat<br>aus<br>2,3-Epoxy-1-(1,2,3,4-tetrahydro-2-oxo-chinolin-5-yloxy)-propan<br>und<br>2-(5-Nitroxy-pentanoylamino)-ethylamin | 53 | 153–155 (Ethanol) |
| 80. | trans-1-(2-Allyloxy-phenoxy)-3-[2-[(2-nitroxy-cyclohexyl)-acetylamino]ethylamino]-2-propanol<br>aus<br>1-(2-Allyloxy-phenoxy)-2,3-epoxy-propan<br>und<br>trans-2-[(2-Nitroxy-cyclohexyl)-acetylamino]ethylamin | 33 | 105–107 (Ethylacetat) |
| 81. | trans-1-(2-Cyanophenoxy)-3-[2-[(2-nitroxy-cyclohexyl)acetylamino]-ethylamino]-2-propanolfumarat<br>aus<br>1-(2-Cyanophenoxy)-2,3-epoxy-propan<br>und<br>trans-2-[(2-Nitroxy-cyclohexyl)-acetylamino]ethylamin | 38 | 145–148 (Ethylacetat) |
| 82. | trans-1-(2-Chloro-5-methyl-phenoxy)-3-[2-[(2-nitroxy-cyclohexyl)acetylamino]ethylamino]-2-propanolfumarat<br>aus<br>1-(2-Chloro-5-methyl-phenoxy)-2,3-epoxy-propan<br>und<br>trans-2-[(2-Nitroxy-cyclohexyl)-acetylamino]ethylamin | 35 | 148–150 (Aceton) |

Beispiel 2 (Fortsetzung)

| Bezeichnung | | Ausb. % | Schmelzpunkt °C (Lösungsmittel) |
|---|---|---|---|
| 83. | trans-1-(4-Methylcarbonyloxy-2,3,5-trimethyl-phenoxy)-3-[2-[(2-nitroxy-cyclohexyl)acetylamino]ethylamino]-2-propanol<br>aus<br>2,3-Epoxy-1-(4-methylcarbonyloxy-2,3,5-trimethyl-phenoxy)-propan<br>und<br>trans-2-[(2-Nitroxy-cyclohexyl)-acetylamino]ethylamin | 25 | 80–83 (Ethylacetat) |
| 84. | trans-1-(1-Naphthyloxy)-3-[2-[(2-nitroxy-cyclohexyl)acetylamino]-ethylamino]-2-propanol fumarat<br>aus<br>2,3-Epoxy-1-(1-naphthyloxy)-propan<br>und<br>trans-2-[(2-Nitroxy-cyclohexyl)-acetylamino]ethylamin | 63 | 154–156 (Ethylacetat) |
| 85. | trans-1-(Indol-4-yloxy)-3-[2[(2-nitroxy-cyclohexyl)acetylamino]-ethylamino]-2-propanol hemifumarat<br>aus<br>2,3-Epoxy-1-(indol-4-yloxy)-propan<br>und<br>trans-2-[(2-Nitroxy-cyclohexyl)-acetylamino]ethylamin | 26 | 139–141 (Ethanol) |
| 86. | trans-3-[2-[(2-Nitroxy-cyclohexyl)-acetylamino]ethylamino]-1-(1,2,3,4-tetrahydro-2-oxo-chinolin-5-yloxy)-2-propanolfumarat<br>aus<br>2,3-Epoxy-1-(1,2,3,4-tetrahydro-2-oxo-chinolin-5-yloxy)-propan<br>und<br>trans-2-[(2-Nitroxy-cyclohexyl)-acetylamino]ethylamin | 38 | 133 (Ethanol) |
| 87. | trans-1-(1,2-Dihydro-2-oxo-chinolin-5-yloxy)-3-[2-[(2-nitroxy-cyclohexyl)-acetylamino]ethylamino]-2-propanolfumarat<br>aus<br>1-(1,2-Dihydro-2-oxo-chinolin-5-yloxy)-2,3-epoxy-propan<br>und<br>trans-2-[(2-Nitroxy-cyclohexyl)acetylamino]ethylamin | 25 | 128–130 (Ethanol) |
| 88. | 3-[2-(2-Methyl-4-nitroxy-butanoylamino]-1-(2-oxo-indolin-4-yloxy)-2-propanol cyclaminat<br>aus<br>2,3-Epoxy-1-(2-oxo-indolin-4-yloxy)-propan<br>und<br>2-(2-Methyl-4-nitroxy-butanoylamino)ethylamin | 17 | 123–125 (Ethanol) |
| 89. | 1-(2-Allylphenoxy)-3-[2-[(2-nitroxy-ethoxy)acetylamino]ethylamino]-2-propanol fumarat<br>aus<br>1-(2-Allylphenoxy)-2,3-epoxy-propan<br>und<br>2-[(2-Nitroxyethoxy)acetylamino]-ethylamin | 12 | 128 (Aceton) |

Beispiel 3

2-(3-Hydroxybutanoylamino)-ethylamin

192 ml 1.2-Diaminoethan werden mit 60 ml Wasser gemischt und unter Ruehren und leichtem Kuehlen bei 20-30°C 74 ml β-Butyrolacton zugetropft. Nach 4 d wird im Vacuum abdestilliert und der Kristallbrei ueber Schwefelsaeure im Exsiccator getrocknet.
Man erhaelt 122.4 g Base (93 % d.Th.) vom Schmp. 71-73°C.

Beispiel 4

In analoger Weise, wie im Beispiel 3 beschrieben, wurden folgende Verbindungen erhalten.

| | Bezeichnung | Ausb. % | Schmelzpunkt °C (Lösungsmittel) |
|---|---|---|---|
| 1. | 2-(4-Hydroxybutanoylamino)-ethylammonium oxalat aus γ-Butyrolacton und 1,2-Diamino-ethan | 93 | 65–66 (2-Propanol) |
| 2. | 2-(5-Hydroxypentanoylamino)-ethylammonium oxalat aus δ-Valerolacton und 1,2-Diamino-ethan | 78 | 75 (2-Propanol) |
| 3. | 2-(2,4-Dihydroxy-3,3-dimethyl-butanoylamino)-ethylammoniumoxalat aus 2-Hydroxy-3,3-dimethyl-γ-butyrolacton und 1,2-Diamino-ethan | 80 | 187–189 (2-Propanol) |
| 4. | 3-(4-Hydroxybutanoylamino)-propylammonium oxalat aus γ-Butyrolacton und 1,3-Diaminopropan | 52 | 80 (2-Propanol) |
| 5. | 3-(4-Hydroxybutanoylamino)-2.2-dimethyl-propylammoniumoxalat aus γ-Butyrolacton und 1,3-Diamino-2,2-dimethyl-porpan | 62 | 127–130 (2-Propanol) |
| 6. | 2-(4-Hydroxy-pentanoylamino)-ethylammonium oxalat aus γ-Valerolacton und 1,2-Diamino-ethan | 78 | 75 (2-Propanol) |
| 7. | trans-2-[(2-Hydroxycyclohexyl)-acetylamino]-ethylamin aus trans-(2-Hydroxycyclohexyl)essigsäurelacton (Lit. J. Am. Chem. Soc. 67 (1945), 233) und 1,2-Diamino-ethan | 88 | 136–137 (2-Propanol) |
| 8. | 1,1-Dimethyl-2-(4-hydroxybutanoyl-amino)-ethylammoniumoxalat aus γ-Butyrolacton und 1,2-Diamino-2-methylpropan | 41 | 179–180 (2-Propanol/ Ethylacetat 1:2) |
| 9. | 2-(3,3-Dimethyl-5-hydroxy-pentanoylamino)ethylammoniumoxalat aus 3,3-Diemthyl-δ-valerolacton (hergestellt aus 3,3-Dimethylglutarsäureanhydrid, J. Org. Chem. 35 (1970), 3574) und 1,2-Diamino-ethan | 63 | 124–126 (2-Propanol) |
| 10. | trans-1,1-Dimethyl-2-[(2-hydroxy-cyclohexyl)acetylamino]ethylammonium oxalat aus trans-(2-Hydroxy-cyclohexyl)essigsäurelacton und 1,2-Diamino-2-methyl-propan | 64 | 210–212 (2-Propanol/ Diethylether 2:1) |

Beispiel 4 (Fortsetzung)

| | Bezeichnung | Ausb. % | Schmelzpunkt °C (Lösungsmittel) |
|---|---|---|---|
| 11. | 1,1-Dimethyl-2-(3-hydroxy-butanoylamino)-ethylammoniumoxalat<br>aus<br>β-Butyrolcaton<br>und<br>1,2-Diamino-2-methyl-propan | 78 | 142–145<br>(2-Propanol/<br>Ethylacetat 1:4) |
| 12. | 2-(4-Hydroxy-2-methyl-butanoylamino)ethylammoniumoxalat<br>aus<br>2-Methyl-γ-butyrolacton<br>und<br>1,2-Diamino-ethan | 75 | 103–106<br>(Methanol/<br>Ethylacetat) |
| 13. | 2-(2,2-Dimethyl-3-hydroxy-Propanoylamino)ethylammoniumoxalat<br>aus<br>2,2-Dimethyl-3-hydroxy-propionsäuremethylester<br>und<br>1,2-Diamino-ethan | 78 | 168–170<br>(2-Propanol) |
| 14. | 2.[(2-Hydroxyethoxy)acetylamino]-ethylammoniumoxalat<br>aus<br>2-(2-Hydroxyethoxy)-essigsäureethylester (Chem. Ber. 93 (1960), 1129)<br>und<br>1,2-Diamino-ethan | 89 | 123–124<br>(2-Propanol) |

Beispiel 5

2-(3-Nitroxy-butanoylamino)ethylamin

30 g 2-(3-Hydroxybutanoylamino)-ethylamin werden unter Ruehren bei 5-10°C langsam in 100 proz. Salpetersaeure eingetragen. Nach 2 h Ruehren bei 0-5°C wird die Reaktionsloesung in 1 l eisgekuehlten, wasserfreien Ether eingeruehrt, der ueberstehende Ether abdekantiert, das ausgefallene Oel mit Methylenchlorid ueberschichtet und mit Natriumcarbonat die Base freigesetzt. Nach Absaugen und Eindampfen i.Vac. bei max. 20°C Badtemperatur erhaelt man 33 g Base (84 % d.Th.) als Oel.

Beispiel 6

In analoger Weise, wie im Beispiel 5 beschrieben, wurden folgende Verbindungen erhalten.

| Bezeichnung | Ausb. % | Schmelzpunkt °C (Lösungsmittel) |
|---|---|---|
| 1. 2-(4-Nitroxy-butanoylamino)-ethylammoniumoxalat aus 2-(4-Hydroxybutanoylamino)-ethylamin und Salpetersäure | 95 | Öl |
| 2. 2-(5-Nitroxy-pentanoylamino)-ethylamin aus 2-(5-Hydroxypentanoylamino)-ethylammonium oxalat und Salpetersäure | 76 | Öl |
| 3. 2-[3,3-Dimethyl-2,4-dinitroxy-butanoylamino)ethylammoniumnitrat aus 2-(2,4-Dihydroxy-3,3-dimethyl-butanoylamino)ethylamin und Salpetersäure | 70 | 158–160 Essigester/Ether |
| 4. 3-(4-Nitroxy-butanoylamino)-propylamin aus 3-(4-Hydroxybutanoylamino)-propylammonium oxalat und Salpetersäure | 73 | Öl |
| 5. 2,2-Dimethyl-3-(4-nitroxy-butanoylamino)propylamin aus 2,2-Dimethyl-3-(4-hydroxybutanoylamino)propylammoniumoxalat und Salpetersäure | 60 | Öl |
| 6. 2-(4-Nitroxy-pentanoylamino)-ethylamin aus 2-(4-Hydroxy-pentanoylamino)-ethylamin und Salletersäure | 76 | Öl |
| 7. trans-2-[(2-Nitroxy-cyclohexyl)-acetylamino]-ethylamin aus trans-2-[(2-Hydroxy-cyclohexyl)-acetylamino]-ethylamin und Salpetersäure | 45 | Öl |
| 8. 1,1-Dimethyl-2-(4-nitroxy-butanoylamino)-ethylamin aus 1,1-Dimethyl-2-(4-hydroxybutanoylamino)-ethylammoniumoxalat und Salpetersäure | 100 | Öl |
| 9. 2-(3,3-Dimethyl-5-nitroxy-pentanoylamino)-ethylamin aus 2-(3,3-Dimethyl-5-hydroxy-pentanoylamino)-ethylammoniumoxalat und Salpetersäure | 86 | Öl |
| 10. trans-1,1-Dimethyl-2-[(2-nitroxy-cyclohexyl)acetylamino]-ethylamin aus trans-1,1-Dimethyl-2-[(2-hydroxy-cyclohexyl)acetylamino]-ethylammonium oxalat und Salpetersäure | 99 | Öl |
| 11. 1,1-Dimethyl-2-(3-nitroxy-butanoylamino)-ethylamin aus 1,1-Dimethyl-2-(3-hydroxy-butanoylamino)-ethylammoniumoxalat und Salpetersäure | 93 | Öl |

Beispiel 6 (Fortsetzung)

| Bezeichnung | Ausb. % | Schmelzpunkt °C (Lösungsmittel) |
|---|---|---|
| 12. 2-(2-Methyl-4-nitroxy-butanoylamino)ethylamin aus 2-(4-Hydroxy-2-methyl-butanoylamino)ethylammoniumoxalat und Salpetersäure | 70 | Öl |
| 13. 2-(2,2-Dimethyl-3-nitroxy-propanoylamino)ethylamin aus 2-(2,2-Dimethyl-3-hydroxy-propanoylamino)ethylammoniumoxalat und Salpetersäure | 100 | Öl |
| 14. 2-[(2-Nitroxyethoxy)acetylamino]-ethylamin aus 2-[(2-Hydroxyethoxy)acetylamino]-ethylammoniumoxalat und Salpetersäure | 68 | Öl |

Beispiel 7

3-[1,1-Dimethyl-2-(2,2-dimethyl-3-nitroxy-propanoylamino)-ethylamino]-1-(3-methyl-benzimidazol-4-yloxy)-2-propanol fumarat

Zu einer Loesung von 2.3 g 3-(2-Amino-1,1-dimethyl-ethylamino)-1-(3-methyl-benzimidazol-4-yloxy)-2-propanol in 150 ml trockenem Tetrahydrofuran tropft man bei Raumtemperatur eine Loesung von 1.4 g 2,2-Dimethyl-3-nitroxy-propanoylchlorid in 50 ml trockenem Tetrahydrofuran und ruehrt noch 2 h weiter. Das Reaktionsgemisch wird eingeengt und an Kieselgel mit Methylenchlorid/Methanol (4:2) chromatographiert. Das gewonnene Produkt (2.0 g) wird in 60 ml Methylenchlorid geloest und mit 0.54 g Fumarsaeure in 100 ml Aceton versetzt. Der nach Zugabe von 200 ml Diethylether abgeschiedene Niederschlag wird abgesaugt und getrocknet.
Man erhaelt 2.3 g (53 % d.Th.) Kristalle vom Schmp. 187-190°C.

Beispiel 8

In analoger Weise, wie in Beispiel 7 beschrieben, erhaelt man aus 1-Aryloxy-3-(2-amino-1,1-dimethyl-ethylamino)-2-propanolen bzw. 1-Hetaryloxy-3-(2-amino-1,1-dimethyl-ethylamino)-2-propanolen und Nitroxy-alkanoylchloriden folgende Verbindungen:

| | Bezeichnung | Ausb. % | Schmelzpunkt °C (Lösungsmittel) |
|---|---|---|---|
| 1. | 3-[1,1-Dimethyl-2-(2,2-dimethyl-3-nitroxy-propanoylamino)-ethylamino]-1-[4-(2-methoxyethyl)-phenoxy]-2-propanol aus 3-(2-Amino-1,1-dimethyl-ethylamino-1-[4-(2-methoxyethyl)-phenoxy]-2-propanol und 2,2-Dimethyl-3-nitroxy-propanoylchlorid | 41 | Öl |
| 2. | 1-(2-Cyclopentyl-phenoxy)-3-[1,1-dimethyl-2-(2,2-diemthyl-3-nitroxy-propanoylamino)-ethylamino]-2-propanol aus 3-(2-Amino-1,1-dimethyl-ethylamino)-1-(2-cyclopentyl-phenoxy)-2-propanol und 2,2-Dimethyl-3-nitroxy-propanoyl-chlorid | 45 | 138–140 (Ethylacetat/ Diethylether 2:3) |
| 3. | 1-(4-Carbamoylmethyl-phenoxy)-3-[1,1-dimethyl-2-[2,2-bis-(nitroxy-methyl)propanoylamino]-ethylamino]-2-propanol aus 3-(2-Amino-1,1-dimethyl-ethylamino)-1-(4-carbamoylmethyl-phenoxy)-2-propanol und 2,2-bis-(nitroxy-methyl)-propancylchlorid | 48 | amorph (Diethylether) |
| 4. | 1-(4-Cyclohexylaminocarbonylamino-phenoxy)-3-[1,1-dimethyl-2-(2,2-dimethyl-3-nitroxy-propanoylamino)-ethylamino]-2-propanol aus 3-(2-Amino-1,1-dimethyl-ethylamino)-1-(4-cyclohexylaminocarbonylamino-phenoxy)-2-propanol und 2,2-Dimethyl-3-nitroxy-propanoylchlorid | 25 | 172–175 (Diethylether/ Ligroin 1:1) |
| 5. | 3-[1,1-Dimethyl-2-(2,2-dimethyl-3-nitroxy-propanoylamino)-ethylamino]-1-(4-methylcarbonyloxy-2,3,5-trimethyl-phenoxy)-2-propanolfumarat aus 3-(2-Amino-1,1-dimethyl-ethylamino)-1-(4-methylcarbonyloxy-2,3,5-trimethyl-pheonoxy)-2-propanol und 2,2-Dimethyl-3-nitroxy-propanoylchlorid | 84 | 68–70 (Aceton) |
| 6. | 3-[1,1-Dimethyl-2-(2,2-dimethyl-3-nitroxy-propanoylamino)-ethylamino]-1-(4-hydroxy-2,3,5-trimethyl-phenoxy)-2-propanolfumarat aus 3-(2-Amino-1,1-dimethyl-ethylamino)-1-(4-hydroxy-2,3,5-trimethyl-phenoxy)-2-propanol und 2,2-Dimethyl-3-nitroxy-propanoylchlorid | 24 | 152–155 (Diethylether) |
| 7. | 1-(Benzimidazolin-2-on-4-yloxy)-3-[1,1-dimethyl-2-(2,2-dimethyl-3-nitroxy-propanoylamino)-ethylamino]-2-propanolfumarat aus 3-(2-Amino-1,1-dimethyl-ethylamino)-1-(benzimidazolin-2-on-4-yloxy)-2-propanol und 2,2-Dimethyl-3-nitroxy-propanoylchlorid | 69 | 118–120 (Diethylether) |
| 8. | 1-(Carbazol-4-yloxy)-3-[1,1-dimethyl-2-(2,2-dimethyl-3-nitroxy-propanoylamino)-ethylamino]-2-propanolfumarat aus 3-(2-Amino-1,1-dimethyl-ethylamino)-1-(carbazol-4-yloxy)-2-propanol und 2,2-Dimethyl-3-nitroxy-propanoylchlorid | 34 | 185–186 (Diethylether) |

Beispiel 8 (Fortsetzung)

| Bezeichnung | Ausb. % | Schmelzpunkt °C (Lösungsmittel) |
|---|---|---|
| 9. 3-[2-(2,2-Bis-(nitroxymethyl)-propanoylamino)-1,1-dimethyl-ethylamino]-1-(4-cyclohexylamino-carbonylamino-phenoxy)-2-propanolfumarat aus 3-(2-Amino-1,1-dimethyl-ethylamino)-1-(4-cyclohexylaminocarbonylamino-phenoxy)-2-propanol und 2,2-Bis-(nitroxymethyl)-propanoylchlorid | 37 | 174–176 (Diethylether) |
| 10. 3-[2-(2,2-Bis-(nitroxymethyl)-propanoylamino)-1,1-dimethyl-ethylamino]-1-(3-methyl-benzimidazol-4-yloxy)-2-propanolfumarat aus 3-(2-Amino-1,1-dimethyl-ethylamino)-1-(3-methyl-benzimidazol-4-yloxy)-2-propanol und 2,2-Bis-(nitroxymethyl)-propanoylchlorid | 24 | 105–108 (Diethylether) |
| 11. 3-[2-[2,2-Bis-(nitroxymethyl)-propanoylamino]-1,1-dimethyl-ethylamino]-1-(carbazol-4-yloxy)-2-propanol fumarat aus 3-(2-Amino-1,1-dimethyl-ethylamino)-1-(carbazol-4-yloxy)-2-propanol und 2,2-Bis-(nitroxymethyl)–propanoylchlorid | 34 | 110–115 (Diethyyether) |
| 12. 3-[1,1-Dimethyl-2-(4-nitroxy-butanoylamino)-ethylamino]-1-[4-(2-methoxy-ethyl)-phenoxy]-2-propanol aus 3-(2-Amino-1,1-dimenthyl-ethylamino)-1-[4-(2-methoxy-ethyl)-phenoxy]-2-propanol und 4-Nitroxy-butanoylchlorid | 100 | Öl |
| 13. 1-(2-Cyclopentyl-phenoxy)-3-[1,1-dimethyl-2-(4-nitroxy-butanoylamino)-ethylamino]-2-propanol cyclaminat aus 3-(2-Amino-1,1-dimethyl-ethylamino)-1-(2-cyclopentyl-phenoxy)-2-propanol und 4-Nitrioxy-butanoylchlorid | 40 | 127–128 (Ethylacetat/ Diethylether 1:9) |
| 14. 1-(4-Cyclohexylaminocarbonylamino-phenoxy)-3-[1,1-dimethyl-2-(4-nitroxy-butanoylamino)-ethylamino]-2-propanolfumarat aus 3-(2-Amino-1,1-dimethyl-ethylamino)-1-(4-cyclohexylamino-carbonylamino-phenoxy)-2-propanol und 4-Nitroxy-butanoylchlorid | 36 | 177–179 (Diethylether) |
| 15. 3-[1,1-Dimethyl-2-(4-nitroxybutanoylamino)-ethylamino]-1-(4-methylcarbonyloxy-2,3,5-trimethyl-phenoxy)-2-propanolfumarat aus 3-(2-Amino-1,1-dimethyl-ethylamino)-1-(4-methylcarbonyloxy-2,3,5-trimethyl-phenoxy)-2-propanol und 4-Nitroxy-butanoylchlorid | 51 | 73–75 (Diethylether) |
| 16. 3-[1,1-Dimethyl-2-(4-nitroxybutanoylamino)-ethylamino]-1-(4-hydroxy-2,3,5-trimethyl-phenoxy)-2-propanolcyclaminat aus 3-(2-Amino-1,1-dimethyl-ethylamino)-1-(4-hydroxy-2,3,5-trimethyl-phenoxy)-2-propanol und 4-Nitroxy-butanoylchlorid | 26 | 133–134 (Ethylacetat) |

Beispiel 8 (Fortsetzung)

| | Bezeichnung | Ausb. % | Schmelzpunkt °C (Lösungsmittel) |
|---|---|---|---|
| 17. | 1-(Benzimidazolin-2-on-4-yloxy)-3-[1,1-dimethyl-2-(4-nitroxy-butanoyl-amino)-ethylamino]-2-propanolfumarat aus 3-(2-Amino-1,1-dimethyl-ethylamino)-1-(benzimidazolin-2-on-4-yloxy)-2-propanol und 4-Nitroxy-butanoylchlorid | 37 | 105–107 (Diethylether) |
| 18. | 1-(2-Cyano-phenoxy)-3-[2-(2,2-dimethyl-3-nitroxy-propanoylamino)-ethylamino]-2-propanolfumarat aus 3-(2-Amino-ethylamino)-1-(2-cyano-phenoxy)-2-propanol und 2,2-Dimethyl-3-nitroxy-propanoylchlorid | 16 | 136–138 (Diethylether) |
| 19. | 3-[2-(2,2-Dimethyl-3-nitroxy-propanoylamino)-ethylamino]-1-(1-naphthyloxy)-2-propanol cyclaminat aus 3-(2-Amino-ethylamino)-1-(1-naphthyloxy)-2-propanol und 2,2-Dimethyl-3-nitroxy-propanoylchlorid | 6 | 107–109 (Ethylacetat) |
| 20. | 3-[2-(2,2-Dimethyl-3-nitroxy-propanoylamino)-ethylamino]-1-(2-oxo-indolin-4-yloxy)-2-propanol hemifumarat aus 3-(2-Amino-ethylamino)-1-(2-oxo-indolin-4-yloxy)-2-propanol und 2,2-Dimethyl-3-nitroxy-propanoylclorid | 10 | 180–183 (Methanol) |

3-(2-Amino-1,1-dimethyl-ethylamino)-1-(4-hydroxy-2,3,5-trimethyl-phenoxy)-2-propanol wurde aus 3-(2-Dibenzylamino-1,1-dimethyl-ethylamino)-1-(4-methylcarbonyloxy-2,3,5-trimethyl-phenoxy)-2-propanol durch Verseifung mit Kaliumhydroxid in Ethanol und anschliessender Hydrierung ueber Pd/C (10 %) in Ethanol bei 60°C gewonnen.

3-(2-Amino-ethylamino)-1-aryloxy-2-propanole wurden durch Umsetzung der entsprechenden 1-Aryloxy-2,3-epoxy-propane mit ueberschuessigem 1,2-Diamino-ethan hergestellt.

Beispiel 9

3-(2-Amino-1,1-dimethyl-ethylamino)-1-(3-methyl-benzimidazol-4-yloxy)-2-propanol

Zu 40.3 g 2-Dibenzylamino-1,1-dimethyl-ethylamin (hergestellt analog H.G. Johnson, J.Am.Chem.Soc. 68 (1946), 12) in 300 ml Ethanol gibt man 10.2 g 2,3-Epoxy-1-(3-methyl-benzimidazol-4-yloxy)-propan und ruehrt 3 d bei Raumtemperatur. Das Reaktionsgemisch wird nun bei 60°C ueber 8 g Pd/C (10 %) bis zur Beendigung der Wasserstoffaufnahme hydriert. Dann wird der Katalysator abgesaugt und das Filtrat zur Trockene eingedampft. Der Rueckstand wird an Kieselgel mit Methanol/konz. Ammoniakloesung (95:5) chromatographiert. Die entsprechenden Fraktionen werden eingedampft, der Rueckstand in Methylenchlorid aufgenommen, ueber Natriumsulfat getrocknet und die Loesung zur Trockne eingedampft. Man erhaelt 8.7 g (66 % d.Th.) hellgelbes Oel.

Beispiel 10

In analoger Weise, wie in Beispiel 9 beschrieben, erhaelt man aus 1-Aryloxy-2,3-epoxy-propanen bzw. 1-Hetaryloxy-2,3-epoxy-propanen und 2-Dibenzylamino-1,1-dimethyl-ethylamin folgende Verbindungen:

| | Bezeichnung | Ausb. % | Schmelzpunkt °C (Lösungsmittel) |
|---|---|---|---|
| 1. | 3-(2-Amino-1,1-dimethyl-ethylamino)-1-[4-(2-methoxy-ethyl)-phenoxy]-2-propanol aus 2,3-Epoxy-1-[4-(2-methoxy-ethyl)-phenoxy-propan | 37 | hellgelbes Öl |
| 2. | 3-(2-Amino-1,1-dimethyl-ethylamino)-1-(2-cyclopentyl-phenoxy)-2-propanol aus 1-(2-Cyclopentyl-phenoxy)-2,3-epoxy-propan | 43 | gelbes Öl |
| 3. | 3-(2-Amino-1,1-dimethyl-ethylamino)-1-(4-cyclohexylamino-carbonylamino-phenoxy)-2-propanol aus 1-(4-Cyclohexylaminocarbonylamino-phenoxy)-2,3-epoxy-propan | 33 | 162–163 (Diethylether) |
| 4. | 3-(2-Amino-1,1-dimethyl-ethylamino)-1-(4-methylcarbonyloxy-2,3,5-trimethyl-phenoxy)-2-propanol aus 2,3-Epoxy-1-(4-methylcarbonyloxy-2,3,5-trimethyl-phenoxy)-propan | 25 | hellgelbes Öl |
| 5. | 3-(2-Amino-1,1-dimethyl-ethylamino)-1-(benzimidazolin-2-on-4-yloxy)-2-propanol aus 1-(2-Amino-3-nitro-phenyloxy)-2,3-epoxy-propan, anschließende Hydrierung und Ringschlußreaktion | 62 | amorph |
| 6. | 3-(2-Amino-1,1-dimethyl-ethylamino)-1-(carbazol-4-yloxy)-2-propanol aus 1-(Carbazol-4-yloxy)-2,3-epoxy-propan | 43 | amorph |
| 7. | 3-(2-Amino-1,1-dimethyl-ethylamino)-1-(4-carbamoylmethyl-phenoxy)-2-propanol aus 1-(4-Carbamoylmethyl-phenoxy)-2,3-epoxy-propan | 81 | 113–115 (Diethylether) |

Beispiel 11

3-[1,1-Dimethyl-2-[(5-0-nitro-isosorbid-2-yloxy)carbonylamino]-ethylamino]-1-(3-methyl-benzimidazol-4-yloxy)-2-propanol fumarat

Zu einer Loesung von 1.63 g 1,1'-Carbonyldiimidazol in 30 ml trockenem Tetrahydrofuran tropft man eine Loesung von 1.91 g 5-0-Nitro-isosorbid* und 1.01 g Triethylamin in 10 ml trockenem Tetrahydrofuran und ruehrt 1 h bei Raumtemperatur. Zu dieser Loesung tropft man eine Loesung von 2.92 g 3-(2-Amino-1,1-dimethyl-ethylamino)-1-(3-methyl-benzimidazol-4-yloxy)-2-propanol in 30 ml trockenem Tetrahydrofuran, ruehrt noch 1 h und destilliert das Loesungsmittel ab. Der Rueckstand (7.2 g) wird an Kieselgel mit Methylenchlorid/Methanol (8:2) chromatographiert. Das gereinigte Produkt (3.6 g) wird in 50 ml Aceton geloest, mit 0.81 g Fumarsaeure in 50 ml Aceton versetzt, dann die Loesung zur Trockne eingedampft. Der Rueckstand wird mit 100 ml Diethylether digeriert, abgesaugt und getrocknet.
Man erhaelt 3.3 g (53 % d.Th.) Kristalle vom Schmelzpunkt 128-130°C.*Anm: 5-0-Nitro - 1:4, 3:6 - dianhydrosorbit

Beispiel 12

In analoger Weise, wie in Beispiel 5 beschrieben, wurden aus 1-Aryloxy-3-aminoalkylamino-2-propanolen bzw. 1-Hetaryloxy-3-aminoalkylamino-2-propanolen und Nitroxyalkanolen folgende Verbindungen:

| | Bezeichnung | Ausb. % | Schmelzpunkt °C (Lösungsmittel) |
|---|---|---|---|
| 1. | 3-[1,1-Dimethyl-2-[(5-0-nitro-isosorbid-2-yloxy)carbonylamino]-ethylamino]-1-[4-(2-methoxy-ethyl)-phenoxy]-2-propanolcyclaminat aus 3-[(2-Amino-1,1-dimethyl)-ethylamino]-1-[4-(2-methoxy-ethyl)-phenoxy]-2-propanol und 5-0-Nitro-isosorbid | 35 | 82 (Aceton) |
| 2. | 3-[1,1-Dimethyl-2-[(5-0-nitro-isosorbid-2-yloxy)carbonylamino]-ethylamino]-1-(4-methylcarbonyl-oxy-2,3,5-trimethyl-phenoxy)-2-propanol aus 3-(2-Amino-1,1-dimethyl-ethylamino)-1-(4-methylcarbonyloxy-2,3,5-trimethyl-phenoxy)-2-propanol und 5-0-Nitro-isosorbid | 91 | amorph |
| 3. | 1-(4-Carbamoylmethyl-phenoxy)-3-[1,1-dimethyl-2-[(5-0-nitro-isosorbid-2-yloxy)carbonylamino]-ethylamino]-2-propanol aus 3-(2-Amino-1,1-dimethyl-ethylamino)-1-(4-carbamoylmethyl-phenoxy)-2-propanol und 5-0-Nitro-isosorbid | 78 | Öl |

Beispiel 13

1-(2-Chloro-5-methyl-phenoxy)-3-[2-[(3-nitroxypropylamino)carbonylamino]ethylamino]-2-propanol

3.1 g 1-(2-Chloro-5-methyl-phenoxy)-2,3-epoxy-propan und 8.5 g 2-[(3-Nitroxypropylamino)carbonylamino]ethylamin werden in 100 ml n-Butanol 18 h bei Raumtemperatur geruehrt. Das Loesungsmittel wird bei 30°C Badtemperatur im Oelpumpenvakuum abdestilliert, der Rueckstand in 150 ml Ethylacetat geloest und diese Loesung 4mal mit je 60 ml Wasser gewaschen. Die organische Phase wird getrocknet und eingeengt und der Rueckstand an Kieselgel mit Methylenchlorid/Methanol (6:4) chromatographiert. Die entsprechenden Fraktionen werden eingeengt, der Rueckstand mit Diethylether verrieben, abgesaugt und getrocknet. Man erhaelt 2.2 g (35 %) Kristalle vom Schmp. 109-111°C.

2-[(3-Nitroxy-propylamino)carbonylamino]ethylamin wurde auf folgendem Weg hergestellt:

140 g Tetrahydro-2H-1,3-Oxazin-2-on (J. Org. Chem. 24 (1959), 1788) und 270.5 g 1,2-Diaminoethan werden in einem Autoklaven unter einer $N_2$-Atmosphaere von 50 bar 8 h bei 100°C geruehrt. Ueberschuessiges 1,2-Diaminoethan wird im Oelpumpenvakuum entfernt, der oelige Rueckstand wird in 2.6 l siedendem 2-Propanol geloest. Zu dieser Loesung wird eine heiße Loesung von 117.3 g Oxalsaeure in 1.3 l 2-Propanol gegeben. Man laeßt abkuehlen und saugt ab. Man erhaelt 215.5 g (66 %) weiße Krsitalle von 2-[(3-Hydroxy-propylamino)carbonylamino]ethylammonium oxalat (Schmp. 160-163°C). 25.1 g dieses Oxalats werden bei -20 bis -17°C innerhalb von 20 Minuten in Salpetersaeure (100 %) eingetragen. Man ruehrt noch 0.5 h bei -20°C und gießt das Reaktionsgemisch in 1 l kalten Diethylether. Man dekantiert und loest den Ruckstand in 150 ml Wasser. Durch Zugabe von $Na_2CO_3$ stellt man einem pH-Wert von 10 ein, versetzt mit 1 l Methylenchlorid und gibt unter Ruehren $Na_2CO_3$ bis zur Saettigung zu. Der Feststoff wird abgesaugt, mit Methylenchorid gewaschen und das Filtrat eingeengt. Man erhaelt 8.5 g gelbes Oel (41 % d.Th.).

Beispiel 14

1-(1-Naphthyloxy)-3-[2-[(4-nitroxy-1-piperidino)carbonylamino]ethylamino]-2-propanol fumarat

0.4 g 2,3-Epoxy-1-(1-naphthyloxy)-propan und 1.4 g 2-[(4-Nitroxy-1-piperidino)carbonylamino]-ethylamin werden in 30 ml n-Butanol 15 h bei Raumtemperatur geruehrt. Das Loesungsmittel wird abdestilliert, der Rueckstand in 50 ml Ethylacetat aufgenommen und diese Loesung 3mal mit je 50 ml Wasser gewaschen. Die organische Phase wird getrocknet und eingeengt und der Rueckstand (1.2 g Oel) an Kieselgel mit Methylenchlorid/Methanol (8:2) chromatographiert. Die entsprechenden Fraktionen werden

eingeengt, der Rueckstand (0.6 g) in 15 ml Ethylacetat geloest und mit 0.16 g Fumarsaeure versetzt. Der abgeschiedene Niederschlag wird abgesaugt und getrocknet. Man erhaelt 0.22 g (6 % d.Th.) weiße Kristalle vom Schmp. 123-125°C.

2-[(4-Nitroxy-1-piperidino)carbonylamino]ethylamin wurde auf folgendem Weg hergestellt:

Zu 49.0 g N,N-Dibenzylethylendiamin und 28.25 ml Triethylamin in 250 ml Tetrahydrofuran tropft man bei 0 bis 2°C eine Loesung von 1,1'-Carbonyldiimidazol in 750 ml Tetrahydrofuran und ruehrt 1 h bei 0°C. Nun tropft man die erhaltene Loesung bei 0 bis 2°C innerhalb von 1.5 h in eine Loesung von 20.6 g 4-Hydroxypiperidin in 250 ml Tetrahydrofuran. Man ruehrt noch 2 h bei 0°C und laeßt ueber Nacht auf Raumtemperatur aufwaermen. Nach dem Entfernen des Loesungsmittels wird der Rueckstand in 0.6 Liter Ethylacetat aufgenommen und die Loesung 3mal mit je 0.2 Liter Wasser gewaschen. Die organische Phase wird getrocknet und eingeengt.

Der Rueckstand (69 g) wird an Kieselgel mit Methylenchlorid/Methanol (10:1) chromatographiert. Man erhaelt 39.2 g (52 % d.Th.) Oel von N,N-Dibenzyl-2-[(4-hydroxy-1-piperidino)carbonylamino]-ethylamin. 39.1 g des erhaltenen Oels werden unter Erwaermung in 400 ml Ethanol geloest. Zu dieser Loesung gibt man 5 g Pd auf Kohle (10 %) und hydriert die warme Loesung unter Ruehren bis zur Beendigung der $H_2$-Aufnahme. Man saugt ab und dampft ein. Der Rueckstand (21.8 g Oel) wird in 200 ml siedendem 2-Propanol geloest und diese Loesung mit 9.6 g Oxalsaeure in 100 ml siedendem 2-Propanol versetzt. Man laeßt abkuehlen, saugt die abgeschiedenen Kristalle ab, waescht mit Ethylacetat und Ether und trocknet. Man erhaelt 22.0 g (70 % d.Th.) oxalsaures 2-[(4-Hydroxy-1-piperidino)carbonylamino]ethylamin vom Schmp. 173-175°C. 5.4 g dieses Salzes werden bei -20°C in 14.4 ml Salpetersaeure (100 %) eingetragen. Nach 20 Minuten wird die Reaktionsloseung langsam auf eine kalte Suspension von 49 g Kaliumcarbonat in 30 ml Wasser gegossen. Das Gemisch wird mit 0.5 Liter Methylenchlorid ueberschichtet und gut geruehrt. Die waeßrige Phase wird durch Zugabe von Natriumcarbonat und Natriumsulfat vollstaendig entfernt. Man saugt ab und engt ein. Es verbleiben 3.0 g eines oeligen Rueckstands von 2-[(4-Nitroxy-1-piperidino)carbonylamino]ethylamin.

## Patentansprüche

1. Verbindungen der allgemeinen Formel I

$$Ar-O-CH_2-\overset{OH}{\overset{|}{C}H}-CH_2-NH-A-NH-CO-X-B-(ONO_2)_n \qquad (I)$$

worin

Ar eine Phenylgruppe, die unsubstituiert oder substituiert sein kann durch Halogen, Cyano, Hydroxy, Amino, Oxo, Nitro, Carboxyl, Carbamoyl, Trifluormethyl, $C_1$–$C_{10}$-Alkyl, $C_2$–$C_{10}$-Alkenyl, $C_2$–$C_{10}$-Alkinyl, $C_3$–$C_8$-Cycloalkyl, $C_4$–$C_{11}$-Bicycloalkyl, $C_1$–$C_7$-Alkanoyl, $C_1$–$C_{10}$-Alkoxy, $C_2$–$C_{10}$-Alkoxylalkyl, Alkoxyalkoxyalkyl oder Alkoxyalkoxy mit bis zu 10 C-Atomen, $C_2$–$C_{10}$-Alkenyloxy, $C_2$–$C_{10}$-Alkenyloxyalkyl, $C_2$–$C_{10}$-Alkinyloxy, $C_3$–$C_{10}$-Alkinyloxyalkyl, $C_3$–$C_8$-Cycloalkoxy, $C_1$–$C_{10}$-Alkylthio, $C_2$–$C_{10}$-Alkylthioalkyl, $C_1$–$C_7$-Acylamino, Acylaminoalkyl mit bis zu 8 C-Atomen, $C_1$–$C_6$-Acyloxy, $C_1$–$C_5$-Alkoxycarbonyl, $C_4$–$C_8$-Cycloalkoxycarbonyl, $C_2$–$C_4$-Alkylaminocarbonylamino, $C_3$–$C_7$-Dialkylaminocarbonylamino, $C_4$–$C_{11}$-Dialkylaminocarbonylaminoalkyl, $C_4$–$C_{11}$-Dialkylaminocarbonylalkyl, $C_4$–$C_{10}$-Dialkylaminocarbonylalkoxy, wobei bei den letzteren vier Gruppen die beiden endständigen Alkylgruppen zusammen mit dem Stickstoffatom auch eine cyclische Gruppe mit 4 oder 5 C-Atomen bilden können, $C_4$–$C_8$-Cycloalkylaminocarbonylamino, $C_3$–$C_9$-Alkylaminocarbonylaminoalkyl, $C_5$–$C_{12}$-Cycloalkylaminocarbonylaminoalkyl, $C_3$–$C_{12}$ Alkoxycarbonylaminoalkyl, $C_5$–$C_{12}$-Cycloalkoxycarbonylaminoalkyl, $C_2$–$C_5$-Carbamoylalkyl, $C_3$–$C_9$-Alkylaminocarbonylalkyl, $C_5$–$C_{12}$-Cycloalkylaminocarbonylalkyl, oder $C_3$–$C_{10}$-Alkylaminocarbonylalkoxy,

oder Ar eine Naphthyl-, Indenyl-, Indolyl-, Indazolyl-, Imidazolyl-, Benzimidazolyl-, Benztriazolyl-, Benzofuranyl-, Benzodioxolyl-, Benzothiophenyl-, Benzthiazolyl-, Benzisothiazolyl-, Chinolyl-, Isochinolyl-, Benzodiazinyl-, Benzopyranyl-, Benzothiinyl-, Benzothiazinyl-, Benzothia-diazinyl-, Benzoxathiinyl-, Benzoxazolyl-, Benzisoxazolyl- oder Carbazolylgruppe bedeutet, wobei eine oder mehrere Doppelbindungen hydriert sein können, und diese zuvor genannten Gruppen unsubstituiert oder substituiert sein können durch $C_1$–$C_{10}$-Alkyl, Cyano, Hydroxyalkyl, Hydroxy, Oxo, Formyl, Alkanoyl oder Alkylcarbonylamino.

A eine geradkettige oder verzweigte Alkylenkette von 1–8 C-Atomen, wobei eine $-CH_2$-Gruppe durch eine Cycloalkylen-Gruppe von 3–7 C-Atomen ersetzt sein kann, bedeutet,

B eine geradkettige, mono- oder bicyclische, gegebenenfalls verzweigte, gesättigte oder ungesättigte Alkylenkette von 1–12 C-Atomen, worin eine $-CH_2$-Gruppe durch eine Cycloalkylen-Gruppe von 3–7 C-Atomen ersetzt sein kann und/oder bis zu 2 $-CH_2$-Gruppen jeweils durch ein Sauerstoffatom oder

Schwefelatom oder eine $-S(=O)-$ oder $-S(=O)_2$-Gruppe ersetzt sein können, bedeutet,
X eine Bindung, ein Sauerstoffatom oder eine Gruppe $-NR^1-$, worin $R^1$ Wasserstoff oder eine geradkettige oder verzweigte, gesättigte oder ungesättigte Alkyl- oder Nitroxyalkyl-Gruppe von 1–6 C-Atomen darstellt, oder $R^1$ auch gemeinsam mit dem Stickstoffatom der Gruppe $-NR^1-$ und einer $-CH_2$-Gruppe der Kette B einen heteroaliphatischen Ring mit 4–6 C-Atomen aufspannen kann, bedeutet,
n die Zahlen 1–3 bedeutet,
sowie deren physiologisch verträgliche Salze.

2. Verbindungen gemäß Anspruch 1, dadurch gekennzeichnet, daß in der allgemeinen Formel I
Ar eine Phenylgruppe, die unsubstituiert oder gegebenenfalls ein- oder mehrfach durch Halogen-, Cyano-, Hydroxyl-, $C_1$–$C_{10}$-Alkyl, $C_2$–$C_{10}$-Alkenyl, $C_2$–$C_{10}$-Alkenyloxy, $C_1$–$C_{10}$-Alkoxy, $C_3$–$C_8$-Cycloalkyl, $C_1$–$C_7$-Alkanoyl, $C_1$–$C_6$-Acyloxy, $C_4$–$C_{11}$-Bicycloalkyl, $C_2$–$C_{10}$-Alkoxyalkyl, $C_1$–$C_7$-Acylamino, $C_2$–$C_5$-Carbamoylalkyl, und $C_4$–$C_8$-Cycloalkylaminocarbonylamino-Gruppen substituiert ist oder eine Indolyl-, Naphthyl-, Chinolinyl-, Benzimidazolyl-, Isochinolinyl-, Benzopyranyl-, Benzdioxolyl-, Indenyl-, Benzoxazolyl- oder Carbyzoyl-Gruppe, die unsubstituiert oder gegebenenfalls ein- oder mehrfach durch Cyano-, Oxo-, Hydroxyl- oder Alkyl-Gruppen substituiert sein kann, darstellt, wobei eine oder zwei Doppelbindungen in den vorgenannten kondensierten und heterocyclischen Systemen hydriert sein können,
A eine geradkettige oder verzweigte Alkylenkette mit 1–8 C-Atomen bedeutet,
B eine geradkettige, mono- oder bicyclische, gegebenenfalls verzweigte, gesättigte oder ungesättigte Alkylenkette von 1–12 C-Atomen, worin eine $-CH_2$-Gruppe durch eine Cycloalkylen-Gruppe von 3–7 C-Atomen ersetzt sein kann und/oder bis zu 2 $-CH_2$-Gruppen jeweils durch ein Sauerstoffatom oder Schwefelatom oder eine $-S(=O)-$ oder $-S(=O)_2$-Gruppe ersetzt sein können, bedeutet,
X eine Bindung, ein Sauerstoffatom oder eine Gruppe $-NR^1-$, worin $R^1$ Wasserstoff, eine geradkettige oder verzweigte, gesättigte oder ungesättigte Alkyl- oder Nitroxyalkylgruppe von 1–6 C-Atomen darstellt,
n die Zahlen 1, 2 bedeutet,
sowie deren physiologisch verträgliche Salze.

3. Verbindungen gemäß Anspruch 1 oder 2, dadurch gekennzeichnet, daß in der allgemeinen Formel I
Ar eine Phenylgruppe, die unsubstituiert oder gegebenenfalls ein- oder zweifach durch Halogen-, Cyano-, $C_1$–$C_{10}$-Alkyl, $C_2$–$C_{10}$-Alkenyl-Gruppen substituiert sein kann, oder eine Indolyl-, Indolinonyl-, Chinolinyl- oder 1,2,3,4-Tetrahydro-2-oxo-chinolinyl-Gruppe darstellt,
A eine geradkettige oder verzweigte Alkylenkette von 1–8 C-Atomen bedeutet,
B eine geradkettige oder verzweigte Alkylengruppe mit 1–12 C-Atomen bedeutet,
X eine Bindung darstellt und
n die Zahl 1 bedeutet,
sowie deren physiologisch verträglichen Salze.

4. Verbindungen gemäß Anspruch 1, 2 oder 3, dadurch gekennzeichnet, daß sie
1-(2-Allylphenoxy)-3-[1,1-dimethyl-2-(4-nitroxy-butanoyl-amino)-ethylamino]-2-propanol cyclaminat
1-(2-Chloro-5-methyl-phenoxy)-3-[1,1-dimethyl-2-(4-nitroxy-butanoylamino)-ethylamino]-2-propanol cyclaminat
1-(2-Cyanophenoxy)-3-[1,1-dimethyl-2-(4-nitroxy-butanoylamino)-ethylamino]-2-propanol fumarat
3-[2-(4-Nitroxy-butanoylamino)-ethylamino]-1-(2-oxoindolin-4-yloxy)-2-propanol fumarat
1-(Indol-4-yloxy)-3-[2-(4-nitroxy-butanoylamino)ethylamino]-2-propanol hemifumarat
1-(1,2,3,4-Tetrahydro-2-oxo-chinolin-5-yloxy)-3-[2-(4-nitroxy-butanoylamino)-ethylamino]-2-propanol fumarat
3-[2-(4-Nitroxy-pentanoylamino)-ethylamino]-1-(2-oxoindolin-4-yloxy)-2-propanol cyclaminat
3-[2-(5-Nitroxy-pentanoylamino)-ethylamino]-1-(2-oxo-indolin-4-yloxy)-2-propanol hemifumarat
sind.

5. Verfahren zur Herstellung von Amino-propanol-Derivaten der allgemeinen Formel I

$$Ar-O-CH_2-\overset{OH}{\overset{|}{C}H}-CH_2-NH-A-NH-CO-X-B-(ONO_2)_n \qquad (I)$$

worin Ar, A, B, X und n die in Anspruch I angegebenen Bedeutungen haben, sowie deren physiologisch verträgliche Salze, dadurch gekennzeichnet, daß man in an sich bekannter Weise
a) eine Verbindung der allgemeinen Formel II

$$Ar-O-CH_2-\overset{OH}{\overset{|}{C}H}-CH_2-NH-A-NH-CO-X-B-(OH)_n \qquad (II)$$

in der A, Ar, B, X und n die oben angegebenen Bedeutungen besitzen, einer Nitratester-Bildungsreaktion unterwirft, oder

b) eine Verbindung der allgemeinen Formel III

Ar'–O–Z (III)

in der Ar' die gleiche Bedeutung wie Ar hat oder gegebenenfalls eine entsprechende Synthesevorstufe sein kann und Z eine der Gruppierungen

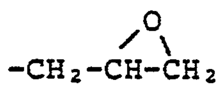

oder

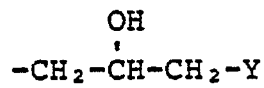

darstellt, worin Y eine reaktive Gruppe bedeutet,
b1) mit einem Amin der allgemeinen Formel IV

$H_2N–A–NH–CO–X–B–(ONO_2)n$ (IV)

in der A, B, X und n die oben angegebenen Bedeutungen haben, oder
b2) mit einem Amin der allgemeinen Formel V

$H_2N–A–(NH_2)_{mask.}$ (V)

worin A die oben angegebene Bedeutung hat und $–(NH_2)_{mask.}$ eine freie $–NH_2$-Gruppe oder gegebenenfalls eine maskierte $–NH_2$-Gruppe darstellt, zu einer Verbindung der allgemeinen Formel VI

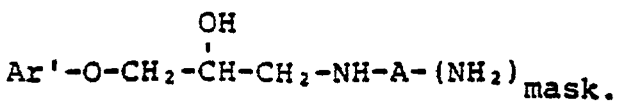

(VI)

umsetzt und diese, gegebenenfalls nach Demaskierung der $–(NH_2)_{mask.}$-Gruppierung zur $–NH_2$-Gruppe und/oder nach Überführung der Gruppe Ar' in die Gruppe Ar, mit einer Verbindung der allgemeinen Formel VII

$Y–CO–X–B–(ONO_2)_n$ (VII)

in der B, X, Y und n die oben angegebenen Bedeutungen besitzen, umsetzt, oder
c) eine Verbindung der allgemeinen Formel VIII

Ar'–OH (VIII)

in der Ar' die oben angegebene Bedeutung hat
c1) mit einer Verbindung der allgemeinen Formel IX

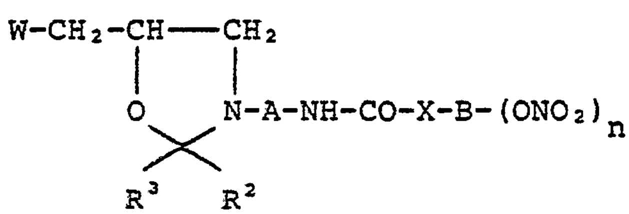

(IX)

worin A, B, X und n die oben angegebenen Bedeutungen besitzen, W Mesyloxy, Tosyloxy oder Halogen, $R^3$ Wasserstoff oder Alkyl und $R^2$ unabhängig Wasserstoff, Alkyl oder Phenyl bedeuten oder $R^3$ und $R^2$ zusammen mit dem benachbarten Kohlenstoffatom ein Carbonyl-Radikal bilden, umsetzt, und gegebenenfalls die Gruppe Ar' in die Gruppe Ar überführt und den Oxazolidin-Ring spaltet, oder
c2) mit einer Verbindung der allgemeinen Formel X

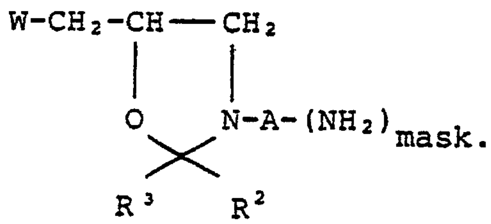

(X)

worin A, $R^3$, $R^2$, W und $-(NH_2)_{mask.}$ die oben genannten Bedeutungen haben, umsetzt, den Oxazolidin-Ring spaltet und die erhaltene Verbindung der allgemeinen Formel VI, gegebenenfalls nach Überführung der Gruppe Ar' in die Gruppe Ar und/oder nach Demaskierung der Gruppe $-(NH_2)_{mask.}$ in die $-NH_2$-Gruppe, mit einer Verbindung der allgemeinen Formel VII umsetzt, oder
c3) mit einer Verbindung der allgmeinen Formel XI

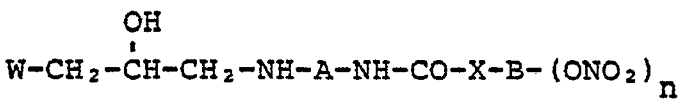

(XI)

in der A, B, W, X und n die oben angegebenen Bedeutungen besitzen, umsetzt und gegebenenfalls die Gruppe Ar' in die Gruppe Ar überführt, oder
c4) mit einer Verbindung der allgemeinen Formel XII

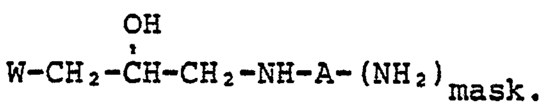

(XII)

in der A, W und $-(NH_2)_{mask.}$ die oben angegebenen Bedeutungen besitzen,
zu einer Verbindung der allgemeinen Formel VI umsetzt und diese, gegebenenfalls nach Überführung der Gruppe Ar' in die Gruppe Ar und/oder nach Demaskierung der Gruppe $-(NH_2)_{mask.}$ ind die $-NH_2$-Gruppe, mit einer Verbindung der allgemeinen Formel VII umsetzt, oder
d) eine Verbindung der allgemeinen Formel XIII

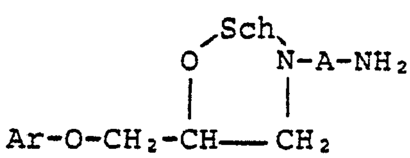

(XIII)

in der A und Ar die oben angegebenen Bedeutungen besitzen und Sch ein Dialkylsilylen-Radikal bedeutet, mit einer Verbindung der allgemeinen Formel VII umsetzt und die Sch-Gruppierung abspaltet und anschließend gewünschtenfalls die erhaltenen Verbindungen in ihre pharmakologisch unbedenklichen Salze überführt.

6. Verfahren zur Herstellung von Amino-propanol-Derivaten der allgemeinen Formel I gemäß Anspruch 5, worin Ar, A, B, X und n die in Anspruch 2 angegebenen Bedeutungen haben, sowie deren physiologisch verträgliche Salze.

7. Verfahren zur Herstellung von Amino-propanol-Derivaten der allgemeinen Formel I gemäß Anspruch 5 oder 6, worin Ar, A, B, X und n die in Anspruch 3 angegebenen Bedeutungen haben, sowie deren physiologisch verträglichen Salze.

8. Arzneimittel enthaltend eine Verbindung gemäß Anspruch 1, 2, 3 oder 4 sowie übliche Träger- und Hilfsstoffe.

9. Verwendung von Verbindungen gemäß Anspruch 1, 2, 3 oder 4 zur Herstellung von Arzneimitteln zur Prophylaxe von Herz- und Kreislauferkrankungen.

10. Verbindungen gemäß Anspruch 1, 2, 3 oder 4 zur Herstellung von Arzneimitteln zur Prophylaxe von Herz- und Kreislauferkrankungen.

## Revendications

1. Composé de formule générale I

$$Ar{-}O{-}CH_2{-}\underset{\displaystyle|}{\overset{OH}{C}}H{-}CH_2{-}NH{-}A{-}NH{-}CO{-}X{-}B{-}(ONO_2)_n \qquad (I)$$

dans laquelle
Ar représente un groupe phényle qui peut être non substitué ou substitué par un groupe halogène, cyano, hydroxy, amino, oxo, nitro, carboxyle, carbamoyle, trifluorométhyle, alkyle en $C_1$–$C_{10}$, alcényle en $C_2$–$C_{10}$, alcynyle en $C_2$–$C_{10}$, cycloalkyle en $C_3$–$C_8$, bicycloalkyle en $C_4$–$C_{11}$, alcanoyle en $C_1$–$C_7$, alcoxy en $C_1$–$C_{10}$, alcoxyalkyle en $C_2$–$C_{10}$, alcoxyalcoxyalkyle ou bien alcoxyalcoxy avec jusqu'à 10 atomes de carbone, alcényloxy en $C_2$–$C_{10}$, alcényloxyalkyle en $C_2$–$C_{10}$, alcynyloxy en $C_2$–$C_{10}$, alcynyloxyalkyle en $C_3$–$C_{10}$, cycloalcoxy en $C_3$–$C_8$, alkylthio en $C_1$–$C_{10}$, alkylthioalkyle en $C_2$–$C_{10}$, acylamino en $C_1$–$C_7$, acylaminoalkyle avec jusqu'à 8 atomes de carbone, acyloxy en $C_1$–$C_6$, alcoxycarbonyle en $C_1$–$C_5$, cycloalcoxycarbonyle en $C_4$–$C_8$, alkylaminocarbonylamino en $C_2$–$C_4$, dialkylaminocarbonylamino en $C_3$–$C_7$, dialkylaminocarbonylaminoalkyle en $C_4$–$C_{11}$, dialkylaminocarbonylalkyle en $C_4$–$C_{11}$, dialkylaminocarbonylalcoxy en $C_4$–$C_{10}$, les quatre derniers groupes de deux groupes alkyle terminaux pouvant former, en commun avec l'atome d'azote, également un groupe cyclique avec quatre ou cinq atomes de C, cycloalkylaminocarbonylamino en $C_4$–$C_8$, alkylaminocarbonylaminoalkyle en $C_3$–$C_9$, cycloalkylaminocarbonylaminoalkyle en $C_5$–$C_{12}$, alcoxycarbonylaminoalkyle en $C_3$–$C_{12}$, cycloalcoxycarbonylaminoalkyle en $C_5$–$C_{12}$, carbamoylalkyle en $C_2$–$C_5$, alkylaminocarbonylalkyle en $C_3$–$C_9$, cycloalkylaminocarbonylalkyle en $C_5$–$C_{12}$ ou alkylaminocarbonylalcoxy en $C_3$–$C_{10}$, ou Ar représente un groupe naphtyle, indényle, indolyle, indazolyle, imidazolyle, benzimidazolyle, benzotriazolyle, benzofuranyle, benzodioxolyle, benzothiophényle, benzothiazolyle, benzisothiazolyle, quinolyle, isoquinolyle, benzodiazinyle, benzopyranyle, benzothiinyle, benzothiazinyle, benzothiadiazinyle, benzoxatiinyle, benzoxazolyle, benzisoxazolyle, ou carbazolyle, une ou plusieurs laisons doubles pouvant être hygrogénées et les groupes mentionnés ci-dessus pouvant être non substitués ou substitués par un groupe alkyle an $C_1$–$C_{10}$, cyano, hydroxyalkyle, hydroxy, oxo, formyle, alcanoyle ou alkylcarbonylamino.
A représente un groupe alkylène à chaîne droite ou ramifiée comportant 1 à 8 atomes de C, un groupe $-CH_2$ pouvant être remplacé par un groupe cycloalkylène avec 3 à 7 atomes de C,
B représente un groupe alkylène à chaîne droite, mono- ou bicyclique, éventuellement ramifiée, saturée ou insaturée, avec 1 à 12 atomes de C, un groupe $-CH_2$ pouvant être remplacé par un groupe cycloalkylène comportant 3 à 7 atomes de C et/ou jusqu'à 2 groupes $-CH_2$ pouvant être remplacé chacun par un atome d'oxygène ou un atome de soufre ou un groupe $-SO$ ou $-SO_2$,
X représente une liaison, un atome d'oxygène ou un groupe $-NR^1$, dans lequel $R^1$ représente l'hydrogène ou un groupe alkyle ou nitroxyalkyle à 1 à 6 atomes de C, à chaîne droite ou ramifiée, saturée ou insaturée, ou $R^1$ peut également former, en commun avec l'atome d'azote du groupe $-NR^1$ et avec un groupe $-CH_2$ de la chaîne B, un cycle hétéroaliphatique avec 4 à 6 atomes de C,
n représente un nombre entier de 1 à 3, ainsi que leurs sels physiologiquement acceptables.

2. Composé selon la revendication 1, caractérisé en ce que dans la formule générale I
Ar représente un groupe phényle qui peut être non substitué ou éventuellement substitué une ou deux fois par un groupe halogène, cyano-, hydroxyle, alkyle en $C_1$–$C_{10}$, alcényle en $C_2$–$C_{10}$, alcényloxy en $C_2$–$C_{10}$, alcoxy en $C_1$–$C_{10}$, cycloalkyle en $C_3$–$C_8$, alcanoyle en $C_1$–$C_7$, acyloxy en $C_1$–$C_6$, bicycloalkyle en $C_4$–$C_{11}$, alkoxyalkyle en $C_2$–$C_{10}$, acylamino en $C_1$–$C_7$, carbamoylalkyle en $C_2$–$C_5$ et cycloalkylaminocarbonylamino en $C_4$–$C_8$, où un groupe indolyle, naphtyle, quinoléinyle, benzimidazolyle, isoquinoléinyle, benzopyranyle, benzodioxolyle, indényle, benzoxazolyle ou carbazolyle, qui peuvent être non substitués ou éventuellement substitués une ou deux fois par un groupe cyano-, oxo-, hydroxyle ou alkyle, une ou plusieurs liaisons doubles pouvant être hydrogénées dans les systèmes condensés et hétérocycliques mentionnés précédemment,
A représente un groupe alkylène à chaîne droite ou ramifiée comportant 1 à 8 atomes de C,
B représente un groupe alkylène à chaîne droite, mono ou bicyclique, éventuellement ramifiée, saturée ou insaturée, comportant 1 à 12 atomes de C, ou un groupe $-CH_2-$ peut être substitué par un groupe cycloalkylène comportant 3 à 7 atomes de C et/ou jusqu'à deux groupes $-CH_2-$, chaque fois par un atome d'oxygène ou un atome de soufre ou un groupe $-S(=O)-$ ou $-S(=O)_2$,
X représente une liaison, un atome d'oxygène ou un groupe $-NR^1-$, où $R^1$ représente l'hydrogène, un groupe alkyle ou nitroxyalkyle saturé ou insaturé, à chaîne droite ou ramifiée, comportant 1 à 6 atomes de C,
n représente 1 ou 2,
ainsi que leurs sels physiologiquement acceptables.

3. Composé selon la revendication 1 ou 2, caractérisé en ce que dans la formule générale I
Ar représente un groupe phényle qui peut être non substitué ou éventuellement substitué une ou deux fois par un groupe halogène, cyano-, alkyle en $C_1$–$C_{10}$, alcényle en $C_2$–$C_{10}$, ou un groupe indolyle, indolinonyle, quinoléinyle ou 1,2,3,4-tétrahydro-2-oxo-quinoléinyle,

A représente un groupe alkylène à chaîne droite ou ramifiée comportant 1 à 8 atomes de C,
B représente un groupe alkylène à chaîne droite ou ramifiée comportant 1 à 12 atomes de C,
X représente une liaison et,
n représente 1,
ainsi que leurs sels physiologiquement acceptables.

4. Composé selon les revendications 1, 2 ou 3, caractérisé en ce que ce sont les:
cyclaminate de 1-(2-allylphénoxy)-3-[1,1-diméthyl-2-(4-nitroxy-butanoylamino)-éthylamino]-2-propanol
cyclaminate de 1-(2-chloro-5-méthyl-phénoxy)-3-[1,1-diméthyl-2-(4-nitroxy-butanoylamino)-éthylamino]-2-propanol
fumarate de 1-(2-cyanophénoxy)-3-[1,1-diméthyl-2-(4-nitroxy-butanoylamino)-éthylamino]-2-propanol
fumarate de 3-[2-(4-nitroxy-butanoylamino)-éthylamino]-1-(2-oxo-indolin-4-yloxy)-2-propanol
semifumarate de 1-(indol-4-yloxy)-3-[2-(4-nitroxy-butanoylamino)-éthylamino]-2-propanol
fumarate de 1-(1,2,3,4-tétrahydro-2-oxo-quinoléin-5-yloxy)-3-[2-(4-nitroxy-butanoylamino)-éthylamino]-2-propanol
cyclaminate de 3-[2-(4-nitroxy-pentanoylamino)-éthylamino]-1-(2-oxo-indolin-4-yloxy)-2-propanol
semifumarate de 3-[2-(5-nitroxy-pentanoylamino)-éthylamino]-1-(2-oxo-indolin-4-yloxy)-2-propanol

5. Procédé de préparation des dérivés d'amino-propanol répondant à la formule générale I

$$Ar-O-CH_2-\overset{\overset{\displaystyle OH}{|}}{CH}-CH_2-NH-A-NH-CO-X-B-(ONO_2)_n \qquad (I)$$

où Ar, A, B, X et n ont les significations indiquées à la revendication 1, ainsi que leurs sels physiologiquement acceptables, caractérisés en ce que

a) on soumet un composé de formule générale II

$$Ar-O-CH_2-\overset{\overset{\displaystyle OH}{|}}{CH}-CH_2-NH-A-NH-CO-X-B-(OH)_n \qquad (II)$$

où Ar, A, B, X et n ont la signification mentionnée plus haut, à une réaction de formation de nitratester, ou bien

b) on fait réagir un composé de formule générale III

Ar'–O–Z (III)

où Ar' a la même signification que Ar, ou éventuellement peut-être un précurseur de synthèse correspondant, et Z représente un groupement

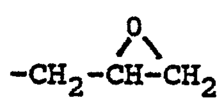 ou $-CH_2-\overset{\overset{\displaystyle OH}{|}}{CH}-CH_2-Y$

où Y représente un groupe réactif

b1) avec une amine de formule générale IV

$H_2N-A-NH-CO-X-B-(ONO_2)_n$ (IV)

où A, B, X et n ont la signification mentionnée plus haut, ou

b2) avec une amine de formule générale V

$H_2N-A-(NH_2)_{mask.}$ (V)

où A a la signification mentionnée plus haut et $-(NH_2)_{mask.}$ représente un groupe $NH_2$ libre ou éventuellement un groupe $-NH_2$ protégé, en un composé de formule générale VI

$$Ar'-O-CH_2-\overset{\overset{\displaystyle OH}{|}}{CH}-CH_2-NH-A-(NH_2)_{mask.} \qquad (VI)$$

et on fait réagir celui-ci, éventuellement après élimination du groupe de protection de $-(NH_2)_{mask.}$ de manière à obtenir un groupe $NH_2$ et/ou après transformation du groupe Ar' en groupe Ar avec un composé de formule générale VII

$Y{-}CO{-}X{-}B{-}(ONO_2)_n$ (VII)

où B, X, Y et n ont la signification mentionnée plus haut, ou
c) on fait réagir un composé de formule générale VIII

Ar′–OH (VIII)

où Ar′ a la signification mentionnée plus haut,
c1) avec un composé de formule générale IX

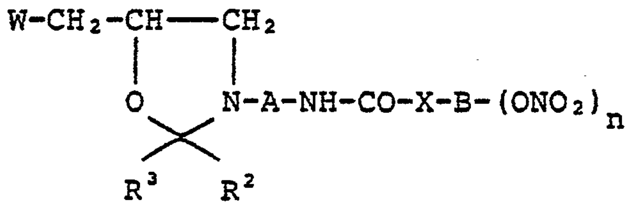 (IX)

où A, B, X et n ont les significations mentionnées plus haut, W représente un groupe mésyloxy, tosyloxy ou halogène, $R^3$ représente un atome d'hydrogène ou un groupe alkyle et $R^2$ représente indépendamment l'hydrogène, un groupe alkyle ou phényle ou $R^3$ et $R^2$ forment conjointement avec l'atome de carbone voisin un groupe carbonyle, et éventuellement, on transforme le groupe Ar′ en groupe Ar et on ouvre le cycle oxyzolidine, ou
c2) avec un composé de formule générale X

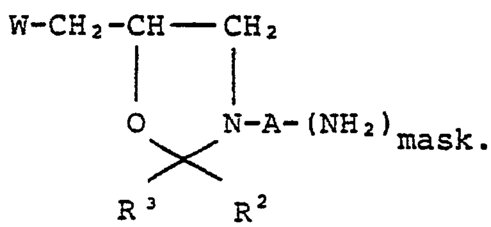 (X)

où A, $R^3$, $R^2$, W et $-(NH_2)_{mask.}$ ont les significations mentionnées plus haut, on ouvre le cycle oxazoldine et on fait réagir le composé obtenu, de formule générale VI, éventuellement après transformation du groupe Ar′ en groupe Ar et/ou après élimination du groupe de protection de $-(NH_2)_{mask.}$ de manière à obtenir le groupe $-NH_2$, avec un composé de formule générale VII, ou
c3) avec un composé de formule générale XI

$$W{-}CH_2{-}\overset{OH}{\overset{|}{C}}H{-}CH_2{-}NH{-}A{-}NH{-}CO{-}X{-}B{-}(ONO_2)_n \qquad (XI)$$

où A, B, W, X et n ont les significations mentionnées plus haut, et éventuellement on transforme Ar′ en groupe Ar, ou
c4) avec un composé de formule générale XII

$$W{-}CH_2{-}\overset{OH}{\overset{|}{C}}H{-}CH_2{-}NH{-}A{-}(NH_2)_{mask.} \qquad (XII)$$

où A, W et $-(NH_2)_{mask.}$ ont les significations mentionnées plus haut, pour obtenir un composé de formule générale VI, et on fait réagir celui-ci, éventuellement après transformation du groupe Ar′ en groupe Ar et/ou après élimination du groupe de protection de $(NH_2)_{mask.}$ de manière à obtenir le groupe $NH_2$, avec un composé de formule générale VII, ou
d) on fait réagir un composé de formule générale XIII

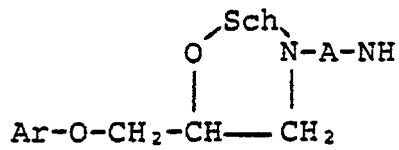

(XIII)

où A et Ar ont les significations mentionnées plus haut, et Sch représente un groupe dialkylsilylène, avec un composé de formule générale VII, et on sépare le groupe Sch, et ensuite on transforme éventuellement les composés obtenus en leurs sels pharmacologiquement acceptables.

6. Procédé de préparation des dérivés d'amino-propanol de formule générale I selon la revendication 5, où Ar, A, B, X et n ont les significations mentionnées à la revendication 2, ainsi que leurs sels physiologiquement acceptables.

7. Procédé de préparation des dérivés d'amino-propanol de formule générale I selon la revendication 5 ou 6, où Ar, A, B, X et n ont les significations mentionnées à la revendication 3, ainsi que leurs sels physiologiquement acceptables.

8. Produits pharmaceutiques contenant un composé selon les revendications 1, 2, 3 ou 4 ainsi que des agents auxiliaires ou excipients habituels.

9. Utilisation des composés selon les revendications 1, 2, 3 ou 4 pour la préparation de produits pharmaceutiques destinés au traitement prophylactique des maladies cardio-vasculaires.

10. Composé selon les revendications 1, 2, 3 ou 4 pour la préparation de produits pharmaceutiques destinés au traitement prophylactique des maladies cardio-vasculaires.

## Claims

1. Compounds of the general formula I

$$Ar-O-CH_2-\overset{OH}{\overset{|}{C}}H-CH_2-NH-A-NH-CO-X-B-(ONO_2)_n \qquad (I)$$

wherein

Ar signifies a phenyl group which can be unsubstituted or substituted by halogen, cyano, hydroxyl, amino, oxo, nitro, carboxyl, carbamoyl, trifluoromethyl, $C_1$–$C_{10}$-alkyl, $C_2$–$C_{10}$-alkenyl, $C_2$–$C_{10}$-alkynyl, $C_3$–$C_8$-cycloalkyl, $C_4$–$C_{11}$-bicycloalkyl, $C_1$–$C_7$-alkanoyl, $C_1$–$C_{10}$-alkoxy, $C_2$–$C_{10}$-alkoxyalkyl, alkoxyalkoxyalkyl or alkoxyalkoxy with up to 10 C-atoms, $C_2$–$C_{10}$-alkenyloxy, $C_2$–$C_{10}$-alkenyloxyalkyl, $C_2$–$C_{10}$-alkynyloxy, $C_3$–$C_{10}$-alkynyloxyalkyl, $C_3$–$C_8$-cycloalkoxy, $C_1$–$C_{10}$-alkylthio, $C_2$–$C_{10}$-alkylthioalkyl, $C_1$–$C_7$-acylamino, acylaminoalkyl with up to 8 C-atoms, $C_1$–$C_6$-acyloxy, $C_1$–$C_5$-alkoxycarbonyl, $C_4$–$C_8$-cycloalkoxycarbonyl, $C_2$–$C_4$-alkylaminocarbonylamino, $C_3$–$C_7$-dialkylaminocarbonylamino, $C_4$–$C_{11}$-dialkylaminocarbonylaminoalkyl, $C_4$–$C_{11}$-dialkylaminocarbonylalkyl, $C_4$–$C_{10}$-dialkylaminocarbonylalkoxy, whereby in the case of the last four groups the two terminal alkyl groups, together with the nitrogen atom, can also form a cyclic group with 4 or 5 C-atomes, $C_4$–$C_8$-cycloalkylaminocarbonylamino, $C_3$–$C_9$-alkylaminocarbonylaminoalkyl, $C_5$–$C_{12}$-cycloalkylaminocarbonylaminoalkyl, $C_3$–$C_{12}$-alkoxycarbonylaminoalkyl, $C_5$–$C_{12}$-cycloalkoxycarbonylaminoalkyl, $C_2$–$C_5$-carbamoylalkyl, $C_3$–$C_9$-alkylaminocarbonylalkyl, $C_5$–$C_{12}$-cycloalkylaminocarbonylalkyl or $C_3$–$C_{10}$-alkylaminocarbonylalkoxy, or

Ar signifies a naphthyl, indenyl, indolyl, indazolyl, imidazolyl, benzimidazolyl, benztriazolyl, benzofuranyl, benzodioxolyl, benzothiophenyl, benzthiazolyl, benzisothiazolyl, quinolyl, isoquinolyl, benzodiazinyl, benzopyranyl, benzothiinyl, benzothiazinyl, benzothiadiazinyl, benzoxathiinyl, benzoxazolyl, benzisoxazolyl or carbazolyl group, whereby one or more double bonds can be hydrogenated and these previously mentioned groups can be unsubstituted or substituted by $C_1$–$C_{10}$-alkyl, cyano, hydroxyalkyl, hydroxyl, oxo, formyl, alkanoyl or alkylcarbonylamino,

A signifies a straight-chained or branched alkylene chain of 1–8 C-atoms, whereby a $-CH_2-$ group can be replaced by a cycloalkylene group of 3–7 C-atoms, B signifies a straight-chained, mono- or bicyclic possibly branched, saturated or unsaturated alkylene chain of 1–12 C-atoms, wherein a $-CH_2-$ group can be replaced by a cycloalkylene group of 3–7 C-atoms and/or up to 2 $-CH_2-$ groups can each be replaced by an oxygen atom or sulphur atom or a $-S(=O)-$ or $-S(=O)_2-$ group, X signifies a bond, an oxygen atom or a group $-NR^1-$, wherein $R^1$ represents hydrogen, a straight-chained or branched, saturated or unsaturated alkyl or nitroxyalkyl group of 1–6 C-atoms or $R^1$, together with the nitrogen atom of the group $-NR^1-$ and a $-CH_2-$ group of the chain B, can also bridge a heteroaliphatic ring with 4–6 C-atoms, n signifies the numbers 1–3, as well as their physiologically acceptable salts.

2. Compounds according to claim 1, characterised in that in the general formula I

Ar represents a phenyl group which is unsubstituted or is possibly substituted one or more times by halogen, cyano, hydroxyl, $C_1$–$C_{10}$-alkyl, $C_2$–$C_{10}$-alkenyl, $C_2$–$C_{10}$-alkenyloxy, $C_1$–$C_{10}$-alkoxy, $C_3$–$C_8$-cycloalkyl, $C_1$–$C_7$-alkanoyl, $C_1$–$C_6$-acyloxy, $C_4$–$C_{11}$-bicycloalkyl, $C_2$–$C_{10}$-alkoxyalkyl, $C_1$–$C_7$-

acylamino, $C_2$–$C_5$-carbamoylalkyl and $C_4$–$C_8$-cycloalkylamino-carbonylamino groups or represents an indolyl, naphthyl, quinolinyl, benzimidazolyl, isoquinolinyl, benzopyranyl, benzdioxolyl, indenyl, benzoxazolyl or carbazolyl group which can be unsubstituted or possibly substituted one or more times by cyano, oxo, hydroxyl or alkyl groups, whereby one or two double bonds in the above-mentioned condensed and heterocyclic systems can be hydrogenated,

A signifies a straight-chained or branched alkylene chain with 1–8 C-atoms,

B signifies a straight-chained, mono- or bicyclic, possibly branched, saturated or unsaturated alkylene chain of 1–12 C-atoms, wherein a $-CH_2-$ group can be replaced by a cycloalkylene group of 3–7 C-atoms and/or up to 2 $-CH_2-$ groups can each be replaced by an oxygen atom or sulphur atom or a $-S(=O)-$ or $-S(=O)_2-$ group,

X represents a bond, an oxygen atom or a group $-NR^1-$, wherein $R^1$ represents hydrogen, a straight-chained or branched, saturated or unsaturated alkyl or nitroxyalkyl group of 1–6 C-atoms, n signifies the numbers 1, 2, as well as their physiologically acceptable salts.

3. Compounds according to claim 1 or 2, characterised in that in the general formula I

Ar represents a phenyl group which can be unsubstituted or possibly substituted once or twice by halogen, cyano, $C_1$–$C_{10}$-alkyl, $C_2$–$C_{10}$-alkenyl groups, or represents an indolyl, indolinonyl, quinolinyl or 1,2,3,4-tetrahydro-2-oxoquinolinyl group,

A signifies a straight-chained or branched alkylene chain of 1–8 C-atoms,

B signifies a straight-chained or branched alkylene group with 1–12 C-atoms,

X represents a bond and n signifies the number 1, as well as their physiologically acceptable salts.

4. Compounds according to claim 1, 2 or 3, characterised in that they are

1-(2-allylphenoxy)-3-[1,1-dimethyl-2-(4-nitroxybutanoylamino)-ethylamino]-2-propanol cyclamate;

1-(2-chloro-5-methylphenoxy)-3[1,1-dimethyl-2-(4-nitroxybutanoylamino)-ethylamino]-2-propanol cyclamate;

1-(2-cyanophenoxy)-3-[1,1-dimethyl-2-(4-nitroxybutanoylamino)-ethylamino]-2-propanol fumarate;

3-[2-(4-nitroxybutanoylamino)-ethylamino]-1-(2-oxo-indolin-4-yloxy)-2-propanol fumarate;

1-(indol-4-yloxy)-3-[2-(4-nitroxybutanoylamino)-ethylamino]-2-propanol hemifumarate;

1-(1,2,3,4-tetrahydro-2-oxoquinolin-5-yloxy)-3-[2-(4-nitroxybutanoylamino)-ethylamino]-2-propanol fumarate;

3-[2-(4-nitroxypentanoylamino)-ethylamino]-1-(2-oxoindolin-4-yloxy)-2-propanol cyclamate;

3-[2-(5-nitroxypentanoylamino)-ethylamino]-1-(2-oxo-indolin-4-yloxy)-2-propanol hemifumarate.

5. Process for the preparation of aminopropanol derivatives of the general formula I

$$Ar-O-CH_2-\overset{OH}{\overset{|}{C}H}-CH_2-NH-A-NH-CO-X-B-(ONO_2)_n \qquad (I)$$

wherein Ar, A, B, X and n have the meanings given in claim 1, as well as of their physiologically acceptable salts, characterised in that, in per se known manner, one

a) subjects a compound of the general formula II

$$Ar-O-CH_2-\overset{OH}{\overset{|}{C}H}-CH_2-NH-A-NH-CO-X-B-(OH)_n \qquad (II)$$

in which A, Ar, B, X and n possess the above-given meanings, to a nitrate ester-forming reaction, or

b) reacts a compound of the general formula III

Ar'–O–Z (III)

in which Ar' has the same meaning as Ar or is optionally an appropriate synthesis precursor and Z represents one of the groupings

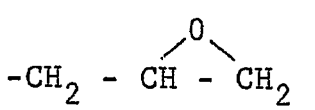 or

$$-CH_2-\overset{OH}{\overset{|}{C}H}-CH_2-Y$$

wherein Y signifies a reactive group,

b1) with an amine of the general formula IV

$H_2N-A-NH-CO-X-B-(ONO_2)_n$ (IV)

in which A, B, X and n have the above-given meanings; or

b2) with an amine of the general formula V

$H_2N-A-(NH_2)_{mask.}$ (V)

wherein A has the above-given meaning and $-(NH_2)_{mask.}$ represents a free $-NH_2$ group and possibly a masked $-NH_2$ group, to a compound of the general formula VI

$$Ar'-O-CH_2-\overset{OH}{\overset{|}{C}}H-CH_2-NH-A-(NH_2)_{mask.} \qquad (VI)$$

and, possibly after demasking of the $-(NH_2)_{mask.}$ grouping to an $-NH_2$ group and/or after conversion of an Ar' radical into an Ar radical, reacts this with a compound of the general formula VII

$Y-CO-X-B-(ONO_2)_n$ (VII)

in which B, X, Y and n possess the above-given meanings; or
c) reacts a compound of the general formula VIII

Ar'–OH (VIII)

in which Ar' has the above-given meaning,
c1) with a compound of the general formula IX

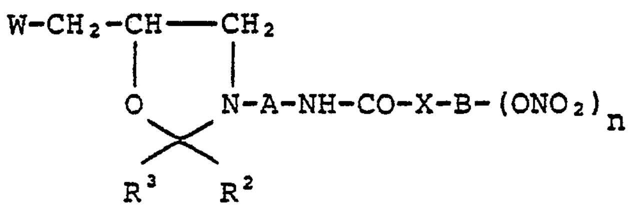

(IX)

wherein A, B, X and n possess the above-given meanings, W signifies mesyloxy, tosyloxy or halogen, $R^3$ hydrogen or alkyl radical and $R^2$ independently signifies hydrogen, alkyl or phenyl or $R^2$ and $R^3$, together with the neighbouring carbon atom, form a carbonyl radical, and possibly converts the group Ar' into the group Ar and cleaves the oxazolidine ring, or
c2) reacts with a compound of the general formula X

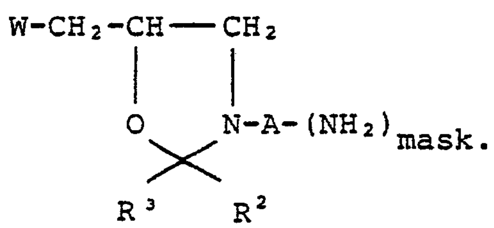

(X)

wherein A, $R^3$, $R^2$, W and $-(NH_2)_{mask.}$ have the above-given meanings, cleaves the oxazolidine ring and reacts the compound obtained of the general formula VI, possibly after conversion of the group Ar' into the group Ar and/or after demasking ot the group $-(NH_2)_{mask.}$ to an $-NH_2$ group, with a compound of the general formula VII; or
c3) reacts with a compound of the general formula XI

$$W-CH_2-\overset{OH}{\overset{|}{C}}H-CH_2-NH-A-NH-CO-X-B-(ONO_2)_n \qquad (XI)$$

in which A, B, W, X and n have the above-given meanings, and possibly converts the group Ar' into the group Ar, or
c4) reacts with a compound of the general formula XII

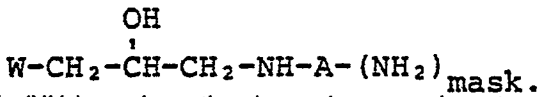 (XII)

in which A, W and $-(NH_2)_{mask.}$ have the above-given meanings, to a compound of the general formula VI and, optionally after conversion of the group Ar' into the group Ar and/or after demasking of the group $-(NH_2)_{mask.}$ to the $-NH_2$ group, reacts this with the compound of the general formula VII; or

d) reacts a compound of the general formula XIII

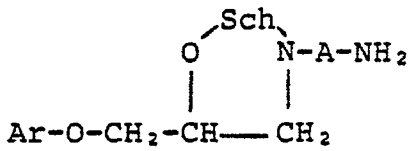 (XIII)

in which A and Ar possess the above-given meanings and Sch signifies a dialkylsilylene radical, with a compound of the general formula VII and splits off the Sch grouping

and subsequently, if desired, converts the compounds obtained into their pharmacologically acceptable salts.

6. Process for the preparation of aminopropanol derivatives of the general formula I according to claim 5, wherein Ar, A, B, X and n have the meanings given in claim 2, as well as their physiologically acceptable salts.

7. Process for the preparation of aminopropanol derivatives of the general formula I according to claim 5 or 6, wherein Ar, A, B, X and n have the meanings given in claim 3, as well as of their physiologically acceptable salts.

8. Medicaments containing a compound according to claim 1, 2, 3 or 4, as well as usual carrier and auxiliary materials.

9. Use of compounds according to claim 1, 2, 3 or 4 for the preparation of medicaments for the prophylaxis of heart and circulatory diseases.

10. Compounds according to claim 1, 2, 3 or 4 for the preparation of medicaments for the prophylaxis of heart and circulatory diseases.